(19) Europäisches Patentamt
European Patent Office
Office européen des brevets

(11) Veröffentlichungsnummer: **0 601 417 A2**

# (12) EUROPÄISCHE PATENTANMELDUNG

(21) Anmeldenummer: **93119098.7**

(22) Anmeldetag: **26.11.93**

(51) Int. Cl.5: **C07H 15/04**, C08G 69/10, A61K 31/70, A61K 47/48, C07H 13/04, G01N 33/50

(30) Priorität: **11.12.92 DE 4241829**
**10.08.93 DE 4326777**

(43) Veröffentlichungstag der Anmeldung:
**15.06.94 Patentblatt 94/24**

(84) Benannte Vertragsstaaten:
**AT BE CH DE DK ES FR GB GR IE IT LI LU MC NL PT SE**

(71) Anmelder: **HOECHST AKTIENGESELLSCHAFT**
**Brüningstrasse 50**
**D-65929 Frankfurt am Main(DE)**

(72) Erfinder: **Stahl, Wilhelm, Dr.**
**Drosselweg 5**
**D-65929 Frankfurt/Main(DE)**
Erfinder: **Ahlers, Michael, Dr.**
**Im Münchfeld 11**
**D-55127 Mainz(DE)**
Erfinder: **Walch, Axel, Dr.**
**Hans-Sachs-Strasse 5**
**D-60487 Frankfurt/Main(DE)**
Erfinder: **Bartnik, Eckhart, Dr.**
**Karlsruher Strasse 8**
**D-65205 Wiesbaden(DE)**
Erfinder: **Kretzschmar, Gerhard, Dr.**
**Ulmenweg 10**
**D-65760 Eschborn(DE)**
Erfinder: **Grabley, Susanne, Dr.**
**Hölderlinstrasse 7**
**D-61462 Königstein(DE)**
Erfinder: **Schleyerbach, Rudolf, Dr.**
**Finkenweg 10**
**D-65719 Hofheim/Taunus(DE)**

(54) **Physiologisch verträglicher und physiologisch abbaubarer, Kohlenhydratrezeptorblocker auf Polymerbasis, ein Verfahren zu seiner Herstellung und seine Verwendung.**

(57) Die Erfindung betrifft physiologisch verträgliche und physiologisch abbaubarer Kohlenhydratrezeptorblocker auf Polymerbasis mit einem HLB*-Wert von 10 bis 20 enthaltend:
Kohlenhydratanteil - bifunktionellen Spacer - hydrophiles, biodegradables Polymer - Wirkungsverstärker, wobei der aus 1 bis 20 natürlich vorkommenden gleichen oder verschiedenen Monosaccharidbausteinen bestehende Kohlenhydratanteil via ein oder mehrere bifunktionelle Spacer, der ein natürliches oder synthetisches Molekül ist, an ein hydrophiles, biodegradabeles Polymer gekoppelt ist, wobei das Polymer seinerseits Träger ein oder mehrerer Spacer ist und das hydrophile, biodegradable Polymer außerdem mit dem Wirkungsverstärker verbunden ist, welcher ein Vernetzer, ein oder mehrere Gruppen mit hydrophoben, hydrophilen oder ionischen Eigenschaften oder ein Löslichkeitsverbesserer ist, ein Verfahren zur Herstellung sowie seine in vivo Verwendung für die Blockierung kohlenhydraterkennender Rezeptoren.

Der Kohlenhydratrezeptorblocker ruft in vivo weder in seiner Gesamtheit noch in Form von Abbauprodukten Unverträglichkeitsreaktion hervor.

EP 0 601 417 A2

Die Erfindung betrifft polymere Kohlenhydratrezeptorblocker, die in vivo weder in ihrer Gesamtheit noch in Form von Abbauprodukten Unverträglichkeitsreaktionen hervorrufen, ihr Herstellungsverfahren sowie ihre in vivo Verwendung für die Blockierung kohlenhydraterkennender Rezeptoren.

Die Bedeutung der Kohlenhydrate im Rahmen physiologisch relevanter Erkennungsprozesse ist erst in den letzten Jahren näher untersucht und entschlüsselt worden (T. Feizi, Biochem. J. 1987, (245), 1; C.L.M. Stults et. al. Meth. Enzym. 1989, (179) 167; S. Hakamori, Adv. Cancer Res. 1989, (52), 257; M.P. Bevilacqua et. al., Science 1989, (243), 1160).

Man erkannte, daß die Kohlenhydrate, neben den Proteinen und Nucleinsäuren, der dritte wichtige Informationsträger des Lebens sind, da sie in der Regel eine große Anzahl von Stereozentren besitzen, die eine Vielzahl von Informationen beinhalten können. Ihre Anordnung auf der Zelloberfläche in Form von Liganden ermöglicht ihnen, aufgrund von Rezeptor-Ligandenbindungen, eine entscheidende Rolle bei der interzellulären Kommunikation und damit auch bei interzellulären Erkennungsprozessen zu spielen.

Grundsätzlich ist eine interzelluläre Kommunikation aufgrund von Rezeptor-Ligandenbindung möglich: Wasserlösliche Signalmoleküle, also Neurotransmitter, Proteinhormone, Wachstumsfaktoren, binden spezifisch an integrale Membramproteine (= Rezeptorproteinen), die sich auf der Zelloberfläche befinden. Nachdem der Zelloberflächenrezeptor das Signalmolekül (Ligand) gebunden hat, wird ein Signal in das Zellinnere transportiert, um damit Änderungen im Stoffwechsel der rezeptortragenden Zelle auszulösen. Nach einer variierenden Anzahl von Stunden wird durch metabolischen Abbau des Rezeptor-Ligandenkomplexes der Zellstoffwechsel wieder auf den Zustand vor der Komplexbildung umgeschaltet (Molecular Biology Of The Cell, 3. Auflage, Bruce Alberts et al., Garland Publishing, Inc., New York, USA, S. 733).

Kohlenhydratliganden auf Oberflächen von Säugerzellen (einschließlich der menschlichen Zellen) sind Erkennungsdomänen für Viren (z. B. J.C. Paulson, The Receptors, Vol, II, Ed.: P.M. Conn, Academic Press, 1985, 131), Bakterien (z. B. Strömberg et. al. EMBO J. 1990, (9), 2001), Toxine (z. B. K.A. Karlsson et. al. Sourcebook of Bacterial Protein Toxins, Eds. J.E. Alouf, J.H: Freer, Academic Press, 1991, 435) und Lectine (z. B. M.-F. Michel et.al. Appl. Environ. Microbiol. 1990, (56), 3537). Demzufolge spielen sie eine entscheidende Rolle bei der Einleitung von Krankheiten, die auf der Erkennung solcher Zelloberflächendeterminanten beruhen (bakterielle und virale Infektionen, entzündliche Erkrankungen, etc.).

Grundsätzlich läuft folgender Mechanismus ab:

Der erste Schritt ist die Anlagerung des Virus an die Zellmembran der zu infizierenden Zelle. Dabei heften sich die viralen Spikeproteine spezifisch an Liganden auf der Zellmembran an. Diese Spikeproteine sind im allgemeinen Glykoproteine mit Rezeptorfunktion. Im zweiten Schritt wird das Virusgenom über einen komplexen Mechanismus in die Zelle aufgenommen. Z. B. wurde für Infektionen mit Influenzaviren gezeigt, daß zu Infektionsbeginn sich das Virus mit Hilfe des Glycoproteins Haemaglutinin (= Rezeptor) an spezifische Liganden der Zelloberfläche heftet (Virology, Dulbecco, R. and Ginsberg H. S., 2. Auflage, S. 223).

Auch die bakterielle Infektion läuft im Prinzip nach dem oben dargestellten Mechanismus.

Rezeptor-Ligandenkomplexe spielen auch bei Entzündungsprozessen eine Rolle [B.K. Bradley et al., Cell 1990 (63), 861]. Untersuchungen haben beispielsweise gezeigt, daß das von Endothelzellen synthetisierte und exprimierte Protein ELAM 1 (= Rezeptor) in vivo wahrscheinlich die Anhaftung von Leukocyten via einen Liganden am Entzündungsherd vermittelt.

Die Therapie bakterieller oder viraler Erkrankungen mit freien Oligosacchariden, die anstelle der natürlichen Liganden an Rezeptoren, wie z. B. auf Viren oder bakteriellen Zellen binden sollen, scheitert an den sehr hohen Mengen des zu verabreichenden Oligosaccharids, da die Affinität zwischen den Rezeptoren und den Oligosacchariden gering ist [$K_D = \sim 10^{-4}$ M bei der Wechselwirkung zwischen einem monovalenten Galactosid und dem entsprechenden Lectin; D.T. Connolly et. al. J. Biol. Chem. 1982, (257), 939].

Es ist bekannt, daß eine erhöhte Wechselwirkung zwischen Rezeptor und Ligand durch die Kopplung mehrerer Liganden auf einer "Oberfläche" erreicht wird. Am Beispiel der Wechselwirkung zwischen dem Virusprotein Hemaglutinin, das an Neuraminsäure auf der Zelloberfläche bindet, wurde gezeigt, wie sich dieser polyvalente Effekt durch Verwendung eines Polymers auf eine solche Wechselwirkung auswirkt [monovalent: $K_D = 2 \times 10^{-4}$ M, polyvalent: $K_D = 3 \times 10^{-7}$ M; A. Spaltenstein et al. J. Am. Chem. Soc. 1991, (113), 686].

Als "Oberfläche" wurden bisher

a) Liposomen [N. Yamazaki, Int. J. Biochem., 1992, (24), 99]
b) Polyacrylamide
c) Polylysine oder
d) sulfatierte Polysccharide

untersucht:

a) Liposomen werden sowohl aus Zellpräparationen gewonnen als auch aus synthetischen Lipiden hergestellt. Der Ligand, z. B. ein Kohlenhydrat, wird auf der Liposomenoberfläche präsentiert, in dem ein hydrophober Alkylrest am Kohlenhydrat unter Ausnutzung hydrophober Wechselwirkungen in die Liposomenmembran integriert wird. Nachteilig bei der Verwendung von Liposomen als Oberfläche ist ihre geringe Stabilität in vivo und kurze Verweildauer im Blutkreislauf. Liposomenpräparationen werden neben anderen oligomeren und polymeren Präsentationsformen des Kohlenhydratanteils in WO 91/19501 und WO 91/19502 beschrieben.

b) Polymere auf Polyacrylamidbasis, die durch Polymerisation kohlenhydrathaltiger Monomerer hergestellt werden, sind als Träger von Liganden sind für eine medizinische Anwendung nicht geeignet. Der Polymeranteil besteht ausschließlich aus C-C-Bindungen. Der entscheidende Nachteil bei in vivo diagnostischen/therapeutischen Verwendung besteht darin, daß sie im Organismus zu toxischen Metaboliten abbaubar und somit in vivo unverträglich sind.

Beispiele für solche Publikationen sind:

- R.C Rathi et. al. [J. Polym. Sci.: Part A: Polym. Chem. 1991, (29), 1895] beschreiben die Polymerisation kohlenhydrathaltiger Acrylamidmonomere für eine Anwendung als Wirkstoffträger.
- S.-I. Nishimura et. al. [Macromolecules 1991, (24), 4236] beschreiben copolymerisierte Pentylglycoside mit Acrylamid und untersuchen die Wechselwirkung dieses Makromoleküls mit Lektinen.

Sowohl R.C. Rathi et al. wie auch S.-I. Nishimura et al. verwenden das Polymer nur als Träger für einen Kohlenhydratteil und ein pharmakologisch wirkendes Makromolekül. Das Wirkprinzip beruht auf einer möglichst spezifischen Rezeptor-Ligandenbindung, damit das pharmakologisch wirkende Makromolekül am Wirkort freigesetzt werden und seine Wirkung entfalten kann.

c) Polylysin besteht grundsätzlich aus physiologisch gut verträglichen, also nicht toxischen Bausteinen. Nachteilig am Polylysin sind jedoch die Vielzahl seiner freien $NH_2$-Gruppen, die unspezifische Wechselwirkungen mit Zellen oder Zelloberflächenstrukturen verursachen, so daß bei Polylysin-Pharmakonjugaten üblicherweise keine spezifische Wirkung des Pharmakons an dem Wirkort zu erreichen ist.

d) Die Zelladhäsion bei Verwendung von üblichen sulfatierten Polysacchariden resultiert aus wenig spezifischen ionischen Wechselwirkungen, so daß eine unspezifische Adhäsion auf beliebigen Zelloberflächen auftritt. Diese Makromoleküle enthalten keinen spezifischen Rezeptorblocker.

Die europäischen Offenlegungsschriften 0 089 938, 0 089 939 und 0 089 940 beanspruchen Zuckerverbindungen unterschiedlicher Kettenlänge, die identisch den auf Zelloberflächen befindlichen Liganden bzw. den auf Mikroorganismen befindlichen Rezeptoren sind. Der Erfindungsgedanke o. g. Offenlegungsschriften ist, die auf den Mikroorganismen befindlichen Rezeptoren durch die beanspruchten Zuckerverbindungen in vitro und in vivo zu blockieren, um damit Erkrankungen, vorallem bakterieller oder viraler Natur, von Säugern, insbesondere des Menschen, diagnostizieren und therapieren zu können. Beispiele für die in vitro Diagnose von bakteriellen Infektionen sind vorhanden, nicht jedoch für in vivo Diagnose oder Therapie.

Es werden außerdem in o. g. Offenlegungsschriften Zuckerverbindungen beansprucht, die an einen Träger - ggf. via Spacer - gekoppelt sind. Der Komplex Zuckerverbindung-(Spacer)-Träger oder die Zuckerverbindung alleine wird zur Herstellung von Antikörpern, Isolierung von Zelloberflächenstrukturen und Wunddesinfektion verwendet.

Weiterhin wurden auch die Wechselwirkungen von Dextran-fixierten $\beta$-Blockern mit $\beta$-Adrenorezeptoren untersucht (J. Pitka, J.W. Kusiak, in "Macromolecules as Drugs and as Carriers for biologically active Material", Ed.: D.A. Tirell, L.G. Donaruma, A.B. Turek; The New York Academy of sciences, Vol. 446, p. 249). $\beta$-Blocker sind künstliche Wirkstoffe, die im vorliegenden Fall über einen Spacer an den Polysaccharidträger Dextran gebunden sind. Die Blockierung der $\beta$-Adrenorezeptoren erfolgt nicht über eine spezifische Rezeptor-Ligandenbindung, sondern über eine vergleichsweise unspezifische Kopplung des Wirkstoffs.

In der Patentanmeldung WO 92/02527 wird ein Makromolekül beansprucht, welches aus einem Träger und Saccharidbausteinen aufgebaut ist und als Ligand für den ELAM-1 Rezeptor fungiert, um durch die Rezeptor-Ligandenbindung entzündliche Prozesse zu diagnostizieren. Die Diagnostik wird in vitro, nicht jedoch in vivo durchgeführt. Die Saccharidbausteine werden von einem festen Träger getragen. Unter einem "festen Träger" versteht der Fachmann Verbindungen, die sich gegenüber physiologischen Systemen inert verhalten müssen, da nur dann in vitro Diagnosen möglich sind.

Aufgabe der Erfindung ist es, Kohlenhydratrezeptorblocker mit verbesserter Wirksamkeit als Arzneimittel zu synthetisieren, die als Gesamtmolekül und als Abbauprodukte physiologisch verträglich sind und die ihre diagnostische oder therapeutische Wirkung durch Blockierung spezifischer Strukturen entfalten.

Diese Aufgabe wird erfindungsgemäß gelöst durch Synthese eines Kohlenhydratrezeptorblockers auf Polymerbasis, der aus den physiologisch gut verträglichen Komponenten: Kohlenhydratanteil - bifunktioneller Spacer - hydrophiles, biodegradabeles Polymer - Wirkungsverstärker besteht, wobei die einzelnen

Komponenten enzymatisch, chemisch und/oder chemoenzymatisch verknüpft sind, der Kohlenhydratrezeptorblocker im Organismus nicht oder kaum akkumuliert wird und seine diagnostische oder therapeutische Wirkung durch Blockierung spezifischer Strukturen als Gesamtmolekül entfaltet.

1. Ein physiologisch verträglicher und physiologisch abbaubarer Kohlenhydratrezeptorblocker auf Polymerbasis mit einem HLB*-Wert von 10 bis 20 enthaltend:
Kohlenhydratanteil - bifunktionellen Spacer - hydrophiles, biodegradables Polymer - Wirkungsverstärker, wobei der aus 1 bis 20 natürlich vorkommenden gleichen oder verschiedenen Monosaccharidbausteinen bestehende Kohlenhydratanteil via ein oder mehrere bifunktionelle Spacer, der ein natürliches oder synthetisches Molekül ist, an ein hydrophiles, biodegradabeles Polymer gekoppelt ist, wobei das Polymer seinerseits Träger ein oder mehrerer Spacer ist und das hydrophile, biodegradable Polymer außerdem mit dem Wirkungsverstärker verbunden ist, welcher ein Vernetzer, ein oder mehrere Gruppen mit hydrophoben, hydrophilen oder ionischen Eigenschaften oder ein Löslichkeitsverbesserer ist.

2. Physiologisch verträglicher und physiologisch abbaubarer Kohlenhydratrezeptorblocker auf Polymerbasis mit einem HLB*-Wert von 10 bis 20, erhältlich durch kovalente chemische, chemoenzymatische und/oder enzymatische Verknüpfung der folgenden Komponenten:

- Kohlenhydratanteil aus 1-20 natürlich vorkommenden, gleichen oder verschiedenen Monosaccharid-Bausteinen,
- bifunktionellem Spacer, der ein natürliches oder synthetisches Molekül ist,
- hydrophiles, biodegradables Polymer sowie
- Wirkungsverstärker, der ein Vernetzer, ein oder mehrere Gruppen mit hydrophoben, hydrophilen oder ionischen Eigenschaften oder ein Löslichkeitsverbesserer ist.

3. Ein Verfahren zur Herstellung des unter 1. genannten Kohlenhydratrezeptor-blockers, das dadurch gekennzeichnet ist, daß Kohlenhydratantteil, bifunktioneller Spacer, hydrophiles, biodegradabeles Polymer und Wirkungsverstärker enzymatisch, chemisch und/oder chemoenzymatisch verknüpft sind.

4. Eine Verwendung des unter 1. genannten Kohlenhydratrezeptorblockers zur diagnostischen, therapeutischen oder prophylaktischen Anwendung.

Die Erfindung wird im folgenden detailliert beschrieben, insbesondere in ihren bevorzugten Ausführungsformen.

Der Kohlenhydratrezeptorblocker ist physiologisch verträglich und hat vorzugsweise ein Molekulargewicht von ≦ 70 kd. Gut verträglich bedeutet, daß durch den erfindungsgemäßen Kohlenhydratrezeptorblokker keine Unverträglichkeitsreaktionen hervorgerufen werden, die das Wohlbefinden des Organismus beträchtlich beeinflussen.

Der Begriff "physiologisch verträglich" wird wie folgt definiert:
Der Kohlenhydratrezeptorblocker und das Polymer alleine dürfen in vivo nicht schädigend für den Organismus sein. Sie dürfen also nicht hämolytisch, nicht antigen und nicht thrombogen wirken.

Außerdem sind unspezifische Adsorptionen an Moleküle, wie z. B. Rezeptoren, Zellmembranbestandteile, Hormone usw., unerwünscht, da auch sie zu Unverträglichkeitsreaktionen führen können.
Der Einsatz eines Spacers, der Unverträglichkeitsreaktionen auslöst, ist möglich, wenn der Kohlenhydratrezeptorblocker als Ganzes oder seine in vivo entstehenden Abbauprodukte physiologisch verträglich sind.

Der Begriff "physiologisch abbaubar" wird wie folgt definiert:
Der Kohlenhydratrezeptorblocker ist in vivo als ganzes Molekül ausscheidbar oder in vivo zu kleineren physiologisch, verträglichen Bausteinen abbaubar, um in dieser Form ausscheidbar (= biodegradabel) zu sein. Dies gilt insbesondere für das Polymer.

Über die Abbaubarkeit des Kohlenhydratrezeptorblockers in vivo wird die Verweildauer des Kohlenhydratrezeptorblockers am jeweiligen Rezeptor und damit die Wirkzeit des Kohlenhydratrezeptorblockers gesteuert. So kann z. B. über die Wahl der Polymerbausteine und deren Verknüpfung die Abbaubarkeit gesteuert werden. Bei Überschuß des Blockers kann so eine Akkumulation im Organismus verhindert werden.

Die Begriffe "Rezeptor" und "Ligand" werden wie folgt definiert:
Der Rezeptor ist ein Makromolekül auf der Oberfläche der Zelle, welches aufgrund seiner Konfiguration eine Bindungsstelle zur spezifischen Bindung einer, selten mehrerer Signalsubstanzen besitzt. Diese Signalsubstanz wird als Ligand bezeichnet. Ziel der spezifischen Bindung des Liganden an den Rezeptor ist die Informationsübertragung in die Zelle und anschließende Zellstoffwechseländerung.

Definition des HLB*-Wertes:
Die Hydrophilie des erfindungsgemäßen Kohlenhydratrezeptorblockers wird über den HLB*-Wert charakterisiert. Der HLB*-Wert definiert das Verhältnis der hydrophilen und hydrophoben Anteile eines Moleküls mit Hilfe der Skala von 1-20 (R. Heusch, Kolloid-Zeitschrift und Zeitschrift für Polymere, Bd. 236, Heft 1, S. 31 ff.).

Der HLB*-Wert des erfindungsgemäßen Kohlenhydratrezeptorblockers liegt im vorliegenden Fall von 10 bis 20, vorzugsweise jedoch von 14 bis 20.

Als Oligosaccharide werden Zucker definiert, die aus ≦ 20 natürlich vorkommenden Monosaccharidbausteinen bestehen.

Der Begriff "Polymerbasis" bedeutet, daß das Polymer ein Bestandteil des Kohlenhydratrezeptorblokkers ist, der mit den weiteren Bestandteilen Spacer und Wirkungsverstärker verknüpft ist,
Definition des Begriffes "Belegungsgrad des Polymers mit dem Kohlenhydratanteil via Spacer": Der Belegungsgrad gibt den prozentualen Anteil (in %) der Repetiereinheiten des Polymers, an die der Kohlenhydratanteil via Spacer geknüpft ist. Der prozentuale Anteil ist bezogen auf die Gesamtheit der Repetiereinheiten.

Unter Repetiereinheiten versteht man die kleinste, regelmässig wiederkehrende Einheit, die den Abbau einer Kette vollständig beschreibt (Makromoleküle, Bd. 1, 5. Auflage, Hüthig & Wepf Verlag, Basel, Heidelberg, New York, 1990).

"Löslichkeitsverbesserer" ist eine chemische Gruppe, die die Löslichkeit des Kohlenhydratrezeptorblokkers verbessert.

Charakteristisch für den erfindungsgemäßen Kohlenhydratrezeptorblocker ist, daß er seine Funktion als Rezeptorblocker als Gesamtmolekül und nicht aufgrund der Wirksamkeit von Teilstrukturen ausübt. Daraus folgt, daß der Kohlenhydratanteil in gekoppelter Form aktiv ist und der Polymeranteil nicht nur als "Transportvehikel" zum Rezeptor sondern auch als aktiver Teil fungiert.

Die Synthese des Kohlenhydratrezeptorblockers erfolgt im Labormaßstab. Das bedeutet, der erfindungsgemäße Kohlenhydratrezeptorblocker wird in Milligrammenge bis Grammenge synthetisiert, während die für seine Synthese notwendigen Zwischenprodukte, d.h. das hydrophile biodegradable Polymer, der bifunktionelle Spacer und der Wirkungsverstärker in Gramm- bis Kilogrammenge hergestellt werden können. Eine Ausnahme bildet jedoch der Kohlenhydratanteil. Er kann lediglich in Milligrammenge bis zu einem Gramm synthetisiert werden. Hier sind die literaturbekannten Synthesekonzepte zur Herstellung von Oligosacchariden so angelegt, daß nach einer Reaktion, die üblicherweise nicht mit quantitativer Ausbeute verläuft, das erhaltene Produktgemisch säulenchromatographisch an Kieselgel gereinigt wird.
Dieses Reinigungsverfahren ist zur Herstellung von Mengen, die dem industriellen Bedarf entsprechen, im allgemeinen zu teuer und aufwendig und wird höchstens zur Reinigung von Endprodukten oder wertvollen Zwischenprodukten eingesetzt. Darüber hinaus werden zur Synthese von Oligosacchariden sehr häufig Schwermetallverbindungen eingesetzt.
Ihr Einsatz für die Synthese von Stoffen mit pharmazeutischer Wirkung ist im Hinblick auf eine zukünftige Zulassung des Kohlenhydratrezeptorblockers als Arzneimittel sehr bedenklich.

KOHLENHYDRATANTEIL:

Als Kohlenhydrate werden alle Verbindungen definiert, die entsprechend der Formel $C_n(H_2O)_n$ aufgebaut sind, sowie Polyhydroxyaldehyde, -ketone, -säuren und -amine sowie deren natürlich vorkommende Derivate.

Der Kohlenhydratanteil besteht aus 1-20 natürlich vorkommenden Monosaccharidbausteinen. Vorzugsweise werden 1-15 und besonders bevorzugt 1-10 natürlich vorkommende Monosaccharidbausteine verwendet.

Natürlich vorkommende Monosaccharidbausteine sind dem Fachmann aus Standardwerken der organischen Chemie oder der Biochemie, so z. B. aus: Beyer/Walter, "Lehrbuch der organischen Chemie", S. Hirzel Verlag Stuttgart, 19. Auflage, S.393 ff.; Albert L. Lehninger, "Biochemie", 2. Auflage, S. 201 ff., VCH-Verlagsgesellschaft, Weinheim, Deutschland oder Lubert Stryer, "Biochemie", Spektrum-der-Wissenschaft-Verlagsgesellschaft GmbH, Heidelberg, Deutschland, 1.Auflage, S. 345 ff., bekannt. Beispiele für Monosaccharidbausteine sind die D- bzw. L-Konfigurationen von Aldosen:

- Glycerinaldehyd, Erythrose, Threose, Ribose, Arabinose, Xylose, Lyxose, Allose, Altrose, Glucose, Mannose, Gulose, Idose, Galactose und Talose;
  und/oder

von Ketosen:

- Dihydroxyaceton, Erythrulose, Ribulose, Xylulose, Puscose, Fructose, Sorbose und/oder Tagatose.

Der Begriff "natürlich vorkommende" Monosaccharidbausteine schließt auch solche Monosaccharide ein, die natürlich vorkommende Substitutionen der angeführten Beispiele darstellen. Darunter versteht der Fachmann vorzugsweise Deoxyzucker, ganz vorzugsweise 2-, 3-, 4- oder 6-Deoxyaldosen, wie z. B. Fucose, Rhamnose, Digitoxase und/oder ganz vorzugsweise 2-, 3-, 5-, 6-Deoxyketosen, vorzugsweise Deoxyaminozucker, wie z. B. Glucosamin, Manosamin, Galactosamin, vorzugsweise Deoxyacylaminozucker, wie z. B. N-Acetylglucosamin, N-Acetylmanosamin, N-Acetylgalactosamin, vorzugsweise Aldon-, Aldar- und/oder Uronsäuren, wie z. B. Gluconsäure oder Glucuronsäure. Es können außerdem verwendet werden: Ascorbinsäure, Aminosäure-tragende Monosaccharide und Monosaccharide, die Lipid-, Phosphatidyl- oder Polyolrest tragen.

Unter "natürlich vorkommenden", substituierten Monosacchariden versteht der Fachmann auch solche mit einer Kohlenstoffkette, die länger als 6 C-Atome sind sowie deren nach den oben aufgeführten Kriterien substituierten Vertreter, wie z. B. Ketodeoxyoctan-, Ketodeoxynonansäure, N-Acylneuraminsäuren, N-Acetylmuraminsäure.

Bevorzugt werden solche Kohlenhydratanteile, die auf Zelloberflächen als Bestandteil von Glycoproteinen, Glycolipiden oder Proteoglycanen vorkommen sowie beliebige Teilstücke daraus.

Besonders bevorzugt sind solche Kohlenhydratanteile, die aus Monosacchariden bestehen, die auch im menschlichen Organismus vorkommen, wie Glucose, N-Acetylglucosamin, Galactose, N-Acetylgalactosamin, Mannose, Fucose, N-Acetylneuraminsäure und/oder Glucuronsäure.

Die den Kohlenhydratanteil bildenden Monosaccharidbausteine können gleich oder verschieden sein. Außerdem kann die Stereochemie der glycosdischen Verknüpfung (axial oder äquatorial bzw. $\alpha$ oder $\beta$) der einzelnen Monosaccharidbausteine gleich oder verschieden sein.

Der gesamte Kohlenhydratanteil kann beispielsweise aus den folgenden Zuckerresten bestehen:

Galβ1-4GlcNAc-;
Galβ1-3GlcNAc-;
SAα2-6Galβ1-4GlcNAc-;
SAα2-3Galβ1-4GlcNAc-;
SAα2-3Galβ1-3GlcNAc-;
Galβ1-4(Fucα1-3)GlcNAc-;
Galβ1-3(Fucα1-3)GlcNAc-;
SAα2-3Galβ1-3(Fucα1-4)GlcNAc-;
SAα2-3Galβ1-4(Fucα1-3)GlcNAc-;
Galβ1-4GlcNAcβ1-4GlcNAc-;
Galβ1-3GlcNAcβ1-4GlcNAc-;
SAα2-6Galβ1-4GlcNAcβ1-4GlcNAc-;
SAα2-3Galβ1-4GlcNAcβ1-4GlcNAc-;
SAα2-3Galβ1-3GlcNAcβ1-4GlcNAc-;
Galβ1-4(Fucα1-3)GlcNAcβ1-4GlcNAc-;
Galβ1-3(Fucα1-4)GlcNAcβ1-4GlcNAc-;
SAα2-3Galβ1-3(Fucα1-4)GlcNAcβ1-4GlcNAc-;
SAα2-3Galβ1-4(Fucα1-3)GlcNAcβ1-4GlcNAc-;
SAα2-3Galβ1-3(Fucα1-4)GlcNAcβ1-4Gal-;
SAα2-3Galβ1-4(Fucα1-3)GlcNAcβ1-4Gal-;
SAα2-3Galβ1-4GlcNAcβ1-3Galβ1-4(Fucα1-3)GlcNAc;
SAα2-6Galβ1-4GlcNAcβ1-3Galβ1-4(Fucα1-3)GlcNAc;
SAα2-3Galβ1-4(Fucα1-3)GlcNAcβ1-3Galβ1-4Glc;
SAα2-6Galβ1-4(Fucα1-3)GlcNAcβ1-3Galβ1-4Glc;
SAα2-3Galβ1-4GlcNAcβ1-3Galβ1-4(Fucα1-3)GlcNAcβ1-3Galβ1-4Glc;
SAα2-6Galβ1-4GlcNAcβ1-3Galβ1-4(Fucα1-3)GlcNAcβ1-3Galβ1-4Glc;
SAα2-3Galβ1-4(Fucα1-3)GlcNAcβ1-3Galβ1-4(Fucα1-3)GlcNAcβ1-3Galβ1-4Glc;
SAα2-6Galβ1-4(Fucα1-3)GlcNAcβ1-3Galβ1-4(Fucα1-3)GlcNAcβ1-3Galβ1-4Glc;
SAα2-3Galβ1-4(Fucα1-3)GlcNAcβ1-3Galβ1-4(Fucα1-3)Glc;
SAα2-6Galβ1-4(Fucα1-3)GlcNAcβ1-3Galβ1-4(Fucα1-3)Glc;
SAα2-3Galβ1-4GlcNAcβ1-3Galβ1-4(Fucα1-3)GlcNAcβ1-3Galβ1-4(Fucα1-3)Glc;
SAα2-6Galβ1-4GlcNAcβ1-3Galβ1-4(Fucα1-3)GlcNAcβ1-3Galβ1-4(Fucα1-3)Glc;
SAα2-3Galβ1-4(Fucα1-3)GlcNAcβ1-3Galβ1-4(Fucα1-3)GlcNAcβ1-3Galβ1-4(Fucα1-3)Glc;
SAα2-6Galβ1-4(Fucα1-3)GlcNAcβ1-3Galβ1-4(Fucα1-3)GlcNAcβ1-3Galβ1-4(Fucα1-3)Glc;
SAα2-3Galβ1-3(Fucα1-4)GlcNAc;
SAα2-6Galβ1-3(Fucα1-4)GlcNAc;
SAα2-3Galβ1-3GlcNAcβ1-4Galβ1-4(Fucα1-3)GlcNAc;
SAα2-6Galβ1-3GlcNAcβ1-4Galβ1-4(Fucα1-3)GlcNAc;
SAα2-3Galβ1-3(Fucα1-4)GlcNAcβ1-3Galβ1-4(Fucα1-3)GlcNAc;
SAα2-6Galβ1-3(Fucα1-4)GlcNAcβ1-3Galβ1-4(Fucα1-3)GlcNAc;
SAα2-3Galβ1-3(Fucα1-4)GlcNAcβ1-3Galβ1-4Glc;

SAα2-6Galβ1-3(Fucα1-4)GlcNAcβ1-3Galβ1-4Glc;
SAα2-3Galβ1-3GlcNAcβ1-4Galβ1-4(Fucα1-3)GlcNAcβ1-3Galβ1-4Glc;
SAα2-6Galβ1-3GlcNAcβ1-4Galβ1-4(Fucα1-3)GlcNAcβ1-3Galβ1-4Glc;
SAα2-3Galβ1-3(Fucα1-4)GlcNAcβ1-3Galβ1-4(Fucα1-3)GlcNAcβ1-3Galβ1-4Glc;
SAα2-6Galβ1-3(Fucα1-4)GlcNAcβ1-3Galβ1-4(Fucα1-3)GlcNAcβ1-3Galβ1-4Glc;
SAα2-3Galβ1-3(Fucα1-4)GlcNAcβ1-3Galβ1-4(Fucα1-3)Glc;
SAα2-6Galβ1-3(Fucα1-4)GlcNAcβ1-3Galβ1-4(Fucα1-3)Glc;
SAα2-3Galβ1-3GlcNAcβ1-4Galβ1-4(Fucα1-3)GlcNAcβ1-3Galβ1-4(Fucα1-3)Glc;
SAα2-6Galβ1-3GlcNAcβ1-4Galβ1-4(Fucα1-3)GlcNAcβ1-3Galβ1-4(Fucα1-3)Glc;
SAα2-3Galβ1-3(Fucα1-4)GlcNAcβ1-3Galβ1-4(Fucα1-3)GlcNAcβ1-3Galβ1-4(Fucα1-3)Glc;
SAα2-6Galβ1-3(Fucα1-4)GlcNAcβ1-3Galβ1-4(Fucα1-3)GlcNAcβ1-3Galβ1-4(Fucα1-3)Glc;
SA2-3Galβ1-4GlcNAcβ1-3Galβ1-3(Fucα1-4)GlcNac;
SA2-6Galβ1-4GlcNAcβ1-3Galβ1-3(Fucα1-4)GlcNac;
SA2-3Galβ1-4(Fucα1-3)GlcNAcβ1-3Galβ1-3(Fucα1-4)GlcNac;
SA2-6Galβ1-4(Fucα1-3)GlcNAcβ1-3Galβ1-3(Fucα1-4)GlcNAc;
SAα2-3Galβ1-4GlcNAcβ1-3Galβ1-3(Fucα1-4)GlcNAcβ1-3Galβ1-4Glc;
SAα2-6Galβ1-4GlcNAcβ1-3Galβ1-3(Fucα1-4)GlcNAcβ1-3Galβ1-4Glc;
SAα2-3Galβ1-4(Fucα1-3)GlcNAcβ1-3Galβ1-3(Fucα1-4)GlcNAcβ1-3Galβ1-4Glc;
SAα2-6Galβ1-4(Fucα1-3)GlcNAcβ1-3Galβ1-3(Fucα1-4)GlcNAcβ1-3Galβ1-4Glc;
SAα2-3Galβ1-4(Fucα1-3)GlcNAcβ1-3Galβ1-3(Fucα1-4)Glc;
SAα2-6Galβ1-4(Fucα1-3)GlcNAcβ1-3Galβ1-3(Fucα1-4)Glc;
SAα2-3Galβ1-4GlcNAcβ1-3Galβ1-3(Fucα1-4)GlcNAcβ1-3Galβ1-4(Fucα1-3)Glc;
SAα2-6Galβ1-4GlcNAcβ1-3Galβ1-3(Fucα1-4)GlcNAcβ1-3Galβ1-4(Fucα1-3)Glc;
SAα2-3Galβ1-4(Fucα1-3)GlcNAcβ1-3Galβ1-3(Fucα1-4)GlcNAcβ1-3Galβ1-4(Fucα1-3)Glc;
SAα2-6Galβ1-4(Fucα1-3)GlcNAcβ1-3Galβ1-3(Fucα1-4)GlcNAcβ1-3Galβ1-4(Fucα1-3)Glc.

[GlcNAcβ1-3Galβ1-4]$_n$GlcNAc-, wobei n eine Zahl aus der Reihe 1 bis 8 bedeutet;
[GlcNAcβ1-3Galβ1-4]$_n$GlcNAcβ1-4GlcNAc-, wobei n eine Zahl aus der Reihe 1 bis 8 bedeutet;
Galβ1-4[GlcNAcβ1-3Galβ1-4]$_n$GlcNAc-, wobei n eine Zahl aus der Reihe 1 bis 8 bedeutet;
Galβ1-3[GlcNAcβ1-3Galβ1-4]$_n$GlcNAc-, wobei n eine Zahl aus der Reihe 1 bis 8 bedeutet;
SAα2-6Galβ1-4[GlcNAcβ1-3Galβ1-4]$_n$GlcNac-, wobei n eine Zahl aus der Reihe 1 bis 8 bedeutet;
SAα2-3Galβ1-4[GlcNAcβ1-3Galβ1-4]$_n$GlcNac-, wobei n eine Zahl aus der Reihe 1 bis 8 bedeutet;
SAα2-3Galβ1-3[GlcNAcβ1-3Galβ1-4]$_n$GlcNAc-, wobei n eine Zahl aus der Reihe 1 bis 4 bedeutet;
Galβ1-4(Fucα1-3)GlcNAcβ1-3[Galβ1-4(Fucα1-3)$_m$-GlcNAcβ1-3]$_n$-Galβ1-4GlcNAc-, wobei m eine Zahl aus der Reihe 0 bis 1 bedeutet und wobei n eine Zahl aus der Reihe 1 bis 4 bedeutet;
Galβ1-3(Fucα1-4)GlcNAcβ1-3[Galβ1-4(Fucα1-3)$_m$-GlcNAcβ1-3]$_n$-Galβ1-4GlcNAc-, wobei m eine Zahl aus der Reihe 0 bis 1 bedeutet und wobei n eine Zahl aus der Reihe 1 bis 8 bedeutet;
SAα2-3Galβ1-3(Fucα1-4)GlcNAcβ1-3[Galβ1-4GlcNAcβ-3]$_n$-Galβ1-4GlcNA-, wobei n eine Zahl aus der Reihe 1 bis 8 bedeutet;
SAα2-3Galβ1-4(Fucα1-3)GlcNAcβ1-3[Galβ1-4(Fucα1-3)$_m$-GlcNAcβ1-3]$_n$-Galβ1-4GlcNAc-, wobei m eine Zahl aus der Reihe 0 bis 1 bedeutet und wobei n eine Zahl aus der Reihe 1 bis 2 bedeutet.

Galβ1-4[GlcNAcβ1-3Galβ1-4]$_n$GlcNAcβ1-4GlcNAc-, wobei n eine Zahl aus der Reihe 1 bis 8 bedeutet;
Galβ1-3[GlcNAcβ1-3Galβ1-4]$_n$GlcNAcβ1-4GlcNAc-, wobei n eine Zahl aus der Reihe 1 bis 8 bedeutet;
SAα2-6Galβ1-4[GlcNAcβ1-3Galβ1-4]$_n$GlcNAcβ1-4GlcNAc-, wobei n eine Zahl aus der Reihe 1 bis 8 bedeutet;
SAα2-3Galβ1-4[GlcNAcβ1-3Galβ1-4]$_n$GlcNAcβ1-4GlcNac-, wobei n eine Zahl aus der Reihe 1 bis 8 bedeutet;
SAα2-3Galβ1-3[GlcNAcβ1-3Galβ1-4]$_n$GlcNAcβ1-4GlcNac-, wobei n eine Zahl aus der Reihe 1 bis 8 bedeutet;
Galβ1-4(Fucα1-3)GlcNAcβ1-3[Galβ1-4(Fucα1-3)$_m$GlcNAcβ1-4]$_n$-Galβ1-4GlcNAcβ1-4GlcNAc-, wobei m eine Zahl aus der Reihe 0 bis 1 bedeutet und wobei n eine Zahl aus der Reihe 1 bis 4 bedeutet;
Galβ1-3(Fucα1-4)GlcNAcβ1-3[Galβ1-4(Fucα1-3)$_m$GlcNAcβ1-3]$_n$-Galβ1-4GlcNAcβ1-4GlcNAc-, wobei m eine Zahl aus der Reihe 0 bis 1 bedeutet und wobei n eine Zahl aus der Reihe 1 bis 4 bedeutet;
SAα2-3Galβ1-3(Fucα1-4)GlcNAcβ1-3[Galβ1-4GlcNAcβ1-3]$_n$-Galβ1-4GlcNAcβ1-4GlcNAc-, wobei n eine Zahl aus der Reihe 1 bis 6 bedeutet;
(GlcNAcβ1-3Galβ1-4)$_n$GlcNAcβ1-3Gal, wobei n eine Zahl aus der Reihe 1 bis 8 bedeutet;
SAα2-6Gal-;
SAα2-6Galβ1-4(GlcNAcβ1-3Galβ1-4)$_n$GlcNAcβ1-3Gal, wobei n eine Zahl aus der Reihe 1 bis 10 bedeutet;
SAα2-3Gal-; and

SAα2-3Galβ1-4(GlcNAcβ1-3Galβ1-4)$_n$GlcNAcβ1-3Gal-, wobei n eine Zahl aus der Reihe 1 bis 10 bedeutet.

Beispiele für bevorzugte Ausführungsformen des Kohlenhydratanteils sind: Sialyl-Lewis X, Sialyl-Lewis A, VIM-2 sowie die folgenden Blutgruppendeterminanten Lewis A, B, X, Y und A-Typ$^1$, A-Typ$^2$, B-Typ$^1$, B-Typ$^2$ und H-Typ$^1$, H-Typ$^2$ (R.U. Lemieux, Chem. Soc. Rev., 1978, S. 423 und 1989, S. 347)

Beispiele für die besonders bevorzugte Ausführungsform des Kohlenhydratanteils sind Sialyl-Lewis X, Sialyl-Lewis A oder VIM-2.

Die Formel von Sialyl-Lewis X lautet: NeuNAcα2-3Galβ1-4(Fucα1-3)GlcNAc und von Sialyl-Lewis A: NeuNAcα2-3Galβ1-3(Fucα1-4)GlcNAc. Die Formel von VIM-2 lautet:
NeuNAcα2-3Galβ1-4GlcNAcβ1-3Galβ1-4(Fucα1-3)GlcNAc;

SPACER:

Der Spacer ist eine physiologisch verträgliche, natürliche oder synthetische Verbindung, die die Aufgabe der räumlichen Trennung von Kohlenhydratanteil und biodegradabelem Polymer hat. Diese räumliche Trennung ist notwendig, um sterische Wechselwirkungen zwischen Kohlenhydratanteil und Polymer zu verhindern und damit eine optimale Zugänglichkeit des Liganden (Kohlenhydratanteils) für einen Rezeptor zu gewährleisten.

Um eine Kopplung des Spacers an den Kohlenhydratanteil bzw. das Polymer zu gewährleisten, muß der Spacer bifunktionell sein.

Der Begriff "bifunktioneller Spacer" wird wie folgt definiert:
Ein bifunktioneller Spacer trägt an beiden Enden reaktive, funktionelle Einheiten, die zur Verknüpfung des Spacers mit dem Polymeranteil einerseits und dem Kohlenhydratanteil andererseits unter Ausbildung einer kovalenten Bindung verwendet werden.

Der Spacer kann ein natürlich vorkommendes Molekül oder ein synthetisches Molekül sein. Unter "synthetisch" versteht man Moleküle, die in der Natur nicht vorkommen.

Grundsätzlich hat der Spacer die folgende Formel:
(Kohlenhydratanteil)-$A_1$-[B-$C_x$]$_y$-$D_z$-$A_2$-(biodegradables, hydrophiles Polymer-Wirkungsverstärker), wobei
x für 0 oder 1;
y für 1, 2, 3, 4, 5, 6 oder 7;
z für 0 oder 1 steht, mit der Maßgabe, daß z nur 1 sein kann, wenn x = 1 ist;
B und D gleich oder verschieden sein können und Alkylen mit 0 bis
30 C-Atomen,
vorzugsweise 0 bis 15 C-Atome und besonders bevorzugt 0 bis 10 C-Atome bedeuten, die geradkettig, verzweigt oder cyclisch sein können, unsubstituiert oder substituiert sind, Einfach- oder/und Doppelbindungen oder 1 bis 3 aromatische Ringe enthalten können.

B und D können unabhängig voneinander die folgenden Substituenten bevorzugt enthalten: Hydroxy-, Alkoxy-, Carboxy-, Amino- oder Carboxyalkylgruppen, der Formel $CO_2$-$R^1$, wobei $R^1$ = H oder $C_1$-$C_6$-Alkyl, vorzugsweise $R^1$ = H oder $C_1$-$C_4$-Alkyl und besonders bevorzugt $R^1$ = H oder $C_1$-$C_2$-Alkyl bedeutet;
C einen Substituenten der folgenden Formel bedeutet:

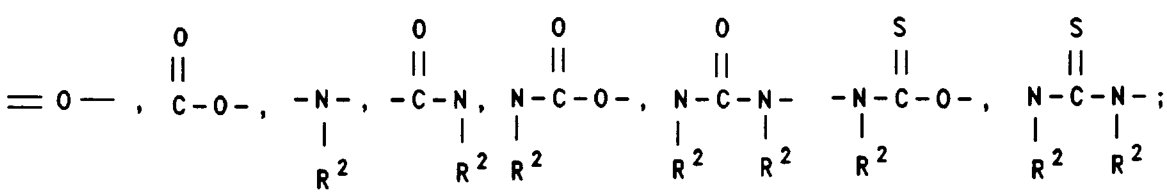

Bevorzugt sind =

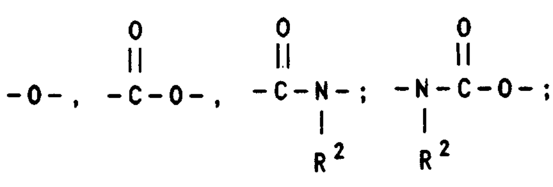

wobei $R^2$ Wasserstoff, Methyl, Ethyl, Benzyl, Propyl, Acetyl oder Benzoyl unabhängig voneinander bedeuten kann.

Die durch den Substituenten y definierten, repetitiven Einheiten können gleich oder verschieden sein.

$A_1$ und $A_2$ können gleich oder verschieden sein.

$A_1$ /$A_2$ ist als Teil des Spacers definiert, der den Spacer mit dem Kohlenhydratanteil und mit dem biodegradabelen, hydrophilen Polymer verbindet.

$A_1$/$A_2$ entstehen aus der kovalenten Verknüpfung von je einer reaktiven mit einer aktivierten Gruppe.

Die Begriffe "reaktive Gruppe" und "aktivierte Gruppe" werden wie folgt definiert:

"reaktive Gruppen" sind funktionelle Gruppen, die als Donor fungieren, bevorzugt OH-, $NH_2$- oder SH-Funktionen, die mit "aktivierten Gruppen" unter Ausbildung einer kovalenten Bindung reagieren. Besonders bevorzugt sind $NH_2$- oder die OH-Gruppe.

"aktivierte Gruppen" sind funktionelle Gruppen, die als Akzeptor fungieren und mit der "reaktiven Gruppen" unter Ausbildung einer kovalenten Bindung reagieren. Vorzugsweise verwendet werden Bromide, Jodide, Aktivester, besonders bevorzugt p-Nitrophenyl- oder N-Hydroxysuccinimid-Derivate. Außerdem bevorzugt werden Carbonsäurechloride und Carbonsäureimidazolide, gemischte Carbonsäureanhydride, besonders bevorzugt aus den folgenden Einheiten:

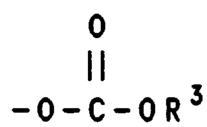

wobei $R^3$ = $C_1$-$C_6$ bedeutet, gerade oder verzweigt, sowie Phenylreste. Vorzugsweise verwendet werden Aldehyde, Acrylester, -amide, Malonimid, Succinimid, 2-Vinylpyridin, Jodessigester, Isothiocyanat und/oder Isocyanat. Besonders bevorzugt sind N-Hydroxysuccinimid-Aktivreste, Maleinimid, Succinimid, Acrylamide, Aldehyd oder Isocyanat.

Reaktive Gruppen sind Bestandteile des Polymers, da das Polymer aus Monomeren aufgebaut ist, die reaktive Gruppen enthalten.

Aktivierte Gruppen sind entweder bereits als ein Teil des Polymers vorhanden oder können nach dem Fachmann bekannten Verfahren (siehe u. a. R. C. Larock: Comprehensive Organic Transformations, VCK-Verlagsgesellschaft, 1989, Weinheim/Deutschland) aus reaktiven Gruppen, die bereits Teil des Polymers sind, hergestellt werden.

HYDROPHILES, BIODEGRADABLES POLYMER:

Polymere, die in ihrer Funktion als Träger Bestandteil von Kohlenhydratrezeptorblockern sind und diagnostische oder therapeutische Verwendung finden sollen, müssen grundsätzlich eine Reihe von Anforderungen erfüllen, um physiologisch gut verträglich zu sein:

a) keine Immunanwort zu induzieren und

b) um unspezifische Wechselwirkungen und Kumulation im RES (Retikuloendotheliales System) zu vermeiden

Definitionsgemäß besteht das biodegradable, hydrophile Polymer aus wenigstens zwei gleichen oder verschiedenen Monomerbausteinen, die linear, verzweigt, ringförmig (z. B. Makrocyclen), sternförmig (Dendrimere) oder kugelförmig (z. B. Fullerene) miteinander verknüpft sind und eine Molekulargewichtsverteilung aufweisen können.

Für die Herstellung polymerer Kohlenhydratrezeptorblocker geeignete Polymere sind: Polyamide, Polycarbonate, Polyiminocarbonate, Polyanhydride, Polyorthoester, Polyester, Polydioxanone, Polyhydroxycarbonsäuren, Polyaminosäuren, Polyphosphazene sowie Polysaccharide.

Das biodegradable, hydrophile Polymer ist vorzugsweise eine Polyaminosäure, eine Polyhydroxycarbonsäure als Polyester, Polyamid oder -anhydrid verknüpft oder ein Polysaccharid vorzugsweise mit einem Molekulargewicht ≦ 70 kd. Vorzugsweise hat das Polymer eine Mindestgröße von 2 kd, um im Vergleich zu niedermolekularen Trägern eine erhöhte Verweilzeit im Blut zu erzielen.

Für die Herstellung polymerer Kohlenhydratrezeptorblocker besonders bevorzugt geeignete Polymere sind die zu den Polyaminosäuren gehörende Polyaspartatamide, Polysuccinimide, Polyglutamate, Polylysinfumaramide, die zu den Polyhydroxycarbonsäuren gehörenden funktionellen Polyhydroxycarbonsäuren auf

Basis der Apfelsäure, Weinsäure oder Zitronensäure in Form ihrer Polyester, Polyamide oder Polyanhydride sowie substituierte Chitosane, Heparine, Hyaluronsäuren oder Stärkederivate.

Der Belegungsgrad des Polymers mit dem Kohlenhydratanteil (via Spacer) liegt zwischen 0,5-50 %, vorzugsweise zwischen 2-25 %.

Die Belegdichte des Polymermoleküls variiert je nach dem zu blockierenden Rezeptormolekül:

- Ein Polymer kann Träger mehrerer, über Spacer gekoppelte Kohlenhydratanteile sein oder nur einen einzigen Kohlenhydratanteil tragen. Die Kohlenhydratanteile können gleich oder verschieden sein. Die Anzahl der vom Polymer getragenen Spacer-Kohlenhydratanteil-Komplexe richtet sich nach der medizinischen Verwendung und Wirksamkeit des erfindungsgemäßen Kohlenhydratrezeptorblockers.
- Mehrere dieser Kohlenhydratanteile-tragenden Polymere können miteinander verbunden sein.

WIRKUNGSVERSTÄRKER:

Der Wirkungsverstärker ist eine Verbindung, die kovalent mit dem Komplex Kohlenhydratanteil-Ligand-Polymer verknüpft ist. Diese Verbindung ist ein intramolekularer Vernetzer, eine hydrophobe, hydrophile oder ionische Verbindung oder auch ein Löslichkeitsverbesserer. Er hat die Aufgabe, die Konformation des Polymers zu beeinflußen, um eine verbesserte Präsentation des Liganden auf dem Polymer und damit auch eine verbesserte Zugänglichkeit des Liganden zum Rezeptor zu erzielen. Außerdem kann mit Hilfe des Wirkungsverstärkers die mangelnde Löslichkeit des Kohlenhydratrezeptorblockers verbessert weden.

Intramolekulare Vernetzer tragen an beiden Enden reaktive, funktionelle Einheiten, die zur kovalenten Verknüpfung mit zwei reaktiven, funktionellen Einheiten, die Teil des Polymers sind, verwendet werden.

Ein intramolekularer Vernetzer hat die folgende Formel:
(hydrophiles, biodegradables Polymer)

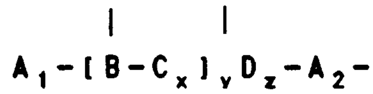

wobei

- x für 0 oder 1;
- y für 1, 2;
- z für 0 oder 1 steht, mit der Maßgabe, daß z nur 1 sein kann, wenn x = 1 ist;

B oder D gleich oder verschieden sind und Alkylen, mit 0-20 C-Atomen, vorzugsweise 0-15 C-Atome und besonders bevorzugt 0-10 C-Atome bedeuten, die geradkettig, verzweigt oder cyclisch sein können, unsubstituiert oder substituiert sind, Einfach- oder/und Doppelbindungen oder 1-2 aromatische Ringe enthalten können. Bevorzugte Substituenten für B oder D sind außerdem Hydroxy-, Alkoxy-, Amino-, Carboxy- Carboxyalkylgruppen der Formel $CO_2R^4$, wobei $R^4$ = H oder $C_1$-$C_6$, besonders bevorzugt $C_1$-$C_4$ und ganz besonders bevorzugt $C_1$-$C_2$ oder vorzugsweise Carbonylamino- und Carbonylaminoalkylgruppen der Formel $CONR^5R^6$, wobei $R^5$ und $R^6$ gleich oder verschieden sein kann und H oder $C_1$-$C_4$-Alkyl, besonders bevorzugt R = H oder $C_1$-$C_2$-Alkyl bedeutet;

C einen Substituenten der folgenden Formel bedeutet:

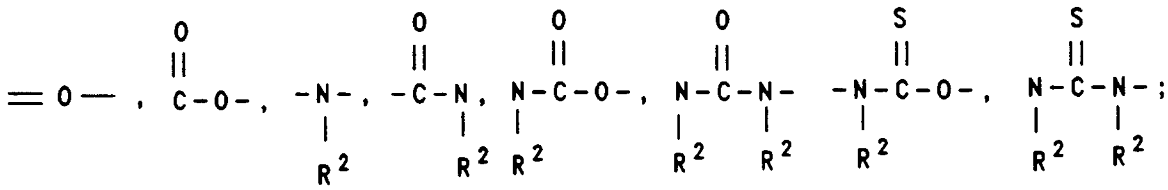

wobei $R^7$ Wasserstoff, Methyl, Ethyl, Benzyl, Propyl, Acetyl, Benzoyl unabhängig voneinander bedeuten kann.

Bevorzugt sind =

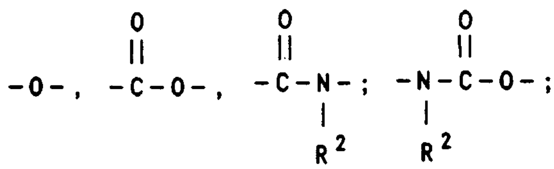

$A_1$ und $A_2$ haben die schon für den Spacer genannten Bedeutungen.

Die durch den Substituenten y definierten, respektiven Einheiten können gleich oder verschieden sein.

Hydrophobe, hydrophile und ionische bzw. ionisierbare Gruppen sind Substituenten, die eine reaktive, funktionelle Einheit tragen und die mit einer weiteren reaktiven, funktionellen Einheit des Polymeranteils unter Ausbildung einer kovalenten Bindung reagieren. Die Anzahl der eingesetzten Gruppen hängt davon ab, inwieweit die Konformation des Polymer beeinflußt werden soll.

Hydrophobe Substituenten sind Moleküle mit einer hydrophoben Oberfläche, die eine reaktive, funktionelle Einheit tragen, wie z. B. Alkohole, Amine, Aktivester oder Säurechloride des Cyclododecans, Adamantan, 2-Methylpropans, 2,2-Dimethylpropans und der Fullerene.

Hydrophile Substituenten sind Moleküle mit einer hydrophilen Oberfläche, die eine reaktive, funktionelle Einheit tragen, wie z. B. Cyclodextrin, Mono- und Oligosaccharide.

Ionische bzw. ionisierbare Substituenten sind Moleküle, die polare, ionische bzw. ionisierbare Gruppen enthalten und eine reaktive, funktionelle Einheit tragen,w ie z. B. Purine, Pyridine, Lysine und Verbindungen mit mehreren Carbon-, Phosphon- oder Sulfonsäuregruppen an einer kurzen Alkylkette ($C_1$-$C_8$), die verzweigt, unverzweigt oder cyclisch sein kann und eine reaktive Gruppe enthält, wie z. B. Weinsäure und Asparaginsäure.

Löslichkeitsverstärker tragen eine reaktive, funktionelle Einheit, die zur kovalenten Verknüpfung mit einer reaktiven, funktionellen Einheit, die Teil des Polymers ist, verwendet wird. Sie sind polare, gut wasserlösliche Verbindungen wie Polyole, Polyethylenglykole, Aminoalkohole oder Verbindungen mit mehreren Carbon-, Phosphon- oder Sulfonsäuregruppen an einer kurzen Alkylkette, vorzugsweise $C_1$-$C_8$, die verzweigt, unverzweigt oder cyclisch sein kann.

Zusätzlich zu den o.g.Verbindungen ist es möglich, Substituenten in Form einer Markergruppe und/oder eines Pharmakons an das Polymer zu koppeln.

Die Markergruppe ermöglicht den Einsatz des Kohlenhydratrezeptorblockers für in vivo Diagnosen. Die Kopplung des Markers an das Polymer erfolgt über kovlente Bindungen. Es können alle dem Fachmann bekannten Marker für in vivo Diagnosen hierfür verwendet werden, wie z. B. radioaktive Marker, die ein gebundenes Radionuklid enthalten, z. B. Technetium, Röntgenkontrastmittel, die z. B. eine jodierte Verbindung beinhalten sowie Kernresonanzkontrastmittel, z. B. auf Basis von Gadoliniumverbindungen. Der Anteil des Markers am Gesamtmolekül beträgt < 1 %.

Es wird jeweils das Pharmakon an das Polymer gekoppelt, von dem bekannt ist, daß es die zu behandelnde Erkrankung therapiert. Die Kopplung des Pharmakons an das Polymer erfolgt über kovalente oder ionischen Bindungen.

Als Pharmakon können:

- Antitumormittel, wie z. B. Daunomycin, Doxorubicin, Vinblastin, Bleomycin;
- Antibiotika, wie z. B. Penecilline, Erythromycine, Azidamfenicol, Cephalotin und Griseofulvin;
- Immunmodulatoren, wie z. B. FK-506, Azthioprin, Levamisol;
- Antagonisten des Blutplättchenaktivierungsfaktoren;
- Leukotrien Antagonisten;
- Inhibitoren des Cyclooxygenase-Systems, wie z. B. Salicylsäureverbindungen;
- Lipoxygenase-Inhibitoren,
- Antiphlogistica, wie z. B. Indomethacin;
- Antirheumatica, wie z. B. Nifenazon,

verwendet werden.

Werden die Rezeptorblocker als Anti-Adhäsionstherapeutika eingesetzt, so sollen sie im Fall von Entzündungen verhindern, daß die ELAM-1-Rezeptoren auf stimulierten Endothelzellen an Sialyl Lewis-X-Strukturen auf der Oberfläche von Leukozyten binden. Im Fall der Influenza-Therapie verhindern die Kohlenhydratrezeptorblocker die Anheftung von Viren an die Neuraminsäure auf der Zelloberfläche und damit auch die Endozytose der Viruspartikel.

Die Kohlenhydratrezeptorblocker sind in der Lage, mit allen natürlich vorkommenden Rezeptoren, die in vivo spezifisch den Kohlenhydratanteil von Liganden erkennen, zu reagieren.

Vorzugsweise handelt es sich hierbei um Rezeptoren, die an der Zelloberfläche exprimiert werden, z. B. von Säugerzellen, einschl. menschlicher Zellen, Bakterienzellen oder Viren. Auch bevorzugt sind Hormone und Toxine, erkennende Rezeptoren, die Hormone oder Toxine erkennen.

Besonders bevorzugt sind solche Zelloberflächenrezeptoren, die zur Klasse der Selektine gehören.

Ganz besonders bevorzugt werden die bei entzündlichen Erkrankungen exprimierten Rezeptoren, beispielsweise Leu-8 (= L-Selektin = gp90$^{mel}$ = LAM-1 = LEC-CAM-1), ELAM-1 (= E-Selektin) und GMP-140 (= P-Selektin = CD62 = PADGEM).

## HERSTELLUNG DES ERFINDUNGSGEMÄßEN

## KOHLENHYDRATREZEPTORBLOCKERS:

Der erfindungsgemäße Kohlenhydratreceptorblocker besteht aus Kohlenhydratanteil, Spacer, Polymer und Wirkungsverstärker.

Grundsätzlich erfolgt die Synthese des Kohlenhydratrezeptorblockers durch Bildung einer kovalenten Bindung zwischen Kohlenhydratanteil und bifunktionellem Spacer, anschließender kovalenten Verknüpfung des Komplexes Kohlenhydratanteil-Spacer mit dem Polymer und abschließender kovalenter Verknüpfung mit dem Wirkungsverstärker.

Alternativ kann die Verknüpfung in anderer Reihenfolge erfolgen:

- Umsetzungen des Polymers mit dem Wirkungsverstärker einerseits und des Kohlenhydratanteils mit dem bifunktionellen Spacer andererseits erfolgen unter Ausbildung kovalenter Bindungen. Der fertige Kohlenhydratrezeptorblocker entsteht durch kovalente Verknüpfung der komplexe Polymer-Wirkungsverstärker mit dem Kohlenhydratanteil-Spacer.
- Durch kovalente Verknüpfung von Polymer und Wirkungsverstärker entsteht eine Verbindung, die unter Ausbildung einer kovalenten Bindung mit dem Spacer umgesetzt wird. Wird diese Verbindung kovalent mit dem Kohlenhydratanteil umgesetzt, so erhält man den erfindungsgemäßen Kohlenhydratrezeptorblocker.
- Die Synthese des Kohlenhydratrezeptorblockers kann auch so erfolgen, daß ein Komplex, der durch kovalente Verknüpfung des Polymers mit dem bifunktionellen Spacer entstanden ist, zuerst kovalent mit dem Kohlenhydratanteil verknüpft wird und anschließend mit dem Wirkungsverstärker unter Ausbildung einer kovalenten Bindung umgesetzt wird.
- Eine weitere Alternative zur Synthese des Kohlenhydratrezeptorblockers stellt die kovalente Verknüpfung des Polymers mit dem Spacer im ersten Reaktionsschritt, gefolgt von kovalenten Verknüpfungen mit dem Wirkungsverstärker im nächsten und mit dem Kohlenhydratanteil im letzten Schritt dar.

Bevorzugt ist der erstgenannte Syntheseweg und der erstgenannte, alternative Syntheseweg.

Die Synthese des Kohlenhydratrezeptorblockers unter kovalenter Verknüpfung seiner Bestandteile erfolgt durch Reaktion von reaktiven mit aktivierten Gruppen. Die Verteilung von reaktiven und aktivierten Gruppen auf die vier Bestandteile ist dabei für die generelle Durchführbarkeit der Synthese nicht von Bedeutung.

Die Reaktion zwischen reaktiver und aktivierter Gruppe erfolgt nach dem Fachmann bekannten Verfahren durch Veresterung, Amidierung, Addition an eine Doppelbindung und Alkylierung (R.C. Larock: Comprehensive Organic Transformation, VCH-Verlagsgesellschaft, Weinheim, Deutschland, 1989, L.-F. Tietze und Th. Eichler, Reaktionen und Synthesen im Org.-chem. Praktikum, G. Thieme Verlag, Stuttgart, Deutschland, 1981).

## KOHLENHYDRATANTEIL UND SEINE VERKNÜPFUNG MIT SPACER:

Der Kohlenhydratanteil, der über einen Spacer kovalent mit dem biodegradablen Polymer, das einen Wirkungsverstärker trägt, verknüpft ist, kann aus natürlichen Quellen stammen, chemisch, chemoenzymatisch oder enzymatisch hergestellt werden.

Geeignete natürliche Quellen für Kohlenhydrate sind dem Fachmann bekannt und können in der biochemischen Literatur nachgelesen werden (z. B. Biochemistry, Biol. Chem. Hoppe-Seyl. J. Biol. Chem. Biochemistry and Cell Biology, Cell). Dort werden ebenfalls etablierte, dem Fachmann bekannte Verfahren zur Aufreingung von Oligosacchariden beschrieben.

Kohlenhydrate, die aus natürlichen Quellen stammen, liegen grundsätzlich mit "freiem reduzierenden Ende" vor.

Verfahren zur chemischen, enzymatischen oder chemoenzymatischen Synthese von Kohlenhydraten, die von Zelloberflächenrezeptoren erkannt werden, sind dem Fachmann aus der chemischen Literatur sowie

aus Übersichtsartkeln bekannt. Für die chemische Synthese z. B. Carbohydrate Research, Elsevier Science Publishers B. U., Amsterdam, Journal of Carbohydrate Chemistry, Marcel Dekker, Inc., New York; H. Paulsen, Angewandte Chemie 1982 (94) 184; R. R. Schmidt, Angewandte Chemie, 1987 (98) 213; H. Kunz, Angewandte Chemie, 1987 (44) 247; H. Paulsen, Angewandte Chemie, 1990 (102) 851). Für die enzymatische Synthese z. B. Carbohydrate Research, Elsevier Science Publishers B. U., Amsterdam, Journal of Carbohydrate Chemistry, Marcel Dekker, Inc., New York; Advances in Carbohydrate Chemistry and Biochemistry, David, S. et al., Vol. 49, 1991, S. 175; Applied Biocatalasis, Nielsson, K.G.I., Vol. 1, 1991, S. 117 ff.; Synthesis, Drueckhammer, D.G. et al., 1991, S. 499 ff.).

Unter chemoenzymatischer Synthese versteht man die Kombination chemischer und enzymatischer Reaktionsschritte zur Synthese des Kohlenhydratanteils bzw. einer kovalenten Verbindung von Kohlenhydratanteil und Spacer.

Synthetisch hergestellte Oligosaccharide können sowohl mit freiem reduzierenden Ende als auch in einer Spacer-verknüpften Form erhalten werden. Die Einführung des Spacers erfolgt nach dem Fachmann bekannten Verfahren zur chemischen oder enzymatischen Glycosylierung (s. o.). Bevorzugt werden solche Herstellungsverfahren verwendet, bei denen ein Oligosaccharid entsteht, das bereits durch kovalente Bindung mit dem Spacer verknüpft ist.

Die Kopplung des Kohlenhydratanteils mit freiem reduzierenden Ende an den Spacer erfolgt über eine kovalente Bindung. Folgende Verfahren zur Kopplung sind durchführbar:

1) Das Oligosaccharid mit freiem reduzierenden Ende wird z. B. analog zu dem Verfahren von Lundquist et al. (Journal of Carbohydrate Chemistry, 1991 (10) 377) in das freie 1-Aminoglycosid überführt, das im Anschluß daran durch Acylierung kovalent mit einem Spacer verknüpft wird. Die Acylierung kann z. B. analog zu den Verfahren von Kochetkow (Carbohydrate Research 1986 (146) C1) unter Verwendung eines N-Hydroxysuccinimid-Aktivesters als aktivierte Gruppe am Spacer mit diesen kovalent verknüpft werden.

2) Das freie reduzierende Ende des Oligosaccharids kann analog zu dem Verfahren Isebell et al. in Methods of Carbohydrate Chemistry, Academic Press, New York, von mit Jod und Kaliumhydroxid in ein Lacton überführt werden. Dieses Lacton kann z. B. analog nach dem Verfahren von Isebell et al. in Methods of Carbohydrate Chemistry, Academic Press, New York, mit einer primären Aminogruppe, die Bestandteil des Spacers ist, mit diesem kovalent verknüpft werden.

3) Die Knüpfung einer kovalenten Bindung zwischen den reduzierenden Ende eines Oligosaccharids und dem Spacer gelingt auch durch reduktive Aminierung mit einem Spacer, der an dem entsprechenden Ende eine primäre Aminofunktion aufweist, z. B. analog Lane, Synthesis, 1975, 135.

4) Enthält das Oligosaccharid an seinem reduzierenden Ende einen Aminozucker mit freier Aminogruppe so kann diese z. B. analog zu dem Verfahren von Kochetkow (s. o.) mit einem N-Hydroxysuccinimid-Aktivester des Spacers mit diesem kovalent verknüpft werden.

SPACER:

Die kovalente Verknüpfung des Polymers mit dem Spacer bzw. mit einer Verbindung bestehend aus kovalent verknüpftem Spacer und Kohlenhydrat sowie einer kovalenten Verbindung aus Polymer und Wirkungsverstärker mit dem Spacer bzw. mit einer Verbindung bestehend aus kovalent verknüpftem Spacer und Kohlenhydrat erfolgt durch Reaktion zwischen einer reaktiven Gruppe und einer aktivierten Gruppe. Dabei können sich sowohl die reaktive Gruppe am Ende des Spacers bzw. einer Verbindung bestehend aus kovalent verknüpftem Spacer und Kohlenhydrat und die aktivierte Gruppe auf Seiten des Polymers bzw. einer Verbindung bestehend aus kovalent verknüpftem Polymer und Wirkungsverstärker als auch die aktivierte Gruppe am Ende des Spacers bzw. einer Verbindung bestehend aus kovalent verknüpftem Spacer und Kohlenhydrat und die reaktive Gruppe auf Seiten des Polymers bzw. einer Verbindung bestehend aus kovalent verknüpftem Polymer und Wirkungsverstärker befinden.

Die Reaktion zwischen einer reaktiven Gruppe und einer aktivierten Gruppe erfolgt nach dem Fachmann bekannten Verfahren zur Veresterung, Amidierung, Addition an eine Doppelbindung und Alkylierung. Diese Verfahren sind dem Fachmann aus der Literatur bekannt (z. B. Larock, R.C., Comprehensive Organic Transformations, 1989, VCH Verlagsgesellschaft Weinheim, Deutschland; L.-F. Tietze und Th. Eicher, Reaktionen und Synthesen im Org.-chem. Praktikum, G. Thieme Verlag, Stuttgart, Deutschland, 1981).

BIODEGRADABLES HYDROPHILES POLYMER:

Die Herstellung von biodegradablem, hydrophilen Polymeren erfolgt nach dem Fachmann bekannten Verfahren. Diese sind z. B. beschrieben in: H.G. Elias, Makromoleküle, Bd. 1 und 2, Hüthig & Wepf Verlag,

Basel, Schweiz, 1991/92 oder D. Braun, H. Cherdron, W. Kern, Praktikum der Makromolekularen, Organischen Chemie, Hüthig-Verlag, 1979.

WIRKUNGSVERSTÄRKER:

Der Wirkungsverstärker ist eine Verbindung, die kovalent mit dem Polymeranteil des Kohlenhydratrezeptorblockers verknüpft ist.
Seine Aufgabe besteht darin, die Struktur des Gesamtmoleküls so zu beeinflussen, daß der Kohlenhydratanteil für den Rezeptor (optimal) zugänglich ist, und die in einigen Fällen auftretende mangelnde Löslichkeit zu verbessern.

Die kovalente Verknüpfung zwischen Wirkungsverstärker und Polymer erfolgt durch Reaktion einer reaktiven Gruppe mit einer aktivierten Gruppe. Die Reaktion zwischen reaktiven Gruppen und aktivierten Gruppen erfolgen nach dem Fachmann bekannten Verfahren zur Alkylierung, Acylierung oder Addition an eine Doppelbindung. Diese Verfahren sind dem Fachmann aus der Literatur bekannt. (Larock, R.C., Comprehensive Organic Transformations, 1989, VCH Verlagsgesellschaft Weinheim, Deutschland; L.-F. Tietze und Th. Eicher, Reaktionen und Synthesen im Org.-chem. Praktikum, G. Thieme Verlag, Stuttgart, Deutschland, 1981).

Die Herstellung des Kohlenhydratrezeptorblockers kann alternativ zu den oben genannten chemischen Synthesewegen auch über eine enzymatische bzw. chemoenzymatische Verknüpfung der Einzelkomponenten erfolgen. Als Enzyme werden Glykosidasen, Glykosyltransferasen, Transglykosidasen und/oder Lipasen verwendet. Glykosidasen, Glykosyltransferasen und/oder Transglykosidasen werden bevorzugt zum Aufbau des Kohlenhydratanteils sowie zur Verknüpfung von Kohlenhydrantanteil und Spacer eingesetzt. Lipasen, wie z. B. aus Pseudomonas, Candida, Mucor oder Rhizopus werden vorzugsweise zur Verknüpfung der reaktiven mit der aktivierten Gruppe verwendet. Als aktivierte Gruppen werden in diesem Fall eingesetzt: Carbonsäuren, Methylester und/oder Vinylester.

Die oben beschriebenen Verfahren zur Herstellung sind beispielhaft aufgeführte Ausführungsformen, die keinen Anspruch auf Vollständigkeit erheben. Demzufolge ist die Herstellung der erfindungsgemäßen Kohlenhydratrezeptorblocker nicht auf die genannten Verfahren beschränkt.

DIAGNOSTISCHE UND THERAPEUTISCHE VERWENDUNG DES ERFINDUNGSGEMÄßEN KOHLENHYDRATREZEPTORBLOCKERS:

Die Kohlenhydratrezeptorblocker dürfen bei in-vivo-Applikation keine nachteiligen Nebenwirkungen aufweisen. So sind im Falle intravenöser Anwendungen beispielsweise hämolytische und immunogene Eigenschaften zu vermeiden. Die Enzyme der Blutgerinnungskaskade dürfen nicht aktiviert werden, um die Bildung von Thromben auszuschließen. Außerdem sollen nicht genutzte Rezeptorblocker zu untoxischen Stoffen metabolisiert oder ausgeschieden werden.

Es hat sich außerdem gezeigt, daß für eine optimale therapeutische und/oder diagnostische Anwendung des Kohlenhydratrezeptorblockers sowohl die Länge des Spacers, der das Polymer mit dem Kohlenhydrat verknüpft, als auch der Belegungsgrad des Polymers mit dem Oligosaccharid innerhalb eines definierten Bereiches von 0,5 - 50 %, vorzugsweise von 2 - 20 %, liegen muß. Die Verweilzeit des Kohlenhydratrezeptorblockers ist über die Natur und Abbaubarkeit des Polymeranteils und das Molekulargewicht so steuerbar, daß die Lebensdauer des Kohlenhydratrezeptorblockers zwischen 1 Min., mehreren Stunden, bis zu wenigen Tagen betragen kann.

Der erfindungsgemäße Kohlenhydratrezeptorblocker und seine physiologisch verträglichen Salze eignen sich aufgrund ihrer wertvollen pharmakologischen Eigenschaften sehr gut zur Anwendung als Heilmittel bei Säugern, insbesondere dem Menschen. Die Arzneimittel eignen sich vorzugsweise zur Prophylaxe und/oder Therapie von bakteriellen und viralen Infektionen sowie von Krankheiten, die unter Beteiligung entzündlicher Prozesse ablaufen, vorzugsweise von Nachinfarktsyndrom, Schocklunge des Erwachsenen, Schock, Schlaganfall, akute und chronische Organabstoßung, Vasculitis, Sepsis, entzündliche Erkrankungen der Haut, rheumatische Arthritis sowie metastasierender Tumore.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig

verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Bei festen Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen.

Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche expirmiert werden und demzufolge die zu verabreichende Tagesdosis geringer ist als bei stark erkrankten Patienten.

Die erfindungsgemäßen Arzneimittel werden dadurch hergestellt, daß man dem Kohlenhydratrezeptorblocker mit üblichen Träger- sowie gegebenenfalls Zusatz- und/oder Hilfsstoffen in die bzw. eine geeignete Darreichungsform bringt.

## A: PRIMÄRASSAYS ZUR UNTERSUCHUNG DER WIRKUNG VON POLYMEREN KOHLENHYDRATREZEPTORBLOCKERN AUF DIE ZELLANHEFTUNG AN REKOMBINANTE LÖSLICHE SELEKTINFUSIONSPROTEINE

Mit diesem Assay wird die Wirkung von polymergebundenen Kohlenhydratbausteinen auf die Zellanheftung von promyelozytischen Zellen mittels Selektinen nachgewiesen:
Um die Wirksamkeit von polymergebundenen Kohlenhydratbausteinen auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### A1. HERSTELLUNG VON L-SELEKTIN-IGG1

Zur Herstellung von löslichem L-Selektion-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F.M., Brent, R., Kingston, R.E., Moore, D.D., Seidman, J.G., Struhl, K. und Smith, J.A. 1990. Current Protocols in Molecular Biology, John Wiley, New York.). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4 °C aufgehoben.

Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.

### A2. HERSTELLUNG VON P-SELEKTIN-IGG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo, A., Kolanus, W., Walz, G., Fredman, P. und Seed, B. 1991. CD62/P-Selectin recognition of myeloid and tumor cell sulfatides. Cell 67, 35-44.

A3. HERSTELLUNG VON CD4-IGG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1.

Zettelmeissl, G., Gregersen, J.-P.-, Duport, J.M., Mehdi, S., Reiner, G. und Seed, B. 1990. Expression and characterization of human CD4: Immunoglobulin Fusion Proteins. DNA and Cell Biology 9, 347-353.

A4. DURCHFÜHRUNG DES HL60 ZELLADHÄSIONSASSAYS AUF REKOMBINANTEN, LÖSLICHEN ADHÄSIONSMOLEKÜLEN

1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Std. bei Raumtemperatur inkubiert. Nach Entfernen der Antikörperlösung wird einmal mit PBS gewaschen.

2. 150 µl des Blockierungspuffers werden für 1 Std. bei Raumtemperatur in den Näpfchen belassen. Die Zusammensetzung des Blockierungspuffers ist:
0,1 % Gelatine, 1 % BSA, 5 % Kalbserum, 0,2 mM PMSF, 0,02 % Natriumazid. Nach Entfernen des Blockierungspuffers wird einmal mit PBS gewaschen.

3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.

4. In die Näpfchen werden 20 µl Bindunspuffer gegeben. Der Bindungspuffer hat die Zusammensetzung: 50 mM Hepes, pH 7,5; 100 mM NaCl; 1 mg/ml BSA;
2 mM MgCl2; 1 mM CaCl2; 3 mM MnCl2; 0,02 % Natriumazid; 0,2 mM PMSF. Dazu werden 5 µl der Testsubstanz pipettiert, durch Schwenken der Platte vermischt und 10 Min. bei Raumtempeartur inkubiert.

5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37 °C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.

6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl2; 1 mM CaCl2; 3 mM MnCl2; 0,02 % Natriumazid). Durch Invertierung wird der Stoppuffer aus den Näpfchen entfernt und die Prozedur noch zweimal wiederholt.

7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

B. SEKUNDÄRASSAY ZUR UNTERSUCHUNG DER WIRKUNG VON POLYMEREN KOHLENHYDRATREZEPTORBLOCKERN AUF DIE ZELLANHEFTUNG AN STIMULIERTE HUMANE ENDOTHELZELLEN

Der Test der Wirksamkeit von polymeren Kohlenhydratrezeptorblockern auf die Zelladhäsion an rekombinante, lösliche Fusionsproteine ist ein hochspezifischer Assay, der auf der Interaktion einer Art von Adhäsionsmolekülen mit den entsprechenden Liganden beruht. Um die in vivo Situation von Zell-Zell Interaktionen zu simulieren, verwenden wir einen Assay, bei dem HL60-Zellen an stimulierte, humane Nabeschnurendothelzellen adhärieren.

1. Gewinnung der humanen Nabelschnurendothelzellen (HUVEC).
Nabelschnüre werden nach der Geburt in PBS mit 100.000 IU/L Penicillin, 100 mg/L Streptomycin, 50 mg/L Gentamycin, 50.000 IU/L Mycostatin bei +4 °C bis zur Weiterverarbeitung aufgehoben.
Die längsten unverletzten Stücke werden aus der Nabeschnur mit einem Skalpell herausgeschnitten und auf frische Aluminiumfolie gelegt. Ein Ende der Nabelschnur wird mit einer Klemme verschlossen. Am anderen Ende inseriert man einen passenden Schlauch in die Nabelschnurvene und fixiert diesen durch Umbinden des Nabelschnurendes.

Die Vene wird durch das Schlauchstück mit Collagenaselösung gefüllt (50 mg Collagenase / 100 ml 25 mM Hepes, pH 7,0) und 15 Min. bei 37 °C inkubiert.

Um die Zellausbeute zu erhöhen, wird die Nabelschnur nach der Inkubation leicht massiert, um die noch anhängenden Endothelzellen abzulösen.

Anschließend läßt man die Zellsuspension aus der Vene in ein Kulturröhrchen mit Zellkulturmedium und 10 % Foetalem Kälberserum ausfließen. Die Vene wird mit PBS gewaschen, um die zurückgebliebenen Zellen zu erhalten.

Die Zellsuspension wird 5 Min. bei 500 g zentrifugiert; das Zellpellet anschließend in 4 ml Kulturmedium resuspendiert und die Zellen ausplattiert. Nach 3-4 Tagen sind die Zellen konfluent gewachsen und können passagiert werden.

Zur Überprüfung der Reinheit der Endothelzellkultur, wird die Kultur mit einem Antikörper gegen Faktor VIII für die Immunfluoreszenz gefärbt. Lediglich Endothelzellen, nicht jedoch kontaminierende Fibroblasten zeigen eine positive Reaktion.

2. Durchführung des Assays

20.000 Endothelzellen werden pro Näpfchen einer 96er Mikrotiterplatte ausplattiert und 24 Std. bei 37 °C inkubiert. Die Endothelzellen dürfen dazu nicht öfter als 5-6 mal passagiert worden sein. Vier Std. vor dem Assay werden die Endothelzellen durch Zugabe von Il-1 (finale Konzentration: 15 U/ml) stimuliert. Nach Entfernung des Kulturmediums werden die Zellen einmal mir RPMI-Medium ohne Serum gewaschen. Nach Entfernung und erneutem Pipettieren von 20 µl RPMI-Medium, erfolgt die Zugabe von Testsubstanzen.

3. Die weiteren Schritte des Assays, Markierung der HL60 Zellen, Aufbringen der HL60 Zellen, werden wie unter A4. Punkte 5. - 7. durchgeführt.

C. SEKUNDÄRASSAY ZUR UNTERSUCHUNG DER WIRKUNG VON POLYMEREN KOHLENHYDRATREZEPTORBLOCKERN AUF DIE ZELLANHEFTUNG AN GEFRIERSCHNITTE VON LYMPHATISCHEM GEWEBE

In vitro läßt sich untersuchen, in welchem Maße Leukozyten an Endothelzellen auf Gefrierschnitten lymphatischer Gewebe binden. Diese Zell-Zell-Interaktionen beruhen auf der Interaktion zwischen Adhäsionsmolekülen auf der Oberfläche der gefriergeschnittenen Endothelzellen und den entsprechenden Liganden auf der Oberfläche von Leukozyten. Stellvertretend für primäre Leukozyten lassen sich HL60-Zellen verwenden, deren Oberflächen-Liganden in der wissenschaftlichen Literatur gut beschrieben sind. Ob eine Anheftung von HL60-Zellen auf Lymphknoten-Gefrierschnitte stattfindet, läßt sich an der Zahl gebundener HL60-Zellen bestimmen.

1. Axilläre, cervicale oder mesenteriale Lymphknoten werden aus frisch getöteten Ratten präpariert und in flüssigem Stickstoff schnell eingefroren.

2. Von den gefrorenen Lymphknoten werden 10 µm dicke Cryostat-Schnitte angefertigt, auf runde Deckgläser (Durchmesser 18 mm) übertragen und 2 Std. bei Raumtemperatur getrocknet.

3. Auf die Schnitte werden 20 µl Bindungspuffer pipettiert. Die Testsubstanzen werden hinzugefügt und 10 Min. bei Raumtemperatur inkubiert. HL60-Zellen werden wie unter A4. Punkt 5. markiert. 200.000 markierte HL60-Zellen in 100 µl Bindungspuffer werden pro Deckglas hinzugefügt und 10 Min. stehen gelassen. Die sedimentierenden Zellen gelangen dabei auf Endothelzellen, an die sie teilweise anheften.

4. Im 45°-Winkel werden die Deckgläser in Stoppuffer getaucht um die nich tangehefteten Zellen abzuspülen. Anschließend werden die Deckgläser in 4 % Formaldehyd in PBS für 10 Min. bei Raumtemperatur fixiert.

5. Im Immunfluoreszenzmikroskop (FITC-Anregung) werden Querschnitte von lymphatischen Blutgefäßen photografisch dokumentiert. Die angehefteten HL60-Zellen heben sich deutlich vom nicht gefärbten Hintergrund ab. Das Ergebnis wird als gebundene HL60-Zellen pro Flächeneinheit Endothel ausgedrückt.

D. TERTIÄRASSAY ZUR UNTERSUCHUNG DER WIRKUNG VON POLYMEREN KOHLENHYDRATREZEPTORBOCKERN AUF DIE LEUKOZYTEN-ADHÄSION IN DER RATTE IN VIVO

Das nachfolgend aufgeführte Verfahren ist in der Lage, die Wirksamkeit von Substanzen in vivo festzustellen, die die Anheftung von Leukozyten an die Gefäßinnenwände hemmen.

Es ist bekannt, daß einige der zirkulierenden weißen Blutzellen die Tendenz besitzen, an den Innenwänden der Blutgefäße anzuheften. Diese Tendenz verstärkt sich erheblich bei Entzündungsvorgängen. Leukozyten stoßen normalerweise ständig gegen die Blutgefäßwände, diese Kollision ist jedoch elastisch, so daß die Zellen gewissermaßen zurückprallen und in die Zirkulation zurückgelangen. Bei Entzündungsvorgängen führen biochemische Veränderungen sowohl in den Leukozyten, aber auch in den die Blutgefäße auskleidenden Endothelzellen zu Veränderungen der Oberflächeneigenschaften beider Zellarten. Die Zellen verhalten sich adhäsiver. Diese Adhäsivität drückt sich zunächst in einer Tendenz der Leukozyten aus, nach Kollision mit dem Endothel auf den Endothelzellen zu rollen. Dieses Rollen der Leukozyten auf dem Endothel induziert weitere biochemische Reaktionen auf beiden Bindungspartnern in deren Folge die Zellanheftung verstärkt wird. Durch diese weitere Verstärkung der Adhäsivität verlangsamt sich das Rollen der Leukozyten, bis diese fest auf dem Endothel anhaften. Dem festen Anhaften folgt als weiterer Schritt das Auswandern der Leukozyten aus dem Blutgefäß.

Das Rollen der Leukozyten, die feste Anheftung und das Auswandern aus den Blutgefäßen läßt sich mit Leukozyten-stimulierenden Faktoren wie FMLP (f-Met-Leu-Phe), LPS (Lipopolysacchariden) oder TNF (tumor necrosis factor) induzieren. Eine mikroskopische Dokumentation dieser Vorgänge ist am präparierten Mesenterialgewebe, z. B. der Ratte, möglich. In die Blutbahn inkizierte Substanzen lassen sich daher daraufhin untersuchen, ob sie in der Lage sind, die induzierten Leukozyten-Adhäsionen zu beeinflussen.

Zur praktischen Durchführung dieser Untersuchung werden Ratten mit anaesthetisiert. Die Bauchhöhe wird eröffnet und ein Abschnitt des Dünndarms herausgezogen. Der Dünndarmabschnitt wird auf einem heizbaren Mikroskopiertisch ständig feucht gehalten. Zur mikroskopischen Betrachtung wird ein Bereich von Mesenterialgewebe mit Paraffinöl abgedeckt. Zur Kontrolle werden in diesem Bereich alle 5 Min. über einen Zeitraum von 30 Min. alle anhaftenden - nicht stimulierten - Leukozyten gezählt. Parallel dazu werden Blutdruck, Körpertemperatur und Fließgeschwindigkeit des Blutes aufgezeichnet. Die Testsubstanz wird durch kontinuerliche venöse Infusion während des gesamten Tests appliziert. Nach Aufbringen von Leukozyten-Stimulantien, die auf das Mesenterialgewebe getropft werden, werden die anheftenden Leukozyten alle 5 Min. über einen Zeitraum von 90 Min. gezählt.

Die Untersuchung erfolgt in Testgruppen, die aus jeweils drei Tieren bestehen. Das erste Tier erhält lediglich das Vehikel, um die spontane Adhäsion zu bestimmen. Das zweite Tier erhält lediglich die Leukozyten-Stimulation zur Bestimmung der pathogenen Kontrolle. Das dritte Tier erhält Leukozyten-Stimulanz und Testsubstanz.

Die Zahl der adhäsierenden Leukozyten in der pathogenen Kontrolle wird gleich 100 % gesetzt. Die prozentuale Änderung der Leukozyten-Adhäsion bei Testsubstanz Gabe gegenüber der pathogenen Kontrolle gibt die Wirksamkeit einer Testsubstanz an.

Im folgenden werden beispielhaft die Vorschriften für die Synthese beschrieben, ohne daß die Synthese des Kohlenhydratrezeptorblockers auf diese Vorschriften eingeschränkt wird.

MONOSACCHARID (Schema 1 und 7)

2-Amino-2-deoxyglucose-Hydrochlorid [1] wird entsprechend der Vorschrift von R. U. Lemieux et. al. (ACS Symp. Ser., 39, 90 (1976)) durch Umsetzung mit Phthalsäureanhydrid und anschließende Reaktion mit Acetanhydrid/Pyridin in 1,3,4,6-Tetraacetyl-2-N-acetyl-2-deoxyglucose [2] überführt.

Durch Behandlung mit Zinntetrachlorid/Thiophenol entsteht nach K. C. Nicolaou et. al. (J. Am. Chem. Soc. 112, 3695 (1990) das entsprechende 1-Thiophenylderivat [3]. Dieses wird nach der Vorschrift von B. A. Silwanis et. al. (J. Carbohydr. Chem. 10, 1067 (1991)) mit 6-Azidohexanol umgesetzt. Analog zu der Vorschrift von K. C. Nicolaou et. al. J. Am. Chem. Soc. 114, 3127 (1992)) erfolgt die Spaltung der Acetyl- und der Phthaloylschutzgruppen mit Hydrazinhydrat. Vor Abspaltung der Benzylschutzgruppen ($H_2$/Pd(OH)$_2$, MeOH) wird die freie Aminogruppe selektiv in Gegenwart der freien Hydroxylgrupen mit überschüssigem Acetanhydrid acetyliert. Es entsteht [4].

DISACCHARID (Schema 1, 2, 5 und 7)

1) Synthese eines geschützten N-Acetylglucosaminbausteins

2-Amino-2-deoxyglucose-Hydrochlorid [1] wird entsprechend der Vorschrift von R. U. Lemieux et. al. (ACS Symp. Ser., 39, 90 (1976)) durch Umsetzung mit Phthalsäureanhydrid und anschließende Reaktion mit Acetanhydrid/Pyridin in 1,3,4,6-Tetraacetyl-2-N-acetyl-2-deoxyglucose [2] überführt.

Durch Behandlung mit Zinntetrachlorid/Thiophenol entsteht nach K. C. Nicolaou et. al. (J. Am. Chem. Soc. 112, 3695 (1990)) das entsprechende 1-Thiophenylderivat [3]. Nach Abspaltung der Acetylschutzgruppen [5] werden die Benzyliden- und die Allylgruppe (Benzyldimethylacetal/CSA; Allylbromid/NaH) eingeführt. Durch Reduktion mit $NaCNBH_3$ wird die 4-OH-Gruppe freigesetzt [6].

2) Synthese eines geschützten Galactosebausteins

Galactose [7] wird entsprechend der Vorschrift von K. C. Nicolaou et. al. (J. Chem. Soc., Chem. Comm. 870 (1991)) durch Versetzen mit Acetanhydrid/Triethylamin/DMAP und Zinntetrachlorid/Thiophenol in das Thioglycosid [8] überführt. Nach Abspaltung der Acectylschutzgruppen entsteht [9] durch Umsetzung mit Benzaldehyddimethylacetal/CSA. Nach Acetylierung (Acetanhydrid/Triethylamin/DMAP) wird durch Reduktion mit $NaCNBH_3$ [10] erhalten. Nach erneuter Acetylierung (Acetanhydrdid/Triethylamin/DMAP) wird das Thioglycosid durch NBS/HF ins Fluorid [11] überführt.

3) Synthese des geschützten N-Acetyllactosamindisaccharids

Entsprechend der Vorschrift von K. C. Nicolaou et. al. (J. Chem. Soc., Chem. Comm. 870 (1991)) wird das Galactosylfluorid [11] mit dem N-Acetylglucoseaminderivat [5] in Gegenwart von $AgClO_4/SnCl_2$ zu [12] verknüpft, das durch Abspaltung der Allylschutzgruppe ($Ru(PPh_3)_4/H_2$; TsOH/MeOH) in [13] überführt wird. Dieses wird analog zu der Vorschrift von B. A. Silwanis et. al. (J. Carbohydr. Chem. 10, 1067 (1991)) mit 6-Azidohexanol umgesetzt.

Analog zu der Vorschrift von K. C. Nicolaou et. al. J. Am. Chem. Soc. 114, 3127 (1992)) erfolgt die Spaltung der Acetyl- und der Phthaloylschutzgruppen mit Hydrazinhydrat. Vor Abspaltung der Benzylschutzgruppen ($H_2/Pd(OH)_2$, MeOH) wird die freie Aminogruppe selektiv in Gegenwart der freien Hydroxylgrupen mit überschüssigem Acetanhydrid acetyliert. Es entsteht [14].

TRISACCHARID (Schema 1, 2, 3, 5 und 8)

1) Synthese eines geschützten N-Acetylglucosebausteins

2-Amino-2-deoxyglucose-Hydrochlorid [1] wird entsprechend der Vorschrift von R. U. Lemieux et. al. (ACS Symp. Ser., 39, 90 (1976)) durch Umsetzung mit Phthalsäureanhydrid und anschließende Reaktion mit Acetanhydrid/Pyridin in 1,3,4,6-Tetraacetyl-2-N-acetyl-2-deoxyglucose [2] überführt.

Durch Behandlung mit Zinntetrachlorid/Thiophenol entsteht nach K. C. Nicolaou et. al. (J. Am. Chem. Soc. 112, 3695 (1990) das entsprechende 1-Thiophenylderivat [4]. Nach Abspaltung der Acetylschutzgruppen [5] werden die Benzyliden- und die Allylgruppe (Benzyldimethylacetal/CSA; Allylbromid/NaH) eingeführt . Durch Reduktion mit $NaCNBH_3$ wird die 4-OH-Gruppe freigesetzt [6].

2) Synthese eines geschützten Galactosebausteins

Galactose [7] wird entsprechend der Vorschrift von K. C. Nicolaou et. al. (J. Chem. Soc., Chem. Comm. 870 (1991)) durch Versetzen mit Acetanhydrid/Triethylamin/DMAP und Zinntetrachlorid/Thiophenol in das Thioglycosid [8] überführt. Nach Abspaltung der Acetylschutzgruppen entsteht [9] durch Umsetzung mit Benzaldehyddimethylacetal/CSA. Nach Acetylierung (Acetanhydrid/Triethylamin/DMAP) wird durch Reduktion mit $NaCNBH_3$ [10] erhalten. Nach erneuter Acetylierung (Acetanhydrdid/Triethylamin/DMAP) wird das Thioglycosid durch NBS/HF ins Fluorid [11] überführt.

3) Synthese eines geschützten Fucosebausteins

Fucose [15] wird nach der Vorschrift von K. C. Nicolaou et. al. (J. Am. Chem. Soc. 112, 3694 (1990)) peracetyliert (Acetanhydrid/Triethylamin/DMAP) und mit Zinntetrachlorid/Thiophenol in das Thioglycosid [16] überführt. Anschließend wird nach einem Austausch der Acetyl- gegen Benzylschutzgruppen (NaOMe/MeOH; NaH/BnBr) das Thiophenol mit NBS/HF oder NBS/DAST in das Fluorid [17] überführt.

4) Synthese des geschützten N-Acetyllactosamindisaccharids

Entsprechend der Vorschrift von K. C. Nicolaou et. al. (J. Chem. Soc., Chem. Comm. 870 (1991)) wird das Galactosylfluorid [11] mit dem N-Acetylglucoseaminderivat [5] in Gegenwart von $AgClO_4/SnCl_2$ zu [12] verknüpft, das durch Abspaltung der Allylschutzgruppe ($Ru(PPh_3)_4/H_2$; TsOH/MeOH) in [13] überführt wird.

5) Synthese des geschützten Lewis-X-trisaccharids

Das Disaccharid [13] wird nach der Vorschrift von K. C. Nicolaou et. al. (J. Chem. Soc., Chem. Comm. 870 (1991)) mit dem Fucosylfluorid [17] ($AgClO_4/SnCl_2$) zum Lewis-X-Derivat [18] umgesetzt. Dieses wird analog zu der Vorschrift von B. A. Silwanis et. al. (J. Carbohydr. Chem. 10, 1067 (1991)) mit 6-Azidohexanol umgesetzt.

Analog zu der Vorschrift von K. C. Nicolaou et. al. J. Am. Chem. Soc. 114, 3127 (1992)) erfolgt die Spaltung der Acetyl- und der Phthaloylschutzgruppen mit Hydrazinhydrat. Vor Abspaltung der Benzylschutzgruppen ($H_2/Pd(OH)_2$, MeOH) wird die freie Aminogruppe selektiv in Gegenwart der freien Hydroxylgrupen mit überschüssigem Acetanhydrid acetyliert. Es entsteht [19].

TETRASACCHARID (Schema 1-6 und 8)

1) Synthese eines geschützten N-Acetylglucosebausteins

2-Amino-2-deoxyglucose-Hydrochlorid [1] wird entsprechend der Vorschrift von R. U. Lemieux et. al. (ACS Symp. Ser., 39, 90 (1976)) durch Umsetzung mit Phthalsäureanhydrid und anschließende Reaktion mit Acetanhydrid/Pyridin in 1,3,4,6-Tetraacetyl-2-N-acetyl-2-deoxyglucose [2] überführt.

Durch Behandlung mit Zinntetrachlorid/Thiophenol entsteht nach K. C. Nicolaou et. al. (J. Am. Chem. Soc. 112, 3695 (1990) das entsprechende 1-Thiophenylderivat [4]. Nach Abspaltung der Acetylschutzgruppen [5] werden die Benzyliden- und die Allylgruppe (Benzyldimethylacetal/CSA; Allylbromid/NaH) eingeführt. Durch Reduktion mit $NaCNBH_3$ wird die 4-OH-Gruppe freigesetzt [6].

2) Synthese eines geschützten Galactosebausteins

Galactose [7] wird entsprechend der Vorschrift von K. C. Nicolaou et. al. (J. Chem. Soc., Chem. Comm. 870 (1991)) durch Versetzen mit Acetanhydrid/Triethylamin/DMAP und Zinntetrachlorid/Thiophenol in das Thioglycosid [8] überführt. Nach Abspaltung der Acetylschutzgruppen entsteht [9] durch Umsetzung mit Benzaldehyddimethylacetal/CSA. Nach Acetylierung (Acetanhydrid/Triethylamin/DMAP) wird durch Reduktion mit $NaCNBH_3$ [10] erhalten. Nach erneuter Acetylierung (Acetanhydrdid/Triethylamin/DMAP) wird das Thioglycosid durch NBS/HF ins Fluorid [11] überführt.

3) Synthese eines geschützten Fucosebausteins

Fucose [15] wird nach der Vorschrift von K. C. Nicolaou et. al. (J. Am. Chem. Soc. 112, 3694 (1990)) peracetyliert (Acetanhydrid/Triethylamin/DMAP) und mit Zinntetrachlorid/Thiophenol in das Thioglycosid [16] überführt. Anschließend wird nach einem Austausch der Acetyl- gegen Benzylschutzgruppen (NaOMe/MeOH; NaH/BnBr) das Thiophenol mit NBS/HF oder NBS/DAST in das Fluorid [17] überführt.

Schema 1

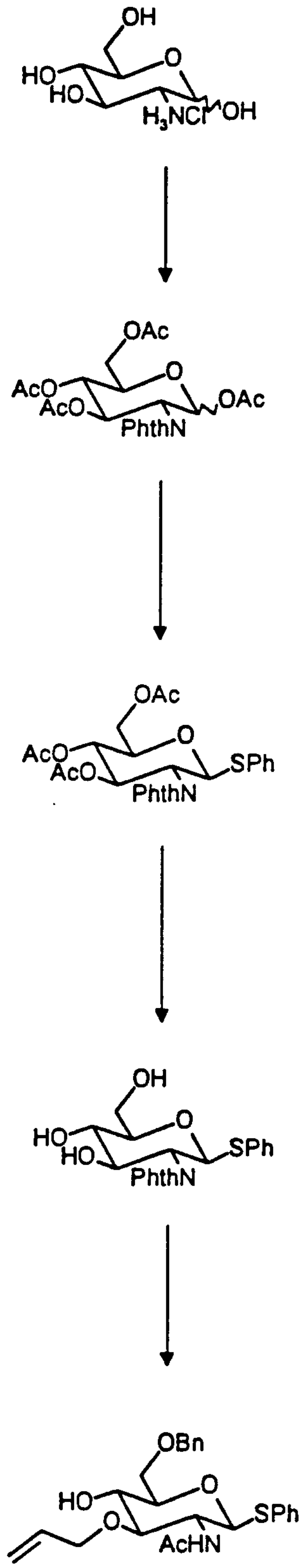

1

2

3

5

6

Schema 2

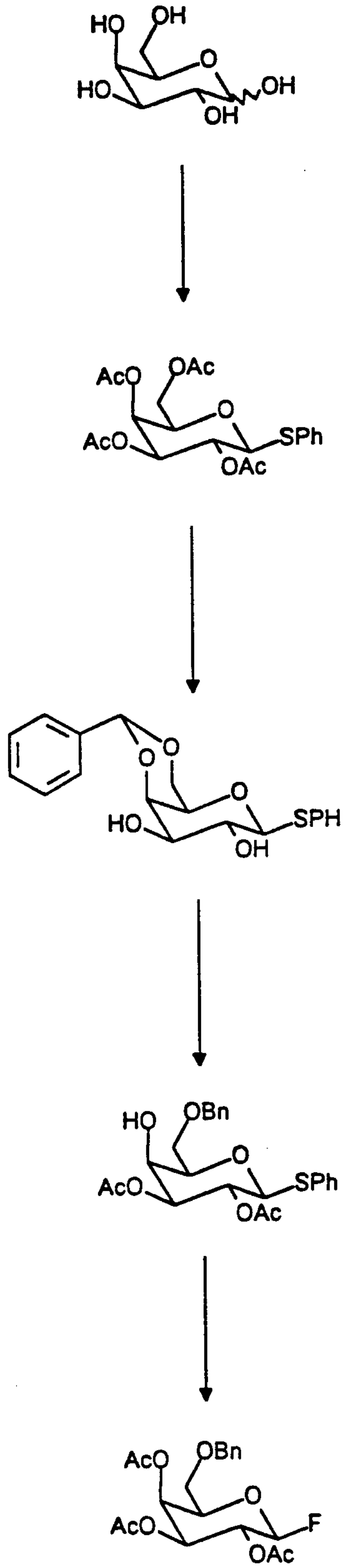

7

8

9

10

11

Schema 3

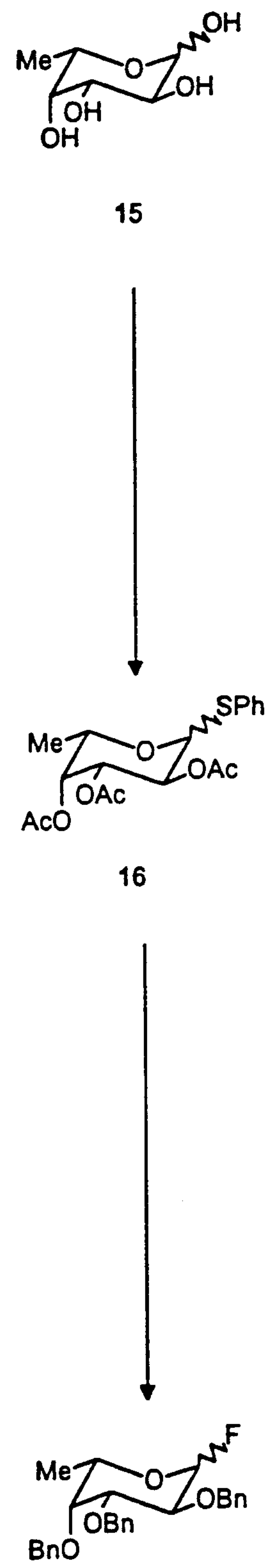

15

16

Schema 4

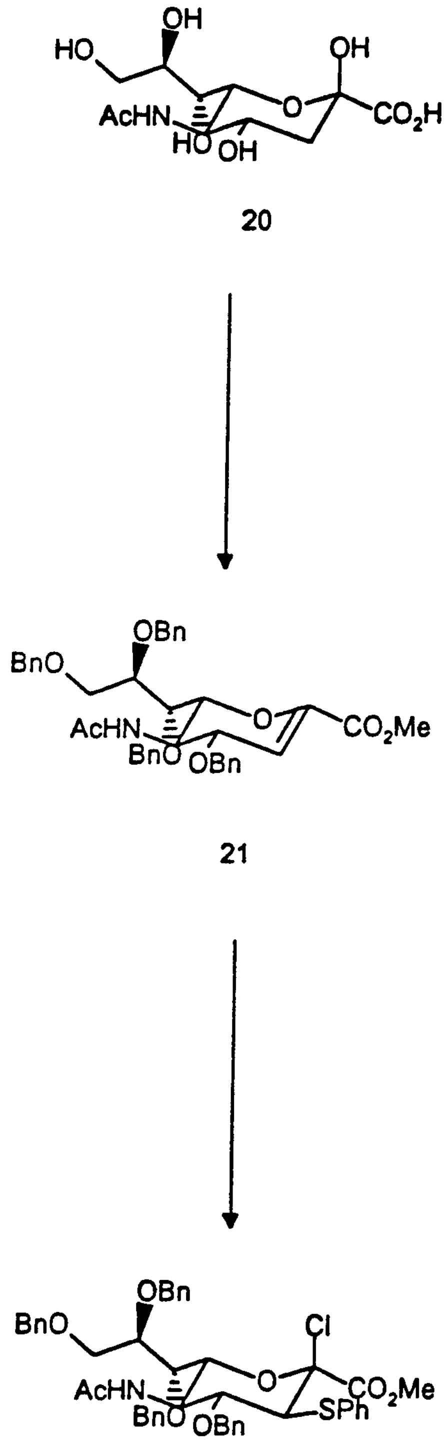

20

21

Schema 5

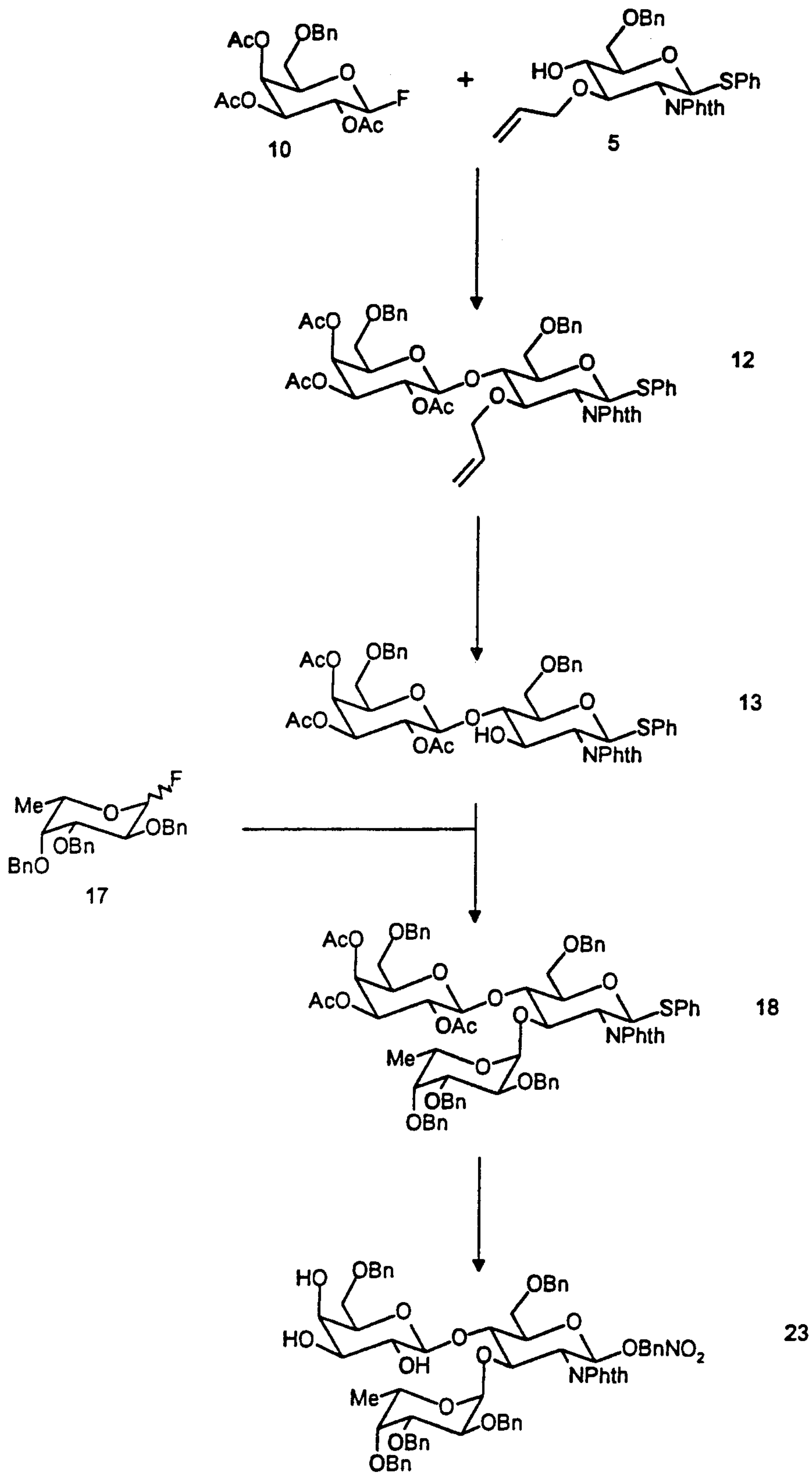

10

5

12

13

17

18

23

Schema 6

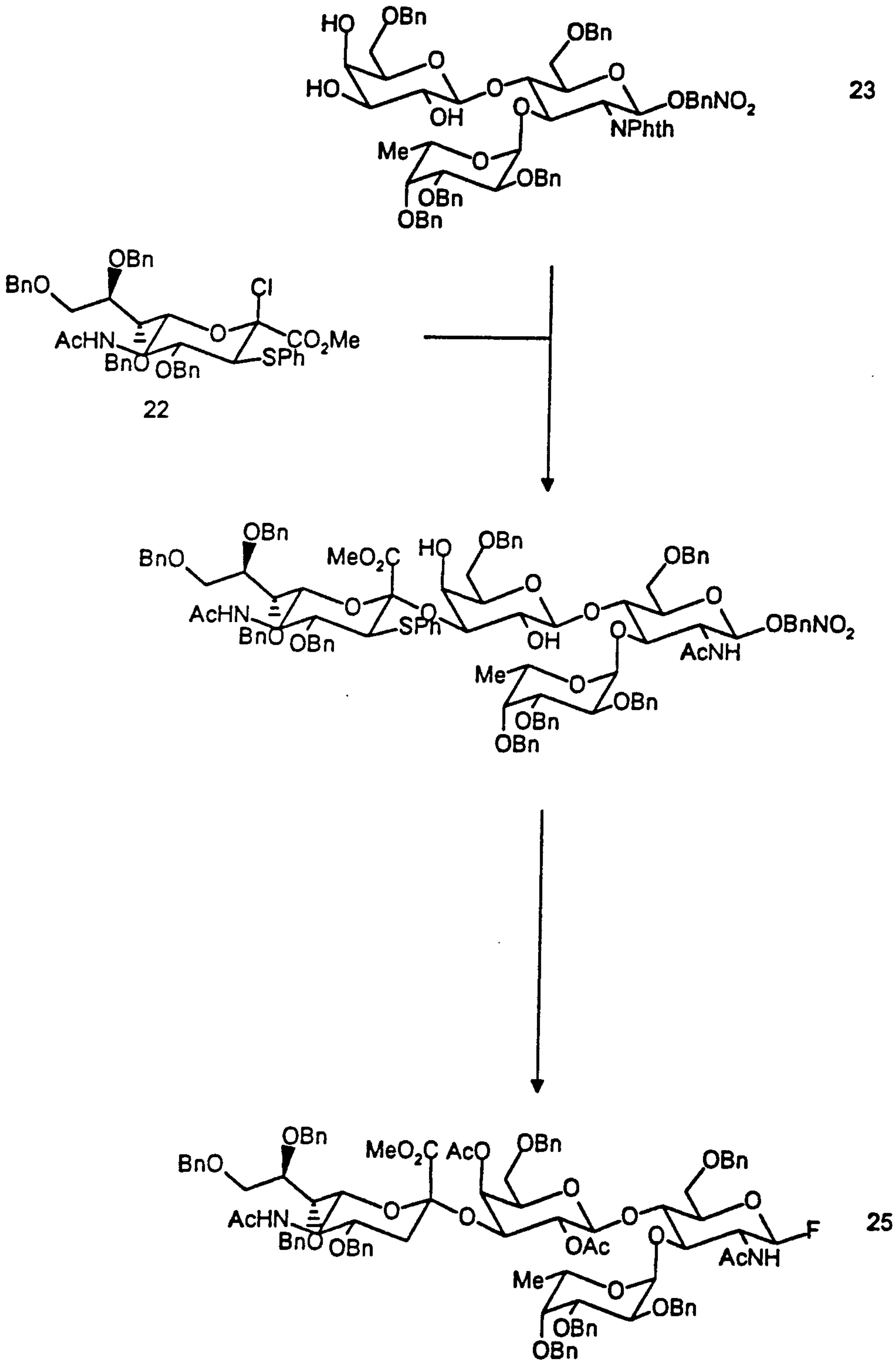

23

22

24

25

Schema 7

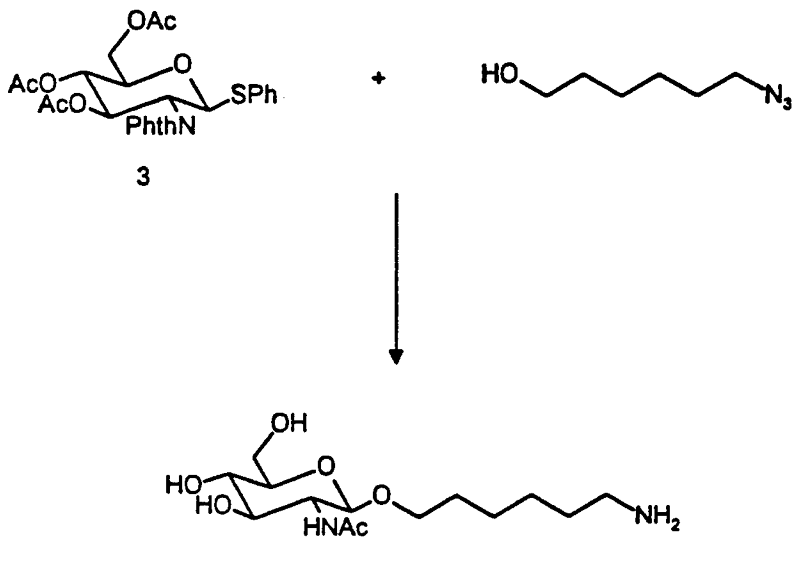

3

4

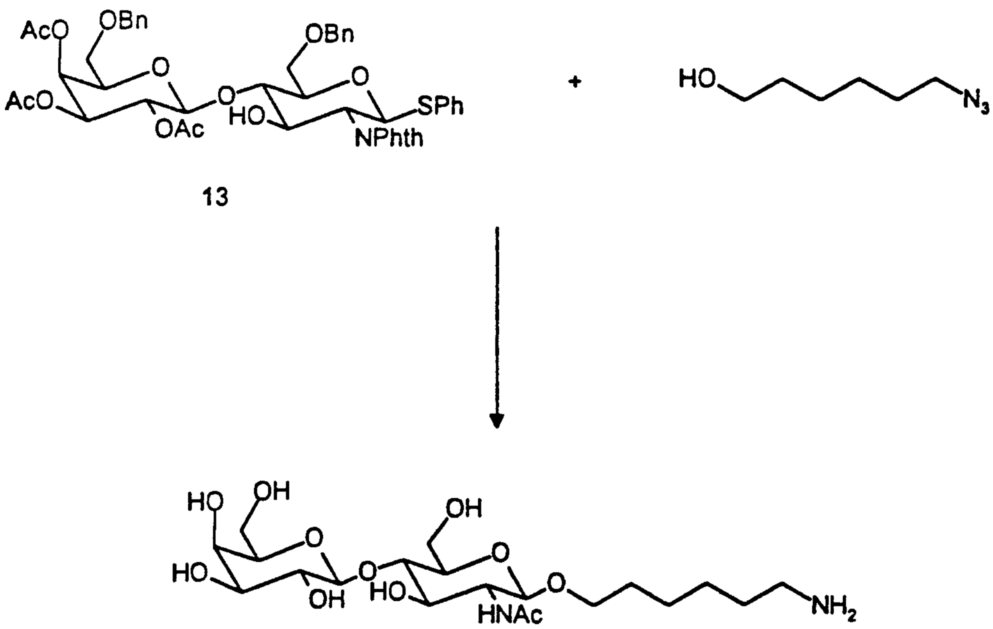

13

Schema 8

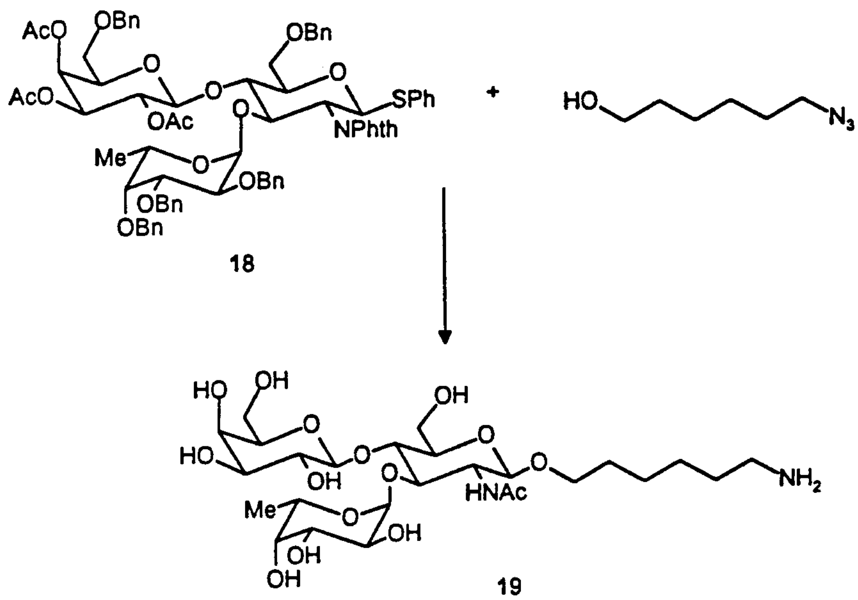

18

19

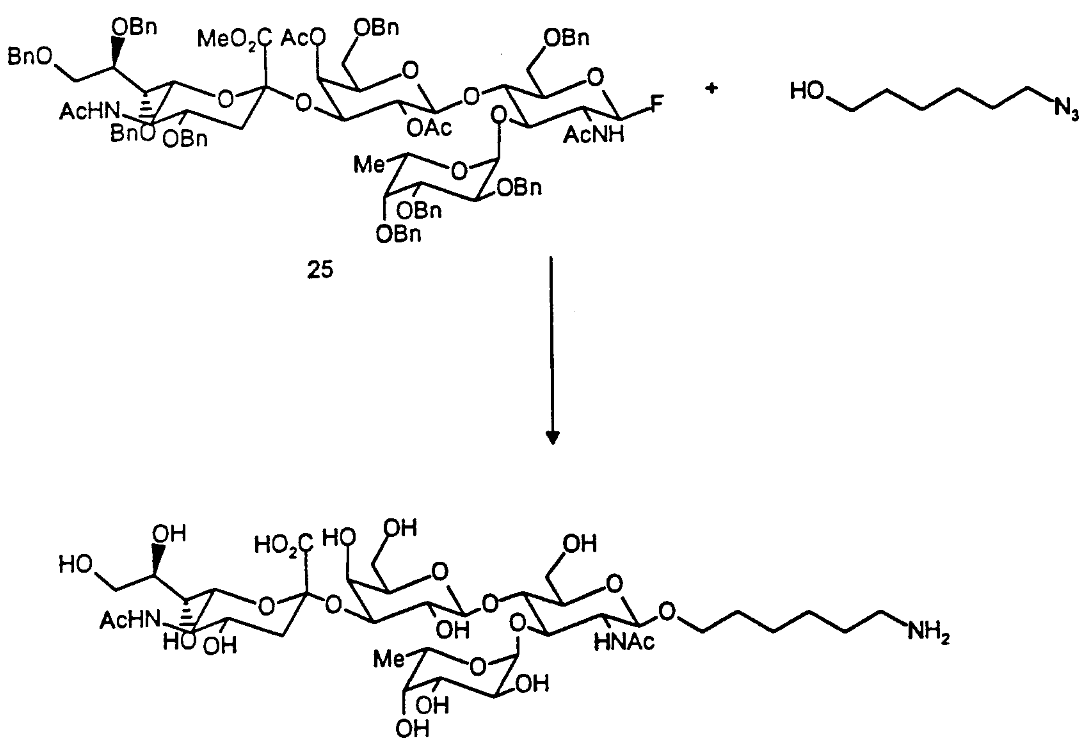

25

4) Synthese eines geschützten N-Acetylneuraminsäurebausteins

N-Acetylneuraminsäure [20] wird durch Behandlung mit Dowex 50 in Methanol nach der Vorschrift von R. Kuhn et. al. (Chem. Ber. 99, 611 (1966))in den entsprechenden Methylester [15] überführt und mit

Acetanhydrid in Gegenwart von katalytischen Mengen Schwefelsäure nach der Vorschrift von K. C. Nicolaou et. al. (J. Chem. Soc., Chem. Comm. 870 (1991)) peracetyliert. Anschließend wird mit Natronlauge deacetyliert und mit Benzylbromid/NaOH/$Bu_4NI$ zu [21] perbenzyliert. Durch Umsetzung mit Phenylsulfenylchlorid entsteht dann der Neuraminsäuredonor [22].

5) Synthese des geschützten N-Acetyllactosamindisaccharids

Entsprechend der Vorschrift von K. C. Nicolaou et. al. (J. Chem. Soc., Chem. Comm. 870 (1991)) wird das Galactosylfluorid [11] mit dem N-Acetylglucoseaminderivat [5] in Gegenwart von $AgClO_4/SnCl_2$ zu [12] verknüpft, das durch Abspaltung der Allylschutzgruppe ($Ru(PPh_3)_4/H_2$; TsOH/MeOH) in [13] überführt wird.

6) Synthese des geschützten Lewis-X-trisaccharids

Das Disaccharid [13] wird nach der Vorschrift von K. C. Nicolaou et. al. (J. Chem. Soc., Chem. Comm. 870 (1991)) mit dem Fucosylfluorid [17] ($AgClO_4/SnCl_2$) zum Lewis-X-Derivat [18] umgesetzt.

7) Synthese des geschützten Sialyl-Lewis-X-Tetrasaccharids

Das Trisaccharid [18] wird entsprechend der Vorschrift von K. C. Nicolaou et. al. J. Am. Chem. Soc. 114, 3127 (1992)) mit NBS/DAST in das Fluorid überführt, das nach Umsetzung mit o-Nitrobenzylalkohol/$AgClO_4/SnCl_2$ das entsprechende Glycosid liefert. Die Reaktion mit NaOMe/HOMe liefert die Trihydroxyverbindung [23].

Diese wird entsprechend der Vorschrift von K. C. Nicolaou et. al. (J. Am. Chem. Soc. 114, 3127 (1992)) durch Umsetzung mit [22] in Gegenwart von $Hg(CN)_2/HgBr_2$ zum Sialyl-Lewis-X-Derivat [24] verknüpft.

Entsprechend der Vorschrift von K. C. Nicolaou et. al. J. Am. Chem. Soc. 114, 3127 (1992)) wird mit Acetanhydrid/Pyridin acetyliert und zur Abspaltung der Nitrobenzylgruppe bestrahlt. Anschließend erfolgt die Abspaltung der Thiophenylgruppe durch Reaktion mit Triphenylzinnhydrid/AIBN sowie die Umwandlung in das Fluorid [25] durch Umsetzung mit Diethylaminoschwefelftifluorid. Diese Verbindung wird dann analog zu obiger Vorschrift mit 6-Azidohexanol in Gegenwart von $AgOTf/HfCp_2Cl_2$ zum entsprechenden Glycosid umgesetzt.

Analog zu der Vorschrift von K. C. Nicolaou et. al. J. Am. Chem. Soc. 114, 3127 (1992)) erfolgt die Spaltung der Acetylschutzgruppen sowie die der Phthaloylschutzgruppe mit Hydrazinhydrat, die des Methylesters mit LiI/Pyridin. Vor Abspaltung der Benzylschutzgruppen ($H_2/Pd(OH)_2$, MeOH) wird die freie Aminogruppe selektiv in Gegenwart der freien Hydroxylgrupen mit überschüssigem Acetanhydrid acetyliert. Es ensteht [26].

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

Die für die Kopplung von Mono-, Di- und Oligosaccharidbausteinen geeigneten biodegradablen Polymere werden nach beschriebenen Verfahren der Polymerchemie hergestellt. Die Kopplung selbst erfolgt nach üblichen polymeranalogen Syntheseverfahren.

So wird z.B. Poly-D,L-Succinimid (PSI) entsprechend der Vorschrift von Neri et al., J. Med. Chem., 16, 893 (1973) durch Einwirkung von 85 %iger Phosphorsäure auf Asparaginsäure bei Temperaturen von 160°C - 180°C gewonnen. Durch polymeranaloge Umsetzung von PSI mit Hydroxyethylamin bei Raumtempertur oder leicht erhöhter Temperatur erhält man Poly-$\alpha,\beta$-(2-hydroxyethyl)-D,L-aspartamid (PHEA) (Neri et al., ibid). Die Alkoholgruppen des PNEA lassen sich nach üblichen Verfahren verestern (US 5.041.291). Bei partieller Umsetzung von PSI mit Ethanolamin erhält man entsprechende Copolymere (US 5.229.469). Die basische Hydrolyse von PSI führt zur Polyasparaginsäure (analog Giammona et al., Chem. Pharm. Bull. 37(8), 2245 (1989).

Analog zur Reaktion mit Hydroxyethylamin kann PSI auch mit anderen Aminen umgesetzt werden (EP 0 548 794), wodurch sich zusätzliche funktionelle Gruppen einführen lassen, die als Wirkungsverstärker fungieren können.

Poly-L-Lysinmethylester-fumaramid, als ein weiteres Ausgangspolymer, wird durch Grenzphasenpolykondensation aus L-Lysinmethylester und Fumarsäuredichlorid hergestellt (US 4.834.248). Die Methylestergruppen können direkt oder nach partieller Hydrolyse und anschließender Aktivierung, z.B. als p-Nitrophenylester, mit den aminogruppenhaltigen Mono-, Di- und Oligosacchariden umgesetzt werden.

Analog, d.h. mittels p-Nitrophenylester, lassen sich polymere Kohlenhydratrezeptorblocker auf Basis von Polyglutarmaten herstellen (Polymersynthese analog: Anderson in "Macromolecules as Drugs and as

Carriers for Biologically active Materials" (Ed: D.A. Tirell), NY Acad. Sci., NY, 1985, S. 67-75).

Hydroxyethylstärke wird vor der Umsetzung mit den aminogruppenhaltigen Kohlenhydratderivaten aktiviert durch Perjodatoxidation (analog Schacht et al. in "Macromolecules as Drugs and as Carriers for Biologically active Materials" (Ed: D.A. Tirell), NY Acad. Sci., NY, 1985, 199 - 212) bzw. durch Reaktion mit Chlorkohlensäureestern wie p-Nitrophenyl- und N-Hydroxysuccinimidylchloroformat (analog Wilchek, M. und Miron, T.; Biochem. Int. 4, 629 (1982)).

Die Umsetzung des Polymers mit dem Wirkungsverstärker einerseits und des Kohlenhydratanteils mit dem bifunktionellen Spacer andererseits findet in homogener Lösung statt. Bevorzugt in Wasser oder in wassermischbaren Lösemitteln, wie vorzugsweise Dimethylformamid oder Acetonitril. Besonders bevorzugt in einem Gemisch aus Wasser und wassermischbaren Lösemitteln, bevorzugt im Verhältnis 1:1.

Die Reaktion wird bei 0 °C - 80 °C, bevorzugt bei 20 °C - 40 °C und besonders bevorzugt bei 35 °C - 40 °C durchgeführt.

Die in den folgenden Beispielen in eckigen Klammern angegebenen Zahlen beziehen sich auf die in den Schemata angegebenen Zahlen.

1. Synthese des Monosaccharids

1,3,4,6-Tetra-O-acetyl-2-deoxy-2-phthalimido-β-D-glucopyranose [2]

D-Glucosaminhydrochlorid [1] (0,22-5,32 g, 1-20 mMol) werden zu einer Natriummethylatlösung (hergestellt aus 0,02-0,56 g Natrium und 1-20 ml Methanol) gegeben, 10 min geschüttelt und von ausgefallenen Natriumchlorid abfiltriert. Das Filtrat wird mit Phthalsäureanhydrid (0,07-1,48 g, 0,5-10 mMol) 10 min gerührt und dann mit Triethylamin (0,10-2,02 g, 1-20 mMol) versetzt. Nach abgeschlossener Reaktion wird das Lösungsmittel eingedampft und der Rückstand unter Kühlung mit Pyridin (2-40 ml) und Acetanhydrid (5-20 ml) versetzt. Nach 16 Std. bei Raumtemperatur wird gießt man auf Eis und extrahiert das Produkt mit Dichlormethan. Nach Waschen mit Wasser, verdünnter Salzsäure und gesättigter Natriumhydrogencarbonatlösung erhält man das Rohprodukt, das aus Ether umkristallisiert wird (0,31-8,02 g, ca. 82 % Ausbeute).

FAB-MS: $M + H^+ = 454$.

NMR-Daten im Einklang mit R. U. Lemieux et. al. (ACS Symp. Ser., 39, 90 (1976)).

1-Thiophenyl-3,4,6-tri-O-acetyl-2-deoxy-2-phthalimido-β-D-glucopyranose [3]

Das Produkt [2] (0,31-8,02 g, 0,68-17,7 mMol) wird in 3,2-100 ml trockenem Dichlormethan gelöst, mit Thiophenol (0,15-3,9 g, 1,36-35,4 mMol) und Zinntetrachlorid (0,35-9,22 g, 1,36-35,4 mMol) versetzt und bis zur vollständigen Reaktion gerührt. Nach Verdünnen des Reaktionsgemischs wäscht man mit gesättigter Natriumhydrogencarbonatlösung. Nach dem Einengen erhält man das Rohprodukt [3]. Die Reinigung erfolgt durch Chromatographie an Kieselgel mit einem Essigester/iso-hexan-Gradienten (0,27-71.4 g, 0,54-14,2 mMol, ca. 80 %).

FAB-MS: $M + H^+ = 504$.

NMR-Daten im Einklang mit K. C. Nicolaou et. al. (J. Am. Chem. Soc. 112, 3695 (1990))T. J. Caulfield, Ph. D. Dissertation, The University of Pennsylvania, 1991.

1-(6-Aminohexyl)-2-deoxy-2-acetamido-β-D-Glucopyranose [4]

Das Rohprodukt [3] (0,27-7,14 g, 0,54-14,2 mMol) und 6-Azidohexanol (0,27-3,54 g, 1,08-14,1 mMol) werden in trockenem Acetonitril gelöst und bei 0°C mit Molsieb (0,2-5 g, 3 Å), Trifluormethansulfonsäuretrimethylsilylester (0,02-1,56 g, 0,1-7 mMol) versetzt. Nach Abgeschlossener Reaktion wird abfiltriert und mit gesättigter Natriumhydrogencarbonatlösung gewaschen. Nach Einengen erhält man das Rohprodukt das durch Chromatographie an Kieselgel mit einem Essigester/iso-Hexan-Gradienten gereinigt wird (0,17-2,23 g, 0,35-4,58 mMol, ca. 69 % Ausbeute). Anschließend wird in Methanol/Hydrazinhydrat (1:1) (1-30 ml) aufgenommen und bis zur vollständigen Reaktion auf 80°C erwärmt. Nach Verdampfen des Lösungsmittels wird das Rohprodukt in Dichlormethan/Methanol (1:1) aufgenommen und bei 0°C mit Acetanhydrid (3,5-45,8 mMol) bis zur vollständigen Reaktion gerührt. Nach Eindampfen erfolgt die Reduktion des Azids mit Wasserstoff in Gegenwart katalytischer Mengen Palladium auf Kohle (10 %) (0,01-1,5 g) in Methanol (0,5-40 ml). Das nach Eindampfen anfallende Rohprodukt wird durch Gelchromatographie an Biogel P2 gereinigt (-[4]; 0,08-1,02 g, 0,25-3,2 mMol, ca.: 70 % Ausbeute).

FAB-MS: $M + H^+ = 321$.

$^1$H-NMR [300 MHz, DMSO]: 7,68 (HNAc), 4,23 (GlcNAc-H-1), 3,78-3,6 (m), 3,49-3,23 (m), 3,08-3,02 (m)

2,58, 1,79 (GlcNCOC$\underline{H}_3$), 1,51-1,21 (O-(C$\underline{H}_2$)-N).

2. Synthese des Disaccharids

2.1 Synthese der einzelnen Zuckerbausteine

2.1.1 Synthese des N-Acetyglucosaminbausteins [6]

1,3,4,6-Tetra-O-acetyl-2-deoxy-2-phthalimido-β-D-glucopyranose [2]

D-Glucosaminhydrochlorid [1] (0,22-5,32 g, 1-20 mMol) werden zu einer Natriummethylatlösung (hergestellt aus 0,02-0,56 g Natrium und 1-20 ml Methanol) gegeben, 10 min geschüttelt und von ausgefallenen Natriumchlorid abfiltriert. Das Filtrat wird mit Phthalsäureanhydrid (0,07-1,48 g, 0,5-10 mMol) 10 min gerührt und dann mit Triethylamin (0,10-2,02 g, 1-20 mMol) versetzt. Nach abgeschlossener Reaktion wird das Lösungsmittel eingedampft und der Rückstand unter Kühlung mit Pyridin (2-40 ml) und Acetanhydrid (5-20 ml) versetzt. Nach 16 Std. bei Raumtemperatur wird gießt man auf Eis und extrahiert das Produkt mit Dichlormethan. Nach Waschen mit Wasser, verdünnter Salzsäure und gesättigter Natriumhydrogencarbonatlösung erhält man das Rohprodukt, das aus Ether umkristallisiert wird (0,31-8,02 g, ca. 82 % Ausbeute).

FAB-MS: M + $H^+$ = 454.

NMR-Daten im Einklang mit R. U. Lemieux et. al. (ACS Symp. Ser., 39, 90 (1976)).

1-Thiophenyl-2-deoxy-2-phthalimido-β-D-glucopyranose [5]

Das Produkt [2] (0,31-8,02 g, 0,68-17,7 mMol) wird in 3,2-100 ml trockenem Dichlormethan gelöst, mit Thiophenol (0,15-3,9 g, 1,36-35,4 mMol) und Zinntetrachlorid (0,35-9,22 g, 1,36-35,4 mMol) versetzt und bis zur vollständigen Reaktion gerührt. Nach Verdünnen des Reaktionsgemischs wäscht man mit gesättigter Natriumhydrogencarbonatlösung. Das so erhaltene Rohprodukt [3]. [3] wird durch Chromatograhie an Kieselgel mit einem Essigester/iso-Hexan-Gradienten gereinigt (0,27-7,14 g, 0,54-14,2 mMol, ca. 80 %), wird in wasserfreiem Methanol (3-90 ml) gelöst und mit katalytischen Mengen Natriummethylat (hergestellt aus 0,002-0,05 g Natrium in 0,1-2 ml Methanol) versetzt. Nach abgeschlossener Reaktion wird mit saurem Ionenaustauscher (0,2-5 g Amberlite IR-120, $H^+$-Form) neutralisiert ([5]; 0,21-5,31 g, 0,54-14,1 mMol, ca. 99 % Ausbeute).

FAB-MS: M + $H^+$ = 378.

NMR-Daten im Einklang mit K. C. Nicolaou et. al. (J. Am. Chem. Soc. 112, 3695 (1990)); bzw. T. J. Caulfield, Ph. D. Dissertation, The University of Pennsylvania, 1991.

1-Thiophenyl-3-O-allyl-6-O-benzyl-2-deoxy-2-phthalimido-B-D-glucopyranose [6]

Das Rohprodukt [5] (0,21-5,31 g, 0,54-14,1 mMol) wird in wasserfreiem Acetonitril (3-90 ml) mit Benzaldehyddimethylacetal (0,12-3,22 g, 0,81-21,1 mMol) und katalytischen Mengen p-Toluolsulfonsäure (4-90 mg) bis zum Ende der Reaktion gerührt. Das nach Waschen mit gesättigter Natriumhydrogencarbonatlösung anfallende Rohprodukt wird in wasserfreiem Dimethylformamid (2,5-85 ml) mit Natriumhydrid (0,02-0,51 g, 0,81-21,15 mMol) und Allylbromid (0,09-2,37 g, 0,75-19,74 mMol] versetzt. Nach abgeschlossener Reaktion versetzt man mit Methanol (0,06-1,55 ml, 2,7-70,5 mMol), verdünnt mit Essigester und wäscht nacheinander mit gesättigter Ammoniumchlorid- und gesättigter Natriumhydrogencarbonatlösung. Das Rohprodukt [5] wird durch Chromatograhie an Kieselgel mit einem Essigester/iso-Hexan-Gradienten gereinigt (0,23-5,98 g, 0,46-12,13 mMol, ca. 86 %), anschließend in trockenem Tetrahydrofuran (3,5-90 ml) aufgenommen und mit Natriumcyanoborhydrid (0,67-17,7 g, 10,8-282 mMol) versetzt. Anschließend wird bis zur abgeschlossenen Reaktion langsam etherischer Salzsäurelösung zugegeben. Nach Filtration wird die organische Phase mit Essigester verdünnt und mit gesättigter Natriumhydrogencarbonatlösung neutral gewaschen. Das Rohprodukt [6] wird durch Chromatographie an Kieselgel mit einem Essigester/iso-Hexan-Gradienten gereinigt (0,17-4,56 g, 0,35-9,17 mMol, ca. 74 % Ausbeute).

FAB-MS: M + $H^+$ = 416.

NMR-Daten im Einklang mit K. C. Nicolaou et. al. (J. Am. Chem. Soc. 112, 3695 (1990)); bzw. T. J. Caulfield, Ph. D. Dissertation, The University of Pennsylvania, 1991.

2.1.2 Synthese des Galactosebausteins [11]

1-Thiophenyl-2,3,4,6-tetra-O-acetylgalacto-β-D-pyranose [8]

Galactose [7] (0,18-3,6 g, 1-20 mMol) wird in Dichlormethan (5-90 ml) suspendiert und mit Acetanhydrid (0,75-15,43 g, 7,5-150 mMol), Triethlyamin (0,8-16,3 g, 8-160 mMol) und katalytischen Mengen Dimethylaminopyridin (0,01-0,1 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan (3,5-110 ml) gelöst und mit Thiophenol (0,22-4,41 g, 2-40 mMol) und Zinntetrachlorid (0,51-10,28 g, 2-40 mMol) versetzt. Nach beendeter Reaktion verdünnt man und erhält nach Waschen mit gesättigter Natriumhydrogencarbonatlösung das Rohprodukt [8], das durch Chromatographie an Kieselgel mit einem Essigester/iso-Hexan-Gradienten gereinigt wird (0,37-7,48 g, 0,85-17 mMol, ca. 85 % Ausbeute).

FAB-MS: M + $H^+$ = 441.

NMR-Daten im Einklang mit K. C. Nicolaou et. al. (J. Am. Chem. Soc. 112, 3695 (1990)); bzw. T. J. Caulfield, Ph. D. Dissertation, The University of Pennsylvania, 1991.

1-Thiophenyl-4,6-O-benzyliden-β-D-galactopyranose [9]

Das Produkt [8] (0,37-7,48 g, 0,85-17 mMol) wird in wasserfreiem Methanol (4-150 ml) gelöst und mit katalytischen Mengen Natriummethylat (hergestellt aus 0,003-0,06 g Natrium in 0,1-2,5 ml Methanol) versetzt. Nach abgeschlossener Reaktion wird mit saurem Ionenaustauscher (0,25-6 g Amberlite IR-120, $H^+$-Form) neutralisiert und eingedampft. Das erhaltene Reaktionsprodukt wird in wasserfreiem Acetonitril (4,5-150 ml) mit Benzaldehyddimethylacetal (0,19-5,05 g, 1,27-33,1 mMol) und katalytischen Mengen p-Toluolsulfonsäure (4-90 mg) bis zum Ende der Reaktion gerührt. Nach Waschen mit gesättigter Natriumhydrogencarbonatlösung entsteht das Rohprodukt [9], das durch Chromatographie an Kieselgel mit einem Dichlormethan/Methanol-Gradienten gereinigt wird (0,28-5,51 g, 0,77-15,3 mMol, ca. 90 % Ausbeute).

FAB-MS: M + $H^+$ = 361.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-2,3-di-O-acetyl-6-O-benzyl-β-D-galactopyranose [10]

Das Produkt [9] (0,28-5,51 g, 0,77-15,3 mMol) wird in Dichlormethan (4-60 ml) suspendiert und mit Acetanhydrid (0,23-4,75 g, 2-40 mMol), Triethlyamin (0,25-5,02 g, 2,46-49,23 mMol) und katalytischen Mengen Dimethylaminopyridin (0,01-0,08 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Tetrahydrofuran (2,45-130 ml) gelöst und mit Natriumcyanoborhydrid (0,96-25,3 g, 15,4-402,8 mMol). Anschließen wird bis zur abgeschlossenen Reaktion langsam mit etherischer Salzsäurelösung versetzt. Nach Filtration wird die organische Phase mit Essigester verdünnt und mit gesättigter Natriumhydrogencarbonatlösung neutral gewaschen. Das Rohprodukt [10] wird durch Chromatographie an Kieselgel mit einem Essigester/isoHexan-Gradienten gereinigt (0,27-5,46 g, 0,62-12,24 mMol, ca. 80 % Ausbeute).

FAB-MS: M + $H^+$ = 447.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Fluor-2,3,4-tri-O-acetyl-6-O-benzyl-β-D-galactopyranose [11]

Das Produkt [9] (0,27-5,46 g, 0,62-12,24 mMol) wird in Dichlormethan (4-60 ml) suspendiert und mit Acetanhydrid (0,14-2,91 g, 1,24-24,8 mMol), Triethlyamin (0,15-3,11 g, 1,53-30,53 mMol) und katalytischen Mengen Dimethylaminopyridin (0,003-0,04 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan bei -78°C (5-80 ml) mit Dimethylaminoschwefeltrifluorid (0,25-4,89 g, 1,86-36,7 mMol) und N-Bromsuccinimid (0,11-3,26 g, 0,93-18,36 mMol) versetzt und auf Raumtemperatur aufgewärmt. Nach Waschen mit gesättigter Natriumhydrogencarbonatlösung engt man ein und trennt die Reaktionsnebenprodukte durch Kieselgelchromatographie (Stufengradient: Essigester/iso-Hexan) ab, so daß [11] entsteht (0,21-4,14 g, 0,53-10,4 mMol, ca. 85 % Ausbeute).

FAB-MS: M + $H^+$ = 399.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

2.2 Aufbau des Disaccharids aus dem N-Acetylglucosaminbaustein und dem Galactosebaustein

1-Thiophenyl-(2,3,4-tri-O-acetyl-6-O-benzyl-β-D-Galactopyranosyl)-(1-4)-3-O-alkyl-2-deoxy-2-acetmido-β-D-glucopyranose [12]

Das N-Acetylglucosaminderivat [5] (0,17-4,56 g, 0,35-9,17 mMol) und das Galactosederivat [11] (0,21-4,14 g, 0,53-10,4 mMol) werden in Dichlormethan (3-120 ml) gelöst, mit Molsieb 4 Å (0,15-7,8 g), Silberperchlorat (0,11-5,39, 0,525-26,0 mMol) und Zinn(II)chlorid (0,1-4,93 g, 0,525-26,0 mMol) bei 0 °C versetzt. Nach vollständiger Reaktion wird abfiltriert und mit gesättigter Natriumhydrogencarbonatlösung gewaschen. Das Rohprodukt [12] wird durch Chromatographie an Kieselgel mit einem Essigester/isoHexan-Gradienten gereinigt (0,23-5,61 g, 0,26-6,42 mMol, ca. 70 % Ausbeute).

FAB-MS: M + $H^+$ = 874.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-(2,3,4-tri-O-acetyl-6-O-benzyl-β-D-Galactopyranosyl)-(1-4)-2-deoxy-2-acetamido-β-D-glucopyranose [13]

[12] (0,23-5,61 g, 0,26-6,42 mMol) werden in Ethanol (30-900 ml) gelöst und mit katalytischen Mengen Tetrakis-(triphenylphosphin)-ruthenium(II)hydrid (0,005-0,04 g) auf 95 °C erwärmt. Nach beendeter Reaktion wird abfiltriert, eingeengt, in Methanol (2,5-85 ml) aufgenommen und mit katalytischen Mengen p-Toluolsulfonsäure (0,001-0,08 g) versetzt. Nach vollständiger Reaktion wäscht man mit gesättigter Natriumhydrogencarbonatlösung und erhält nach Einengen das Rohprodukt [13]. Die Reinigung erfolgt durch Chromatographie an Kieselgel mit einem Essigester/isoHexan-Gradienten (0,2-4,81 g, 0,234-5,78 mMol, ca. 90 % Ausbeute).

FAB-MS: M + $H^+$ = 834.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-(6-Aminohexyl)-O-(β-D-Galactopyranosyl)-(1-4)-2-deoxy-2-acetamido-β-D-glucopyranose [14]

[13] (0,21-2,66 g, 0,54-7,05 mMol) und 6-Azidohexanol (0,27-3,54 g, 1,08-14,1 mMol) werden in trockenem Acetonitril gelöst und bei 0 °C mit Molsieb (0,2-5 g), Trifluormethansulfonsäuretrimethylsilylester (0,02-1,56 g, 1-70 mMol) versetzt. Nach abgeschlossener Reaktion wird abfiltriert und mit gesättigter Natriumhydrogencarbonatlösung gewaschen. Nach Einengen erhält man das Rohprodukt das durch Chromatographie an Kieselgel mit einem Essigester/iso-Hexan-Gradienten gereinigt wird (0,17-2,23 g, 0,35-4,58 mMol, ca. 65 % Ausbeute). Anschließend wird in Methanol/Hydrazinhydrat (1:1) (1-30 ml) aufgenommen und bis zur vollständigen Reaktion auf 80°C erwärmt. Nach Verdampfen des Lösungsmittels wird das Rohprodukt in Dichlormethan/Methanol (1:1) aufgenommen und bei 0°C mit Acetanhydrid (3,5-45,8 mMol) bis zur vollständigen Reaktion gerührt. Nach Eindampfen erfolgt die Reduktion des Azids mit Wasserstoff in Gegenwart katalytischer Mengen Palladium auf Kohle (10 %) (0,01-1,5 g] in Methanol (0,5-40 ml). Das nach Eindampfen anfallende Rohprodukt wird durch Gelchromatographie an Biogel P2 gereinigt [14] (0,12-1,54 g, 0,25-3,2 mMol, ca.: 70 % Ausbeute).

FAB-MS: M + $H^+$ = 483.

$^1$H-NMR [300 MHz $D_2O$]: 4,98 (Fuc-$\underline{H}$-1), 4,28 (Gal-$\underline{H}$-1), 4,33, 3,99-3,38 (m), 2,99, 2,09 (GlcNCOC$\underline{H}_3$), 1,72-1,32 (O-(C$\underline{H}_2$)$_5$-$CH_2$-N), 1,11 (Fuc-C$\underline{H}_3$) ppm.

3. Synthese des Trisaccharids

3.1 Synthese der einzelnen Zuckerbausteine

3.1.1 Synthese des N-Acetylglucosaminbausteins [6]

1,3,4,6-Tetra-O-acetyl-2-deoxy-2-phthalimido-β-D-glucopyranose [2]

D-Glucosaminhydrochlorid [1] (0,216-5,32 g, 1-20 mMol) wird zu einer Natriummethylatlösung (hergestellt aus 0,023-0,56 g Natrium und 1-20 ml Methanol) gegeben, 10 min geschüttelt und von ausgefallenen Natriumchlorid abfiltriert. Das Filtrat wird mit Phthalsäureanhydrid (0,074-1,48 g, 0,5-10 mMol) 10 min gerührt und dann mit Triethylamin (0,1-2,02 g, 1-20 mMol) versetzt. Nach abgeschlossener Reaktion wird das Lösungsmittel eingedampft und der Rückstand unter Kühlung mit Pyridin (2-40 ml) und Acetanhydrid (5-20 ml) versetzt. Nach 16 Std. bei Raumtemperatur wird gießt man auf Eis und extrahiert das Produkt mit Dichlormethan. Nach Waschen mit Wasser, verdünnter Salzsäure und gesättigter Natriumhydrogencarbonatlösung erhält man das Rohprodukt, das aus Ether umkristallisiert wird (0,31-8,02 g, ca. 82 % Ausbeute).
FAB-MS: $M + H^+ = 454$.
NMR-Daten im Einklang mit R. U. Lemieux et. al. (ACS Symp. Ser., 39, 90 (1976)).

1-Thiophenyl-2-deoxy-2-phthalimido-β-D-glucopyranose [5]

[2] (0,31-8,02 g, 0,68-17,7 mMol) wird in 3,2-100 ml trockenem Dichlormethan gelöst, mit Thiophenol (0,15-3,9 g, 1,36-35,4 mMol) und Zinntetrachlorid (0,35-9,22 g, 1,36-35,4 mMol) versetzt und bis zur vollständigen Reaktion gerührt. Nach Verdünnen des Reaktionsgemischs wäscht man mit gesättigter Natriumhydrogencarbonatlösung. Das so erhaltene Rohprodukt wird durch Chromatographie an Kieselgel mit einem Essigester/iso-Hexan-Gradienten gereinigt (0,27-7,14 g, 0,54-14,2 mMol, ca. 80 %). [3] wird in wasserfreiem Methanol (3-90 ml) gelöst und mit katalytischen Mengen Natriummethylat (hergestellt aus 0,002-0,05 g Natrium in 0,1-2 ml Methanol) versetzt. Nach abgeschlossener Reaktion wird mit saurem Ionenaustauscher (0,2-5 g Amberlite IR-120, $H^+$-Form) neutralisiert (4:0,21-5,31 g, 0,54-14,1 mMol, ca. 99 % Ausbeute).
FAB-MS: $M + H^+ = 378$.
NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

1-Thiophenyl-3-O-allyl-6-O-benzyl-2-deoxy-2-phthalimido-β-D-glucopyranose [6]

Das Rohprodukt [5] (0,21-5,31 g, 0,54-14,1 mMol) wird in wasserfreiem Acetonitril (3-90 ml) mit Benzaldehyddimethylacetal (0,12-3,22 g, 0,81-21,1 mMol) und katalytischen Mengen p-Toluolsulfonsäure (4-90 mg) bis zum Ende der Reaktion gerührt. Das nach Waschen mit gesättigter Natriumhydrogencarbonatlösung anfallende Rohprodukt wird in wasserfreiem Dimethylformamid (2,5-85 ml) mit Natriumhydrid (0,02-0,51 g, 0,81-21,15 mMol) und Allylbromid (0,09-2,37 g, 0,75-19,74 mMol) versetzt. Nach abgeschlossener Reaktion versetzt man mit Methanol (0,06-1,55 ml, 2,7-70,5 mMol), verdünnt mit Essigester und wäscht nacheinander mit gesättigter Ammoniumchlorid- und gesättigter Natriumhydrogencarbonatlösung. Das Rohprodukt [5] wird durch Chromatographie an Kieselgel mit einem Essigester/iso-Hexan-Gradienten gereinigt (0,23-5,98 g, 0,46-12,13 mMol, ca. 86 %) und anschließend in trockenem Tetrahydrofuran (3,5-90 ml) aufgenommen und mit Natriumcyanoborhydrid (0,67-17,7 g, 10,8-282 mMol) versetzt. Anschließend wird bis zur abgeschlossenen Reaktion langsam etherischer Salzsäurelösung zugegeben. Nach Filtration wird die organische Phase mit Essigester verdünnt und mit gesättigter Natriumhydrogencarbonatlösung neutral gewaschen. Das Rohprodukt [6] wird durch Chromatographie an Kieselgel mit einem Essigester/iso-Hexan-Gradienten gereinigt (0,17-4,56 g, 0,35-9,17 mMol, ca. 74 % Ausbeute).
FAB-MS: $M + H^+ = 416$.
NMR-Daten im Einklang mit K. C. Nicolaou et. al. (J. Am. Chem. Soc. 112, 3695 (1990))T. J. Caulfield, Ph. D. Dissertation, The University of Pennsylvania, 1991.

3.1.2 Synthese des Galactosebausteins [11]

1-Thiophenyl-2,3,4,6-tetra-O-acetyl-β-D-galactopyranose [8]

Galactose [7] (0,18-3,6 g, 1-20 mMol) wird in Dichlormethan (5-90 ml) suspendiert und mit Acetanhydrid (0,75-15,43 g, 7,5-150 mMol), Triethlyamin (0,8-16,3 g, 8-160 mMol) und katalytischen Mengen Dimethylaminopyridin (0,01-0,1 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan (3,5-110 ml) gelöst und mit Thiophenol (0,22-4,41 g, 2-40 mMol) und Zinntetrachlorid (0,51-10,28 g, 2-40 mMol) versetzt. Nach beendeter Reaktion verdünnt man und erhält man nach Waschen mit gesättigter Natriumhydrogencarbonatlösung das Rohprodukt [8], das durch Chromatographie an Kieselgel mit einem Essigester/iso-Hexan-Gradienten gereinigt wird (0,374-7,48 g, 0,85-17 mMol, ca. 85% Ausbeute).

FAB-MS: $M + H^+ = 441$.

NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

1-Thiophenyl-4,6-O-benzyliden-β-D-galactopyranose [9]

[8] (0,374-7,48 g, 0,85-17 mMol) wird in wasserfreiem Methanol (4-150 ml) gelöst und mit katalytischen Mengen Natriummethylat (hergestellt aus 0,003-0,06 g Natrium in 0,1-2,5 ml Methanol) versetzt. Nach abgeschlossener Reaktion wird mit saurem Ionenaustauscher (0,25-6 g Amberlite IR-120, $H^+$-Form) neutralisiert und eingedampft. Das erhaltene Reaktionsprodukt wird in wasserfreiem Acetonitril (4,5-150 ml) mit Benzaldehyddimethylacetal (0,19-5,05 g, 1,27-33,1 mMol) und katalytischen Mengen p-Toluolsulfonsäure (4-90 mg) bis zum Ende der Reaktion gerührt. Nach Waschen mit gesättigter Natriumhydrogencarbonatlösung entsteht das Rohprodukt [9] (0,28-5,51 g, 0,77-15,3 mMol, ca. 90 % Ausbeute).

FAB-MS: $M + H^+ = 361$.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-2,3-di-O-acetyl-6-O-benzyl-β-D-galactopyranose [10]

[9] (0,28-5,51 g, 0,77-15,3 mMol) wird in Dichlormethan (4-60 ml) suspendiert und mit Acetanhydrid (0,23-4,75 g, 2-40 mMol), Triethlyamin (0,25-5,02 g, 2,46-49,23 mMol) und katalytischen Mengen Dimethylaminopyridin (0,01-0,08 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Tetrahydrofuran (2,45-130 ml) gelöst und mit Natriumcyanoborhydrid (0,96-25,3 g, 15,4-402,8 mMol). Anschließen wird bis zur abgeschlossenen Reaktion langsam mit etherischer Salzsäurelösung versetzt. Nach Filtration wird die organische Phase mit Essigester verdünnt und mit gesättigter Natriumhydrogencarbonatlösung neutral gewaschen. Das Rohprodukt [10] wird durch Chromatographie an Kieselgel mit einem Essigester/isoHexan-Gradienten gereinigt (0,27-5,46 g, 0,62-12,24 mMol, ca. 80 % Ausbeute).

FAB-MS: $M + H^+ = 447$.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Fluor-2,3,4-tri-O-acetyl-6-O-benzyl-β-D-galactopyranose [11]

[9] (0,27-5,46 g 0,62-12,24 mMol) wird in Dichlormethan (4-60 ml) suspendiert und mit Acetanhydrid (0,14-2,91 g, 1,24-24,8 mMol), Triethlyamin (0,15-3,11 g, 1,53-30,53 mMol) und katalytischen Mengen Dimethylaminopyridin (0,003-0,04 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan bei -78°C (5-80 ml) mit Dimethylaminoschwefeltrifluorid (0,25-4,89 g, 1,86-36,7 mMol) und N-Bromsuccinimid (0,11-3,26 g, 0,93-18,36 mMol) versetzt und auf Raumtemperatur aufgewärmt. Nach Waschen mit gesättigter Natriumhydrogencarbonatlösung engt man ein und trennt die Reaktionsnebenprodukte durch Kieselgelchromatographie (Stufengradient: Essigester/iso-Hexan) ab so daß [11] entsteht (0,21-4,14 g, 0,53-10,4 mMol, ca. 85 % Ausbeute).

FAB-MS: M + $H^+$ = 399.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

3.1.3 Synthese des Fucosebausteins [17]

1-Thiophenyl-2,3,4-tri-O-acetyl-β-L-fucose [16]

Fucose [15] (0,16-32,8 g, 1-20 mMol) wird in Dichlormethan (5-90 ml) suspendiert und mit Acetanhydrid (0,75-15,43 g, 7,5-150 mMol), Triethlyamin (0,8-16,3 g, 8-160 mMol) und katalytischen Mengen Dimethylaminopyridin (0,01-0,1 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan (3,5-110 ml) gelöst und mit Thiophenol (0,22-4,41 g, 2-40 mMol) und Zinntetrachlorid (0,51-10,28 g, 2-40 mMol) versetzt. Nach beendeter Reaktion verdünnt man und erhält nach Waschen mit gesättigter Natriumhydrogencarbonatlösung das Rohprodukt [16], das durch Chromatographie an Kieselgel mit einem Essigester/iso-Hexan-Gradienten gereinigt wird (0,34-6,88 g, 0,9-18 mMol, ca. 90 % Ausbeute).

FAB-MS: M + $H^+$ = 382.

NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

1-Thiophenyl-2,3,4-tri-O-benzyl-b-L-fucose [17]

[16] (0,34-6,88 g, 0,9-18 mMol) wird in wasserfreiem Methanol (5-180 ml) gelöst und mit katalytischen Mengen Natriummethylat (hergestellt aus 0,005-0,09 g Natrium in 0,2-2,9 ml Methanol) versetzt. Nach abgeschlossener Reaktion wird mit saurem Ionenaustauscher (0,3-6,7 g Amberlite IR-120, $H^+$-Form) neutralisiert und eingedampft. Das erhaltene Reaktionsprodukt wird in wasserfreiem Dimethylformamid (5-140 ml) mit Natriumhydrid (0,16-3,21 g, 6,75-135 mMol) und Benzylbromid (0,92-18,46 g, 5,4-108 mMol) versetzt. Nach abgeschlossener Reaktion versetzt man mit Methanol (0,16-3,2 g, 5-100 mMol), verdünnt mit Essigester und wäscht nacheinander mit gesättigter Ammoniumchlorid- und gesättigter Natriumhydrogencarbonatlösung. Das Rohprodukt wird nach Chromatographie an Kieselgel (Essigester/iso-Hexan-Gradient) anschließend in trockenem Dichlormethan bei -78$^0$C (8-120 ml) mit Dimethylaminoschwefeltrifluorid (0,36-7,09 g, 2,69-53,18 mMol) und N-Bromsuccinimid (0,16-4,73 g, 1,35-26,6 mMol) versetzt und auf Raumtemperatur aufgewärmt. Nach Waschen mit gesättigter Natriumhydrogencarbonatlösung engt man ein und trennt die Reaktionsnebenprodukte durch Kieselgelchromatographie (Stufengradient: Essigester/iso-Hexan) ab, so daß [17] entsteht (0,33-6,47 g, 0,72-14,4 mMol, ca. 80 % Ausbeute).

FAB-MS: M + $H^+$ = 450.

NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

3.2 Aufbau des Disaccharids aus dem N-Acetylglucosaminbaustein und dem Galactosebaustein

1-Thiophenyl-(2,3,4-tri-O-acetyl-6-O-benzyl-β-Galactopyranosyl)-(1-4)-3-O-alkyl-2-deoxy-2-acetamido-b-D-glucopyranose [12]

Das N-Acetylglucosaminderivat [5] (0,17-4,56 g, 0,35-9,17 mMol) und das Galactosederivat [11] (0,21-4,14 g, 0,53-10,4 mMol) werden in Dichlormethan (3-120 ml) gelöst, mit Molsieb, 4 Å (0,15-7,8 g), Silberperchlorat (0,11-5,39, 0,525-26,0 mMol) und Zinn(II)chlorid (0,1-4,93 g, 0,525-26 mMol) bei 0$^0$C versetzt. Nach vollständiger Reaktion wird abfiltriert und mit gesättigter Natriumhydrogencarbonatlösung gewaschen. Das Rohprodukt [12] wird durch Chromatographie an Kieselgel mit einem Essigester/isoHexan-Gradienten gereinigt (0,23-5,61 g, 0,26-6,42 mMol, ca. 70 % Ausbeute).

FAB-MS: M + $H^+$ = 874.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-(2,3,4-tri-O-acetyl-6-O-benzyl-β-D-galactopyranosyl)-(1-4)-2-deoxy-2-acetamido-β-D-glucopyranose [13]

[12] (0,23-5,61 g, 0,26-6,42 mMol) werden in Ethanol (3-90 ml) gelöst und mit katalytischen Mengen Tetrakis-(triphenylphosphin)-ruthenium(II)hydrid (0,005-0,04 g) auf 95°C erwärmt. Nach beendeter Reaktion wird abfiltriert, eingeengt, in Methanol (2,5-85 ml) aufgenommen und mit katalytischen Mengen p-Toluolsulfonsäure (0,001-0,08 g) versetzt. Nach vollständiger Reaktion wäscht man mit gesättigter Natriumhydrogencarbonatlösung und erhält nach Einengen das Rohprodukt [13]. Die Reinigung erfolgt durch Chromatographie an Kieselgel mit einem Essigester/isoHexan-Gradienten gereinigt (0,2-4,81 g, 0,234-5,78 mMol, ca. 90 % Ausbeute).

FAB-MS: $M + H^+ = 834$.

NMR-Daten im Einklang mit K. C. Nicolaou et. al. J. Am. Chem. Soc. 114, 3127 (1992)); C. W: Hummel, Ph. D. Dissertation, University of California, San Diego, 1993.

3.3 Aufbau des Trisaccharids aus dem Disaccharid und dem Fucosebaustein

1-Thiophenyl-(2,3,4-tri-O-acetyl-6-O-benzyl-β-D-Galactopyranosyl)-(1-4)-[(2,3,4-tri-O-benzyl-α-L-fucopyranosyl)-(1-3)]-2-deoxy-2-acetamido-β-D-glucopyranose [18]

[13] (0,2-4,81 g, 0,234-5,78 mMol] werden mit [17] (0,17-4,15 g, 0,37-9,25 mMol) in trockenem Diethylether (5-100 ml) gelöst und mit Molsieb 4 Å (0,1-6,8 g), Silberperchlorat (0,15-3,59 g, 0,702-17,34 mMol), Zinn(II)chlorid (0,13-3,29 g, 0,702-17,34 mMol) bis zum Ablauf der Reaktion gerührt. Anschließend filtriert man und reinigt das nach Einengen anfallende Rohprodukt [18] durch Chromatographie an Kieselgel mit einem Essigester/isoHexan-Gradienten (0,25-6,2 g, 0,2-4,91 mMol, ca. 85 % Ausbeute).

FAB-MS: $M + H^+ = 1262$.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-(6-Aminohexyl)-O-[(-β-D-Galactopyranosyl)-(1-4)]-O-[(-α-L-fucopyranosyl)-(1-3)-]-2-deoxy-2-acetamido-β-D-glucopyranose [19]

[18] (0,25-6,2 g, 0,2-4,91 mMol] und 6-Azidohexanol (0,11-2,47 g, 0,4-9,82 mMol) werden in trockenem Acetonitril gelöst und bei 0°C mit Molsieb, 3 Å (0,1-2,5 g), Trifluormethansulfonsäuretrimethylsilylester (0,01-0,78 g, 0,02-7 mMol) versetzt. Nach abgeschlossener Reaktion wird abfiltriert und mit gesättigter Natriumhydrogencarbonatlösung gewaschen. Nach Einengen erhält man das Rohprodukt, das durch Chromatographie an Kieselgel mit einem Essigester/iso-Hexan-Gradienten gereinigt wird (0,18-4,48 g, 0,13-3,19 mMol, ca. 65 % Ausbeute). Anschließend wird in Methanol/Hydrazinhydrat (1:1) (1-50 ml) aufgenommen und bis zur vollständigen Reaktion auf 80°C erwärmt. Nach Verdampfen des Lösungsmittels wird das Rohprodukt in Dichlormethan/Methanol (1:1) aufgenommen und bei 0°C mit Acetanhydrid (1,3-40 mMol) bis zur vollständigen Reaktion gerührt. Nach Eindampfen erfolgt die Reduktion des Azids mit Wasserstoff in Gegenwart katalytischer Mengen Palladium auf Kohle (10 %) (0,01-1,5 g) in Methanol (0,5-35 ml). Das nach Eindampfen anfallende Rohprodukt wird durch Gelchromatographie an Biogel P2 gereinigt [19] (0,06-1,35 g, 0,09-2,23 mMol, ca.: 70 % Ausbeute).

FAB-MS: $M + H^+ = 608$.

$^1$H-NMR [300 MHz, DMSO]: 7,98 ($N\underline{H}_3{}^+OAc^-$), 4,92 (Fuc-$\underline{H}$-1), 4,65 ($\underline{H}$NAc), 4,36 (GlcNAc-$\underline{H}$-1), 4,28 (Gal-$\underline{H}$-1), 4,1-3,35 (m), 3,26 ($C\underline{H}_2$-$NH_3{}^+OAc^-$), 1,79 ($GlcNCOC\underline{H}_3$)1,58-1,18 (O-$(C\underline{H}_2)_5$-$CH_2$-$NH_3{}^+\underline{O}Ac^-$), 0,99 (Fuc-$C\underline{H}_3$) ppm.

4. Synthese des Tetrasaccharids

4.1 Synthese der einzelenen Zuckerbausteine

4.1.1 Synthese des N-Acetylglucosaminbausteins [6]

1,3,4,6-Tetra-O-acetyl-2-deoxy-2-phthalimido-β-D-glucopyranose [2]

D-Glucosaminhydrochlorid [1] (0,216-5,32 g, 1-20 mMol) wird zu einer Natriummethylatlösung (hergestellt aus 0,023-0,56 g Natrium und 1-20 ml Methanol) gegeben, 10 min geschüttelt und von ausgefallenen

Natriumchlorid abfiltriert. Das Filtrat wird mit Phthalsäureanhydrid (0,074-1,48 g, 0,5-10 mMol) 10 min gerührt und dann mit Triethylamin (0,1-2,02 g, 1-20 mMol) versetzt. Nach abgeschlossener Reaktion wird das Lösungsmittel eingedampft und der Rückstand unter Kühlung mit Pyridin (2-40 ml) und Acetanhydrid (5-20 ml) versetzt. Nach 16 Std. bei Raumtemperatur wird gießt man auf Eis und extrahiert das Produkt mit Dichlormethan. Nach Waschen mit Wasser, verdünnter Salzsäure und gesättigter Natriumhydrogencarbonatlösung erhält man das Rohprodukt, das aus Ether umkristallisiert wird (0,31-8,02 g, ca. 82 % Ausbeute).

FAB-MS: M + $H^+$ = 454.

NMR-Daten im Einklang mit R. U. Lemieux et. al. (ACS Symp. Ser., 39, 90 (1976)).

1-Thiophenyl-2-deoxy-2-phthalimido-β-D-glucopyranose [5]

[2] (0,31-8,02 g, 0,68-17,7 mMol) wird in 3,2-100 ml trockenem Dichlormethan gelöst, mit Thiophenol (0,15-3,9 g, 1,36-35,4 mMol) und Zinntetrachlorid (0,35-9,22 g, 1,36-35,4 mMol) versetzt und bis zur vollständigen Reaktion gerührt. Nach Verdünnen des Reaktionsgemischs wäscht man mit gesättigter Natriumhydrogencarbonatlösung. Das so erhaltene Rohprodukt wird durch Chromatographie an Kieselgel mit einem Essigester/iso-Hexan-Gradienten gereinigt (0,27-7,14 g, 0,54-14,2 mMol, ca. 80 %). [3] wird in wasserfreiem Methanol (3-90 ml) gelöst und mit katalytischen Mengen Natriummethylat (hergestellt aus 0,002-0,05 g Natrium in 0,1-2 ml Methanol) versetzt. Nach abgeschlossener Reaktion wird mit saurem Ionenaustauscher (0,2-5 g Amberlite IR-120, $H^+$-Form) neutralisiert [41 (0,21-5,31 g, 0,54-14,1 mMol, ca. 80 % Ausbeute).

FAB-MS: M + $H^+$ = 378.

NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

1-Thiophenyl-3-O-allyl-6-O-benzyl-2-deoxy-2-phthalimido-β-D-glucopyranose [6]

Das Rohprodukt [5] (0,21-5,31 g, 0,54-14,1 mMol) wird in wasserfreiem Acetonitril (3-90 ml) mit Benzaldehyddimethylacetal (0,12-3,22 g, 0,81-21,1 mMol) und katalytischen Mengen p-Toluolsulfonsäure (4-90 mg) bis zum Ende der Reaktion gerührt. Das nach Waschen mit gesättigter Natriumhydrogencarbonatlösung anfallende Rohprodukt wird in wasserfreiem Dimethylformamid (2,5-85 ml) mit Natriumhydrid (0,02-0,51 g, 0,81-21,15 mMol) und Allylbromid (0,09-2,37 g, 0,75-19,74 mMol) versetzt. Nach abgeschlossener Reaktion versetzt man mit Methanol (0,06-1,55 ml, 2,7-70,5 mMol), verdünnt mit Essigester und wäscht nacheinander mit gesättigter Ammoniumchlorid- und gesättigter Natriumhydrogencarbonatlösung. Das Rohprodukt [5] wird durch Chromatographie an Kieselgel mit einem Essigester/iso-Hexan-Gradienten gereinigt (0,23-5,98 g, 0,46-12,13 mMol, ca. 86 %) und anschließend in trockenem Tetrahydrofuran (3,5-90 ml) aufgenommen und mit Natriumcyanoborhydrid (0,67-17,7 g, 10,8-282 mMol) versetzt. Anschließend wird bis zur abgeschlossenen Reaktion langsam etherischer Salzsäurelösung zugegeben. Nach Filtration wird die organische Phase mit Essigester verdünnt und mit gesättigter Natriumhydrogencarbonatlösung neutral gewaschen. Das Rohprodukt [6] wird durch Chromatographie an Kieselgel mit einem Essigester/iso-Hexan-Gradienten gereinigt (0,17-4,56 g, 0,35-9,17 mMol, ca. 74 % Ausbeute).

FAB-MS: M + $H^+$ = 416.

NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

4.1.2 Synthese des Galactosebausteins [11]

1-Thiophenyl-2,3,4,6-tetra-O-acetyl-β-D-galactopyranose [8]

Galactose [7] (0,18-3,6 g, 1-20 mMol) wird Dichlormethan (5-90 ml) suspendiert und mit Acetanhydrid (0,75-15,4 g, 7,5-150 mMol), Triethlyamin (0,8-16,3 g, 8-160 mMol) und katalytischen Mengen Dimethylaminopyridin (0,01-0,1 g] versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan (3,5-110 ml) gelöst und mit Thiophenol (0,22-4,41 g, 2-40 mMol) und Zinntetrachlorid (0,51-10,28 g, 2-40 mMol) versetzt. Nach beendeter Reaktion verdünnt man und erhält man nach Waschen mit gesättigter Natriumhydrogencarbonatlösung das Rohprodukt [8], das durch Chromatographie an Kieselgel mit einem Essigester/iso-Hexan-Gradienten gereinigt wird (0,374-7,48 g, 0,85-17 mMol, ca. 85 % Ausbeute).

FAB-MS: M + $H^+$ = 441.
NMR-Daten im Einklang mit K. C. Nicolaou et. al. (J. Am. Chem. Soc. 112, 3695 (1990)), bzw. T. J. Caulfield, Ph. D. Dissertation, The University of Pennsylvania, 1991.

1-Thiophenyl-4,6-O-benzyliden-β-D-galactopyranose [9]

[8] (0,374-7,48 g, 0,85-17 mMol) wird in wasserfreiem Methanol (4-150 ml) gelöst und mit katalytischen Mengen Natriummethylat (hergestellt aus 0,003-0,06 g Natrium in 0,1-2,5 ml Methanol) versetzt. Nach abgeschlossener Reaktion wird mit saurem Ionenaustauscher (0,25-6 g Amberlite IR-120, $H^+$-Form) neutralisiert und eingedampft. Das erhaltene Reaktionsprodukt wird in wasserfreiem Acetonitril (4,5-150 ml) mit Benzaldehyddimethylacetal (0,19-5,05 g, 1,27-33,1 mMol) und katalytischen Mengen p-Toluolsulfonsäure (4-90 mg] bis zum Ende der Reaktion gerührt. Nach Waschen mit gesättigter Natriumhydrogencarbonatlösung entsteht das Rohprodukt [9] (0,28-5,51 g, 0,77-15,3 mMol, ca. 90 % Ausbeute).
FAB-MS: M + $H^+$ = 361.
NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-2,3-di-O-acetyl-6-O-benzyl-β-D-galactopyranose [10]

[9] (0,28-5,51 g, 0,77-15,3 mMol] wird Dichlormethan (4-60 ml) suspendiert und mit Acetanhydrid (0,23-4,75 g, 2-40 mMol), Triethlyamin (0,25-5,02 g, 2,46-49,23 mMol) und katalytischen Mengen Dimethylaminopyridin (0,01-0,08 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Tetrahydrofuran (2,45-130 ml) gelöst und mit Natriumcyanoborhydrid (0,96-25,3 g, 15,4-402,8 mMol). Anschließen wird bis zur abgeschlossenen Reaktion langsam mit etherischer Salzsäurelösung versetzt. Nach Filtration wird die organische Phase mit Essigester verdünnt und mit gesättigter Natriumhydrogencarbonatlösung neutral gewaschen. Das Rohprodukt [10] wird durch Chromatographie an Kieselgel mit einem Essigester/isoHexan-Gradienten gereinigt (0,27-5,46 g, 0,62-12,24 mMol, ca. 80 % Ausbeute).
FAB-MS: M + $H^+$ = 447.
NMR-Daten im Einklang mit K. C. Nicolaou et. al. J. Am. Chem. Soc. 114, 3127 (1992)); C. W: Hummel, Ph. D. Dissertation, University of California, San Diego, 1993.

1-Fluor-2,3,4-tri-O-acetyl-6-O-benzyl-β-D-galactopyranose [11]

[9] (0,27-5,46 g, 0,62-12,24 mMol) wird Dichlormethan (4-60 ml] suspendiert und mit Acetanhydrid (0,14-2,91 g, 1,24-24,8 mMol), Triethlyamin (0,15-3,11 g, 1,53-30,53 mMol) und katalytischen Mengen Dimethylaminopyridin (0,003-0,04 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan bei -78$^0$C (5-80 ml) mit Dimethylaminoschwefeltrifluorid (0,25-4,89 g, 1,86-36,7 mMol) und N-Bromsuccinimid (0,11-3,26 g, 0,93-18,36 mMol] versetzt und auf Raumtemperatur aufgewärmt. Nach Waschen mit gesättigter Natriumhydrogencarbonatlösung engt man ein und trennt die Reaktionsnebenprodukte durch Kieselgelchromatograpie (Stufengradient: Essigester/iso-Hexan) ab, so daß [11] entsteht (0,21-4,14 g, 0,53-10,4 mMol, ca. 85 % Ausbeute).
FAB-MS: M + $H^+$ = 399.
NMR-Daten im Einklang mit K. C. Nicolaou et. al. J. Am. Chem. Soc. 114, 3127 (1992)); C. W: Hummel, Ph. D. Dissertation, University of California, San Diego, 1993.

4.1.3 Synthese des Fucosebausteins [17]

1-Thiophenyl-2,3,4-tri-O-acetyl-β-L-fucose [16]

Fucose [15] (0,16-32,8 g, 1-20 mMol) wird in Dichlormethan (5-90 ml) suspendiert und mit Acetanhydrid (0,75-15,43 g, 7,5-150 mMol), Triethlyamin (0,8-16,3 g, 8-160 mMol) und katalytischen Mengen Dimethylaminopyridin (0,01-0,1 g) versetzt. Nach beendeter Reaktion gießt man auf Eiswasser und extrahiert das Produkt mit Dichlormethan. Das Rohprodukt, das durch Waschen des Extrakts mit verdünnter Salzsäure und gesättigter Natriumhydrogencarbonatlösung erhalten wird, wird in trockenem Dichlormethan (3,5-110

ml) gelöst und mit Thiophenol (0,22-4,41 g, 2-40 mMol) und Zinntetrachlorid (0,51-10,28 g, 2-40 mMol) versetzt. Nach beendeter Reaktion verdünnt man und erhält man nach Waschen mit gesättigter Natriumhydrogencarbonatlösung das Rohprodukt [16], das durch Chromatographie an Kieselgel mit einem Essigester/iso-Hexan-Gradienten gereinigt wird (0,34-6,88 g, 0,9-18 mMol, ca. 90 % Ausbeute).

FAB-MS: M + $H^+$ = 382.

NMR-Daten im Einklang mit K. C. Nicolaou et. al. J. Am. Chem. Soc. 114, 3127 (1992)); C. W: Hummel, Ph. D. Dissertation, University of California, San Diego, 1993.

1-Thiophenyl-2,3,4-tri-O-benzyl-β-L-fucose [17]

[16] (0,34-6,88 g, 0,9-18 mMol) wird in wasserfreiem Methanol (5-180 ml) gelöst und mit katalytischen Mengen Natriummethylat (hergestellt aus 0,005-0,09 g Natrium in 0,2-2,9 ml Methanol) versetzt. Nach abgeschlossener Reaktion wird mit saurem Ionenaustauscher (0,3-6,7 g Amberlite IR-120, $H^+$-Form) neutralisiert und eingedampft. Das erhaltene Reaktionsprodukt wird in wasserfreiem Dimethylformamid (5-140 ml) mit Natriumhydrid (0,16-3,21 g, 6,75-135 mMol) und Benzylbromid (0,92-18,46 g, 5,4-108 mMol) versetzt. Nach abgeschlossener Reaktion versetzt man mit Methanol (0,16-3,2 g, 5-100 mMol), verdünnt mit Essigester und wäscht nacheinander mit gesättigter Ammoniumchlorid- und gesättigter Natriumhydrogencarbonatlösung. Das Rohprodukt wird nach Chromatographie an Kieselgel (Essigester/iso-Hexan-Gradient) anschließend in trockenem Dichlormethan bei -78°C (8-120 ml) mit Dimethylaminoschwefeltrifluorid (0,36-7,09 g, 2,69-53,18 mMol) und N-Bromsuccinimid (0,16-4,73 g, 1,35-26,6 mMol) versetzt und auf Raumtemperatur aufgewärmt. Nach Waschen mit gesättigter Natriumhydrogencarbonatlösung engt man ein und trennt die Reaktionsnebenprodukte durch Kieselgelchromatographie (Stufengradient: Essigester/iso-Hexan) ab, so daß [17] entsteht (0,33-6,47 g, 0,72-14,4 mMol, ca. 80 % Ausbeute).

FAB-MS: M + $H^+$ = 450.

NMR-Daten im Einklang mit K. C. Nicolaou et. al. J. Am. Chem. Soc. 114, 3127 (1992)); C. W: Hummel, Ph. D. Dissertation, University of California, San Diego, 1993.

4.1.4 Synthese des Neuraminsäurebausteins [22]

Methyl-5-acetamido-4,7,8,9-tetra-O-benzyl-2,3,5-trideoxy-2,3-dehydro-D-glycero-α-D-galacto-2-nonulopyranosylonat [21]

N-Acetylneuraminsäure [20] (0,31-6,18 g, 1-20 mMol) wird in Essigsäureanhydrid (3-100 ml) gegeben und mit katalytischen Mengen Schwefelsäure (0,001-0,5 g) versetzt. Nach vollständiger Reaktion gießt man auf Eiswasser und extrahiert das Rohprodukt mit Essigsäureethylester. Die Extrakte werden mit gesättigter Natriumhydrogencarbonatlösung gewaschen und eingeengt. Das Rohprodukt wird mit Wasser vermischt und anschließend mit 1 M Natronlauge versetzt. Nach dem Ablauf der Reaktion wird das Lösungsmittel entfernt und das Rohprodukt in Dimethylformamid (5-260 ml) aufgenommen. Anschließend versetzt man mit Natriumhydroxid (0,03-7,2 g, 0,8-180 mMol), Benzylbromid (0,14-30,78 g, 0,8-180 mMol) sowie mit katalytischen Mengen Tetrabutylammoniumjodid (0,005-0,75 g) und erhitzt bis zur abgeschlossenen Reaktion auf 60°C. Danach versetzt man mit Methanol (1,5-35 ml) und bringt das Reaktionsgemisch durch Zugabe von 1M wässriger Salzsäure auf pH 2. Das Rohprodukt erhält man durch Extraktion mit Essigsäureethylester und waschen der Extrakte mit gesättigter Natriumhydrogencarbonatlösung. Die Reinigung erfolgt durch Chromatographie an Kieselgel mit einem Dichlormethan/Methanol-Gradienten [21] (0,29-5,77 g, 0,45-9 mMol, Ausbeute ca. 45 %).

FAB-MS: M + $H^+$ = 642.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

Chlor-(methyl-5-acetamido-4,7,8,9-tetra-O-benzyl-3,5-dideoxy-3-thiophenyl-D-glycero-α-D-galacto-2-nonulopyranosylonat [22]

[21] (0,29-5,77 g, 0,45-9 mMol) werden in Dichlormethan (2,5-80 ml) mit Phenylsulfenylchlorid verletzt. Nach Ablauf der Reaktion wäscht man mit gesättigter Natriumhydrogencarbonatlösung und engt ein. Das Rohprodukt [22] wird durch Chromatographie an Kieselgel mit einem Essigester/isoHexan-Gradienten gereinigt (0,29-5,83 g, 0,36-7,2 mMol, ca. 80 % Ausbeute).

FAB-MS: M + $H^+$ = 810.

NMR-Daten im Einklang mit K. C. Nicolaou et. al. J. Am. Chem. Soc. 114, 3127 (1992)); C. W: Hummel, Ph.

D. Dissertation, University of California, San Diego, 1993.

4.2 Aufbaus des Disaccharids aus dem N-Acetylglucosaminbaustein und dem Galactosebaustein

1-Thiophenyl-(2,3,4-tri-O-acetyl-6-O-benzyl-β-D-Galactopyranosyl)-(1-4)-3-O-alkyl-2-deoxy-2-acetamido-β-D-Glucopyranose [12]

Das N-Acetylglucosaminderivat [5] (0,17-4,56 g, 0,35-9,17 mMol) und das Galactosederivat [11] (0,21-4,14 g, 0,53-10,4 mMol) werden in Dichlormethan (3-120 ml) gelöst, mit Molsieb, 4 Å, (0,15-7,8 g), Silberperchlorat (0,11-5,39; 0,525-26 mMol) und Zinn(II)chlorid (0,1-4,93 g, 0,525-26 mMol) bei 0°C versetzt. Nach vollständiger Reaktion wird abfiltriert und mit gesättigter Natriumhydrogencarbonatlösung gewaschen. Das Rohprodukt [12] wird durch Chromatographie an Kieselgel mit einem Essigester/isoHexan-Gradienten gereinigt (0,23-5,61 g, 0,26-6,42 mMol, ca. 70 % Ausbeute).

FAB-MS: M + $H^+$ = 874.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Thiophenyl-(2,3,4-tri-O-acetyl-6-O-benzyl-β-D-galactopyranosyl)-(1-4)-2-deoxy-2-acetamido-β-D-glucopyranose [13]

[12] (0,23-5,61 g, 0,26-6,42 mMol) werden in Ethanol (3-90 ml) gelöst und mit katalytischen Mengen Tetrakis-(triphenylphosphin]-ruthenium(II)hydrid (0,005-0,04 g) auf 95°C erwärmt. Nach beendeter Reaktion wird abfiltriert, eingeengt, in Methanol (2,5-85 ml) aufgenommen und mit katalytischen Mengen p-Toluolsulfonsäure (0,001-0,08 g) versetzt. Nach vollständiger Reaktion wäscht man mit gesättigter Natriumhydrogencarbonatlösung und erhält nach Einengen das Rohprodukt [13]. Die Reinigung erfolgt durch Chromatographie an Kieselgel mit einem Essigester/isoHexan-Gradienten gereinigt (0,2-4,8 g, 0,234-5,78 mMol, ca. 90 % Ausbeute).

FAB-MS: M + $H^+$ = 834.

NMR-Daten im Einklang mit K. C. Nicolaou et. al. J. Am. Chem. Soc. 114, 3127 (1992)); C. W: Hummel, Ph. D. Dissertation, University of California, San Diego, 1993.

4.3 Aufbau des Trisaccharids aus dem Disaccharid und dem Fucosebaustein

1-Thiophenyl-(2,3,4-tri-O-acetyl-6-O-benzyl-β-D-Galactopyranosyl)-(1-4)-[(2,3,4-tri-O-benzyl-α-L-fucopyranosyl)-(1-3)]-2-deoxy-2-acetamido-β-D-glucopyranose [18]

[13] (0,2-4,81 g, 0,234-5,78 mMol) wird mit [17] (0,17-4,15 g, 0,37-9,25 mMol) in trockenem Diethylether (5-100 ml) gelöst und mit Molsieb, 4 Å, (0,1-6,8 g), Silberperchlorat (0,15-3,59 g, 0,702-17,34 mMol), Zinn-(II)chlorid (0,13-3,29 g, 0,702-17,34 mMol) bis zum Ablauf der Reaktion gerührt. Anschließend filtriert man und reinigt das nach Einengen anfallende Rohprodukt [18] durch Chromatographie an Kieselgel mit einem Essigester/isoHexan-Gradienten (0,25-6,2 g, 0,2-4,91 mMol, ca. 85 % Ausbeute).

FAB-MS: M + $H^+$ = 1262.

NMR-Daten im Einklang mit K. C. Nicolaou et. al. J. Am. Chem. Soc. 114, 3127 (1992)); C. W: Hummel, Ph. D. Dissertation, University of California, San Diego, 1993.

4.4 Aufbau des Tetrasaccharids aus dem Trisaccharid und dem N-Acetylglucosaminbaustein

1-(o-Nitrobenzyl)-(6-O-benzyl-β-D-Galactopyranosyl)-(1-4)-[(2,3,4-tri-O-benzyl-α-L-fucopyranosyl)-(1-3)]-2-deoxy-2-acetamido-β-D-glucopyranose [23]

[18] (0,25-6,2 g, 0,2-4,91 mMol) wird in trockenem Dichlormethan bei -78°C (3-85 ml) mit Dimethylaminoschwefeltrifluorid (0,08-1,93 g, 0,6-14,73 mMol) und N-Bromsuccinimid (0,05-1,23 g, 0,28-6,87 mMol) versetzt und auf Raumtemperatur aufgewärmt. Nach Waschen mit gesättigter Natriumhydrogencarbonatlösung engt man ein und trennt die Reaktionsnebenprodukte durch Kieselgelchromatographie (Stufengradient: Essigester/iso-Hexan) ab [22] (0,33-6,47 g, 0,72-14,4 mMol, ca. 80 % Ausbeute). Das Reaktionsprodukt wird in Dichlormethan gelöst und mit o-Nitrobenzylalkohol (0,08-1,88 g, 0,5-12,28 mMol), Molsieb, 4 Å, (0,1-7,5 g), Silbertrifluormethansulfonat (0,18-4,42 g, 0,7-17,19 mMol), Hafnocendichlorid (0,27-6,53 g, 0,7-17,19 mMol) bei -78°C versetzt und auf Raumtemperatur aufwärmen gelassen. Nach Abfiltrieren wäscht man mit

gesättigter Natriumhydrogencarbonatlösung und engt ein. Das so erhaltene Rohprodukt wird durch Chromatographie an Kieselgel (Dichlormethan/Methanol-Gradient) in trockenem Methanol (3-80 ml) gelöst und mit katalytischen Mengen Natriummethylat (hergestellt aus 0,001-0,09 g Natrium in 0,2-2,5 ml Methanol) versetzt. Nach abgeschlossener Reaktion wird mit saurem Ionenaustauscher (0,2-6,5 g Amberlite IR-120, $H^+$-Form] neutralisiert. Nach Eingedampft erhält man [23].

FAB-MS: M + $H^+$ = 1186.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-(O-N-Nitrobenzyl)-[(methyl-5-acetamido-4,7,8,9-tetra-O-benzyl-3,5-dideoxy-D-glycero-α-D-galacto-2-nonulopyranosylonatyl)-(2-3)-(6-O-benzyl-β-D-Galactopy ranosyl)-(1-4)]-(2,3,4-tri-O-benzyl-α-L-fucopyranosyl)-(1-3)]-2-deoxy-2-acetamido -β-D-glucopyranose [24]

Das erhaltene Reaktionsprodukt [23] wird mit [22] (0,1-2,81 g, 0,12-3,47 mMol) in Tetrachlorkohlenstoff gelöst, bei 0°C mit Molsieb, 4 Å, (0,08-0,7 g), Quecksilber(II)cyanid (0,13-2,67 g, 0,36-10,43 mMol) und Quecksilber(II)bromid (0,14-1,25 g, 0,12-3,47 mMol) versetzt und langsam auf Raumtemperatur aufgewärmt. Nach Waschen mit gesättigter Natriumhydrogencarbonatlösung wird eingeengt und das Rohprodukt durch Chromatographie mit einem Essigester/isoHexan-Gradienten an Kieselgel gereinigt [24] (0,14-3,36 g, 0,07-1,72 mMol, ca. 35 % Ausbeute).

FAB-MS: M + $H^+$ = 1959.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

1-Fluor-[(methyl-5-acetamido-4,7,8,9-tetra-O-benzyl-3,5-dideoxy-D-glucero-α-D-galacto-2-nonulopyranosylonatyl)-(2,3)-(6-O-benzyl-2,4-di-O-acetyl-β-D-galactopyranosyl)-(1-4)-(2,3,4-tri-O-benzyl-α-L-fucopyranosyl)-(1-3)-2-deoxy-2-acetamido-β-D-glucopyranose

[24] (0,14-3,36 g, 0,07-1,72 mMol) wird in Essigsäureanhydrid (0,5-10 ml) und Pyridin (0`5-10 ml) gelöst und mit katalytischen Mengen Dimethylaminopyridin (0,001-0,1 g) versetzt. Nach vollständiger Reaktion gießt man auf Eiswasser und extrahiert das Rohprodukt mit Essigsäureethylester. Anschließend wäscht man nacheinander mit verdünnter Salzsäure und gesättigter Natriumhydrogencarbonatlösung. Das Reaktionsprodukt wird in einem 10:1-Tetrahydrofuran-Wasser-Gemisch (0,5-30 ml) aufgenommen und bei 0°C mit einer UV-Lampe bestrahlt. Nach Ablauf der Reaktion wird eingeengt, in Toluol (0,5-60 ml) aufgenommen und mit Triphenylzinnhydrid (0,25-6,04 g; 0,7-17,2 mMol) sowie Azoisobutyronitril (0,01-0,25 g, 0,06-1,55 mMol) am Rückfluß erhitzt. Nach Ablauf der Reaktion wird eingeengt und das Reaktionsprodukt an Kieselgel mit einem Dichlormethan/Methanol-Gradienten chromatographiert (0,036-0,92 g, 0,02-0,52 mMol, ca. 30 % Ausbeute).

FAB-MS: M + $H^+$ = 1644.

NMR-Daten im Einklang mit K. C. Nicolaou et. al. J. Am. Chem. Soc. 114, 3127 (1992)); bzw. C. W: Hummel, Ph. D. Dissertation, University of California, San Diego, 1993.

Das gereinigte Produkt wird in trockenem Dichlormethan (0,1-10 ml) gelöst und bei -78°C mit Diethylaminoschwefeltrifluorid (0,016-0,41 g, 0,12-3,1 mMol) versetzt. Nach abgelaufener Reaktion extrahiert man mit gesättigter Natriumhydrogencarbonatlösung und erhält das Rohprodukt [25] nach Einengen, das durch Chromatographie an Kieselgel mit einem Dichlormethan/Methanol-Gradienten gereinigt wird.

1-(6-Aminohexyl)-O-[(methyl-5-acetamido-3,5-dideoxy-D-glycero-α-D-galacto-2-nonulopyranosylonatyl)-(2-3)-(-β-D-Galactopyranosyl)-(1-4)]-(α-L-fucopyranosyl)-(1-3)-2-deoxy-2-acetamido-β-D-glucopyranose [26]

[25] und 6-Azidohexanol (0,017-0,44 g, 0,12-3,1 mMol) werden in trockenem Acetonitril gelöst und bei 0°C mit Molsieb, 4 Å, (0,06-1,5 g), Trifluormethansulfonsäuretrimethylsilylester (0,001-1,14 g, 0,005-5,1 mMol) versetzt. Nach abgeschlossener Reaktion wird abfiltriert und mit gesättigter Natriumhydrogencarbonatlösung gewaschen. Nach Einengen erhält man das Rohprodukt das durch Chromatographie an Kieselgel mit einem Essigester/iso-Hexan-Gradienten gereinigt wird (0,021-0,51 g, 0,01-0,27 mMol, ca. 55 % Ausbeute). Anschließend wird in Methanol/Hydrazinhydrat (1:1) (1-30 ml) aufgenommen und bis zur vollständigen Reaktion auf 80°C erwärmt. Nach Verdampfen des Lösungsmittels wird das Rohprodukt in Dichlormethan/Methanol (1:1) aufgenommen und bei 0°C mit Acetanhydrid (0,03-4,1 mMol) bis zur vollständigen Reaktion gerührt. Nach Eindampfen erfolgt die Reduktion des Azids mit Wasserstoff in Gegenwart katalytischer Mengen Palladium auf Kohle (10 %) (0,001-0,15 g) in Methanol (1-45 ml). Das nach

Eindampfen anfallende Rohprodukt wird durch Gelchromatographie an Biogel P2 gereinigt [26] (0,0064-0,17 g, 0,007-0,18 mMol, ca. 65 % Ausbeute).
FAB-MS: M + $H^+$ = 920.
$^1$H-NMR [300 MHz, $D_2O$]: 4,92 (Fuc-H-1), 4,36 (GlcNAc-H-1), 4,28 (Gal-H-1), 4,1-3,35 (m), 3,29 ($CH_2$-$NH_3^+OAc^-$), 2,19 (NANA-H-3eq.), 1,93 (NANA-NCOC$H_3$), 1,81 (GlcNCOC$H_3$), 1,77 (NANA-H-3ax.), 1,58-1,18 (O-($CH_2$)$_5$-$CH_2$-$NH_3^+OAc^-$), 0,99 (Fuc-$CH_3$) ppm.

Umsetzung des Polymers mit dem Wirkungsverstärker einerseits und des Kohlenhydratanteils mit dem bifunktionellen Spacer andererseits unter Ausbildung kovalenter Bindungen.

5. Poly-D,L-succinimid-co-aspartamido-$C_6$-GlcNAc (Belegungsgrad: 12 %) (= Poly-D,L-succinimid-co-α,β-D,L-aspartamido-6-hexyl-2-deoxy-2-acetamido-β-D-Glucopyranose)

PSI (1 g, 10,3 µmol, MW: 24.200) wird in DMF (= Dimethylformamid, 4 ml, getrocknet u. dest.) vorgelegt und mit 1-(6-Aminohexyl)-2-deoxy-2-acetamido-β-D-glucopyranose ($H_2N$-$C_6$-GlcNAc) (0,45 g, 1,41 mmol) in 4 ml DMF versetzt. Der Ansatz wird 4 Std. bei Raumtemperatur unter $N_2$ gerührt. Danach wird in 80 ml 1-Butanol gefällt und das erhaltene Polymer mit Methanol gewaschen. Nach einer zweiten Fällung aus DMF in 1-Butanol wird erneut mit Methanol gewaschen und anschließend im Ölpumpenvakuum getrocknet.

Ausbeute: 1,2 g (86 %)
Substitutionsgrad (DS) laut NMR: 12 %

6. PHEA-co-aspartamido-$C_6$-GlcNAc (Belegungsgrad: 12 %) (= Poly-α,β-(2-hydroxyethyl)-D,L-aspartamid-co-α,β-D,L-aspartamido-6-hexyl-2-deoxy-2-acetamido-β-D-Glucopyranose)

PSI-co-aspartamido-$C_6$-GlcNAc aus Beispiel 5 (0,8 g, 5,9 µmol) wird in 6 ml DMF gelöst und mit frisch dest. Hydroxyethylamin (325 mg, 5,3 mmol) versetzt. Nach 4 Std. Rühren unter $N_2$ bei Raumtemperatur wird in 1-Butanol gefällt. Das erhaltene weiße, flockige Polymer wird mit Methanol gewaschen, in $H_2O$ gelöst und gefriergetrocknet. Nach der zweiten Fällung aus DMF/$H_2O$ (15:1) in 1-Butanol wird das Produkt erneut mit Methanol gewaschen und aus $H_2O$ gefriergetrocknet.

Ausbeute: 1 g (90 %)
DS laut NMR: 12 % $H_2N$-$C_6$-GlcNAc, 88 % HEA
$^1$H-NMR (300 MHz, $D_2O$): 1,3 (-($CH_2$)$_2$-), 1,5 (-$CH_2$-$CH_2$-NHCO- und -$CH_2$-$CH_2$-O-GlcNAc), 2,04 (GlcNCO$CH_3$), 2,8 (Asp. -$CH_2$-CO), 3,35 (-CONH-$CH_2$-$CH_2$-OH), 3,65 (-CONH-$CH_2$-$CH_2$-OH), 4,5 (GlcNAc-H-1), 4,7 (Asp. -CH(NH-)-CO).

7. Poly-succinimid-co-HEA-co-aspartamido-$C_6$-GlcNAc (Belegungsgrad: 12 %) (= Poly-D,L-succimid-co-α,β-(2-hydroxyethyl)-D,L-aspartamid-co-D,L-aspartamido-6-hexyl-2-deoxy-2-acetamido-β-D-Glucopyranose

PSI-co-aspartamido-$C_6$-GlcNAc aus Beispiel 5 (0,4 g, 3 µmol) und Hydroxyethylamin (0,1 g, 1,6 mmol) werden analog Beispiel 6 umgesetzt.

Ausbeute: 380 mg (78 %)
DS laut NMR: 53 % HEA, 12 % $H_2N$-$C_6$-GlcNAc

8. Poly-succinimid-co-aspartamido-$C_6$-GlcNAc (Belegungsgrad: 22 %) (= Poly-D,L-succimid-co-α,β-D,L-aspartamido-6-hexyl-2-deoxy-2-acetamido-β-D-Glucopyranose)

Analog zu Beispiel 5 werden 0,32 g (3,3 mmol) PSI mit 0,3 g (1,0 mmol) $H_2N$-$C_6$-GlcNAc zur Reaktion gebracht und durch Fällung aufgearbeitet.

Ausbeute: 400 mg (72 %)
DS laut NMR: 22 %

9. Poly-succinimid-co-HEA-co-aspartamido-$C_6$-GlcNAc (Belegungsgrad: 22 %) (= Poly-D,L-succimid-co-α,β-(2-hydroxyethyl)-D,L-aspartamid-co-D,L-aspartamido-6-hexyl-2-deoxy-2-acetoamido-β-D-Glucopyranose)

PSI-co-aspartamido-$C_6$-GlcNAc aus Beispiel 8 (0,4 g, 2,39 mmol) und Hydroxyethylamin (0,1 g, 1,67 mmol) werden analog zu Beispiel 6 umgesetzt.

Ausbeute: 410 mg (84 %)
DS laut NMR: 22 % 4, 60 % HEA

10. Poly-HEA-co-3-dimethylamino-1-propylamidoaspartamid-co-aspartamido-$C_6$-GlcNAc (Belegungsgrad: 12 %) ( = Poly-α,β-(2-hydroxyethyl)-D,L-aspartamid-co-α,β-(3-dimethylamino-1-propylamido-D,L-aspartamid-co-D,L-aspartamido-6-hexyl-2-deoxy-2-acetamido-β-D-Glucopyranose)

PSI (0,1 g, 1,0 µmol) wird in 1 ml DMF gelöst, mit $H_2N$-$C_6$-GlcNAc (45 mg, 0,14 mmol) und Hydroxyethylamin 12 mg, 0,2 mmol) versetzt. Nach 3 Std. bei 35 °C wird frisch dest. 3-Dimethylamino-1-propylamin (105 mg, 0,1 mmol) und nach weiteren 3 Std. bei 35 °C Hydroxyethylamin (37 mg, 0,6 mmol) zugegeben. Der Ansatz wird 3 Std. gerührt und analog Beispiel 6 aufgearbeitet.
Ausbeute: 160 mg (82 %)
DS laut NMR: 78 % HEA, 10 % Dimethylaminopropylamid, 12 % $H_2N$-$C_6$-GlcNAc

11. Poly-HEA-co-4-aminobutyl-1-amidoaspartamid-co-aspartamido-$C_6$-GlcNAc (Belegungsgrad: 12 %) ( = Poly-α,β-(2-hydroxyethyl)-D,L-aspartamid-co-α,β-4-aminobutyl-1-amido-D,L-aspartamid-co-D,L-aspartamido-6-hexyl-2-deoxy-2-acetamido-β-D-Glucopyranose)

PSI (0,75 g, 7,7 µmol) wird in getrocknetem und dest. DMF (150 ml) gelöst, mit 1,4-Diaminobutan (34 mg, 0,39 mmol) versetzt und 2 Tage bei Raumtemperatur gerührt. Im Ölpumpenvakuum wird bis auf ca. 1 ml eingeengt, mit $H_2N$-$C_6$-GlcNAc (34 mg, 1,1 mmol) versetzt und 3 Std. bei 35 °C gerührt. Nach Zugabe von Hydroxyethylamin (0,47 g, 7,7 mmol) wird weitere 3 Std. bei 35 °C gerührt und anschließend analog Beispiel 6 aufgearbeitet.
Ausbeute: 100 mg (70 %)
DS laut NMR: 83 % HEA, 12 % $H_2N$-$C_6$-GlcNAc, 5 % Diaminobutan

12. Poly-HEA-co-aspartamido-$C_6$-LacNAc ( = Poly-α,β-(2-hydroxyethyl)-D,L-aspartamid-co-D,L-aspartamido-6-hexyl-O-(β-D-Galactopyranosyl)-(1-4)-2-deoxy-2-acetamido-β-D-glycopyranose)

PSI (70 mg, 0,72 µmol) wird in 0,5 ml DMF gelöst, mit einer Lösung von Hydroxyethylamin (9 mg, 0,147 mmol) und $H_2N$-$C_6$-LacNAc (41 mg, 0,086 mmol) in 0,5 ml DMF/NMP (10:1) versetzt und bei 35 °C 6 Std. gerührt. Nach Zugabe von Hydroxyethylamin (35 mg, 0,58 mmol) wird weitere 3 Std. gerührt. Die Aufarbeitung erfolgt analog Beispiel 6.
Ausbeute: 90 mg (66 %)
DS laut NMR: 93 % HEA, 7 % $H_2N$-$C_6$-LacNAc
$^1$H-NMR (300 MHz, $D_6$DMSO): 1,2 (-$(CH_2)_2$-), 1,4 (-$CH_2$-$CH_2$-NHCO- und -$CH_2$-$CH_2$-O-LacNAc), 1,80 (GlcNCO$CH_3$), 3,15 (-CONH-$CH_2$-$CH_2$-OH), 4,2 (Gal-H-1), 4,3 (GlcNAc-H-1), 4,7 (Asp. -CH(NH-)-CO).

13. Poly-HEA-co-aspartamido-$C_6$-Sialyl Lewis X ( = Poly-α,β-(2-hydroxyethyl)-D,L-aspartamid-co-D,L-aspartamido-6-hexyl-O-[(methyl-5-acetamido-3,5-dideoxy-D-glycero-α-D-galacto-2-nonulopyranosylonatyl)-(2-3)-(β-D-Galactopyranosyl)-(1-4)]-(α-L-fucopyranosyl)-(1-3)-2-deoxy-2-acetamido-β-D-glucopyranose)

PSI (70 mg, 0,72 µmol) wird in 0,5 ml DMSO gelöst, mit Hydroxyethylamin (18 mg, 0,3 mmol) versetzt und 2 Std. bei 35 °C gerührt. Verbindung Nr. 26 (s. S. 91) (46 mg, 0,05 mmol) wird mit Triethylamin (5 mg) in 0,2 ml $H_2O$ gelöst, zum Polymeransatz gegeben und 6 Std. bei 35 °C gerührt. Danach läßt man unter Zugabe von $H_2O$ mit zusätzlichem Hydroxylamin (27 mg, 0,45 mmol) weitere 4 Std. reagieren. Die Aufarbeitung erfolgt analog Beispiel 6.
Ausbeute: 92 mg (64 %)
DS laut NMR: 95 % HEA, 5 % Verbindung Nr. 26

14) Poly-L-Lysin-(2-hydroxyethyl)amid-co-L-lysinamido-$C_6$-GlcNAc-fumaramid (Belegungsgrad: 13 %) ( = Poly-L-Lysin-(2-hydroxyethyl)-amid-co-L-lysinamido-6-hexyl-2-deoxy-2-acetamido-β-D-Glucopyranose-fumaramid)

Poly-L-Lysinmethylester-fumaramid (240 mg, 1 µmol) wird in 5 ml DMSO bei 80 °C gelöst. Nach Abkühlung auf Raumtemperatur wird mit $H_2N$-$C_6$-GlcNAc (48 mg, 0,15 mmol) versetzt und 4 Std. bei 35 °C gerührt. Hydroxyethylamin (101 mg, 1 mmol) wird zugegeben und der Ansatz für weitere 3 Std. bei Raumtemperatur gerührt. Zur Aufarbeitung wird zweimal in Aceton gefällt und aus $H_2O$ gefriergetrocknet.

Ausbeute: 166 mg (55 %)
DS laut NMR: 87 % HEA, 13 % $H_2N-C_6$-GlcNAc

## Patentansprüche

1. Physiologisch verträglicher und physiologisch abbaubarer Kohlenhydratrezeptorblocker auf Polymerbasis mit einem HLB*-Wert von 10 bis 20 enthaltend:
Kohlenhydratanteil - bifunktionellen Spacer - hydrophiles, biodegradabeles Polymer - Wirkungsverstärker,
wobei der aus 1 bis 20 natürlich vorkommenden gleichen oder verschiedenen Monosaccharidbausteinen bestehende Kohlenhydratanteil via ein oder mehrere bifunktionelle Spacer, der ein natürliches oder synthetisches Molekül ist, an ein hydrophiles, biodegradabeles Polymer gekoppelt ist, wobei das Polymer seinerseits Träger ein oder mehrerer Spacer ist und das hydrophile, biodegradable Polymer außerdem mit dem Wirkungsverstärker verbunden ist, welcher ein Vernetzer, ein oder mehrere Gruppen mit hydrophoben, hydrophilen oder ionischen Eigenschaften oder ein Löslichkeitsverbesserer ist.

2. Physiologisch verträglicher und physiologisch abbaubarer Kohlenhydratrezeptorblocker auf Polymerbasis mit einem HLB*-Wert von 10 bis 20, erhältlich durch kovalente chemische, chemoenzymatische und/oder enzymatische Verknüpfung der folgenden Komponenten:
   - Kohlenhydratanteil aus 1-20 natürlich vorkommenden, gleichen oder verschiedenen Monosaccharid-Bausteinen,
   - bifunktionellem Spacer, der ein natürliches oder synthetisches Molekül ist,
   - hydrophiles, biodegradabeles Polymer sowie
   - Wirkungsverstärker, der ein Vernetzer, ein oder mehrere Gruppen mit hydrophoben, hyrophilen oder ionischen Eigenschaften oder ein Löslichkeitsverbesserer ist.

3. Kohlenhydratrezeptorblocker nach Anspruch 1 oder 2, dadurch gekennzeichnet, daß sein Molekulargewicht $\leqq$ 70 kd ist.

4. Kohlenhydratrezeptorblocker nach Anspruch 1 oder 2, dadurch gekennzeichnet, daß der HLB*-Wert des Polymers 14 bis 20 ist.

5. Kohlenhydratrezeptorblocker nach Anspruch 1 oder 2, dadurch gekennzeichnet, daß der Belegungsgrad des Polymers mit dem Kohlenhydratanteil via Spacer zwischen 0,5 und 50 % liegt.

6. Kohlenhydratrezeptorblocker nach Anspruch 1 oder 2, dadurch gekennzeichnet, daß der Kohlenhydratanteil Bestandteil von Glycoproteinen, Glycolipiden oder Proteoglycanen auf Virus- oder Zelloberflächen ist.

7. Kohlenhydratrezeptorblocker nach Anspruch 1 oder 2, dadurch gekennzeichnet, daß der Kohlenhydratanteil aus 1-15 natürlich vorkommenden gleichen oder verschiedenen Monosaccharidbausteinen besteht, die substituiert und/oder unsubstituiert sind.

8. Kohlenhydratrezeptorblocker nach Anspruch 1, 2, oder 7, dadurch gekennzeichnet, daß substituierte Monosaccharidbausteine mit einer Kohlenstoffkette, die länger als 6 C-Atome ist, verwendet werden.

9. Kohlenhydratrezeptorblocker nach Anspruch 1, 2 oder 6, dadurch gekennzeichnet, daß der Kohlenhydratanteil Sialyl-Lewis X, Sialyl-Lewis A oder VIM-2 ist.

10. Kohlenhydratrezeptorblocker nach Anspruch 1 oder 2, dadurch gekennzeichnet, daß der bifunktionelle Spacer die folgende Formel hat: (Kohlenhydratanteil)-$A_1$-$[B-C_x]_y$-$D_z$-$A_2$-(biodegradables, hydrophiles Polymer-Wirkungsverstärker),
wobei
x für 0 oder 1;
y für 1, 2, 3, 4, 5, 6 oder 7;
z für 0 oder 1 steht, mit der Maßgabe, daß z nur 1 sein kann, wenn x = 1 ist;
B, D gleich oder verschieden sind und Alkylen mit 0 bis 30 C-Atomen, die geradkettig, verzweigt oder

mono- oder bicyclisch sein können, unsubstituiert oder substituiert sind, Einfach- und/oder Doppelbindungen oder 1 bis 3 aromatische Ringe bedeutet,
C einen Substituenten der folgenden Formel bedeutet:

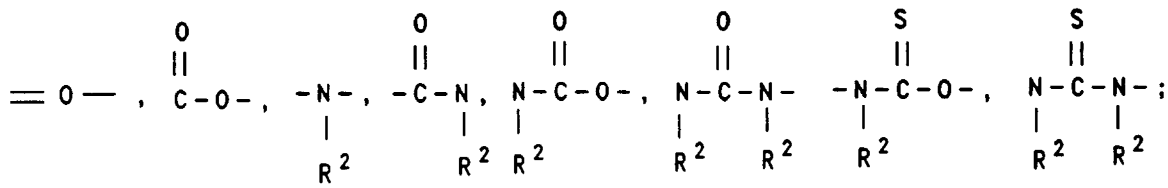

wobei $R^2$ Wasserstoff, Methyl-, Ethyl-, Benzyl-, Propyl-, Aceyl- oder Benzoylgruppe unabhängig voneinander bedeuten kann;
die durch den Substituenten y definierten, repetitiven Einheiten gleich oder verschieden sein können;
$A_1$ und $A_2$ gleich oder verschieden sind und aus der kovalenten Verknüpfung von je einer reaktiven mit einer aktivierten Gruppe entstehen.

**11.** Kohlenhydratrezeptorblocker nach Anspruch 1, 2 oder 10, dadurch gekennzeichnet, daß B oder D unabhängig voneinander 0 bis 15 C-Atome bedeuten.

**12.** Kohlenhydratrezeptorblocker nach Anspruch 1, 2 oder 10, dadurch gekennzeichnet, daß C einen Substituenten der folgenden Formel bedeutet:
Bevorzugt sind =

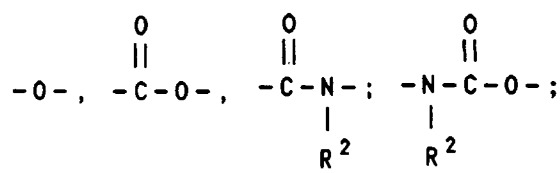

wobei $R^1$ die schon genannten Bedeutungen haben kann.

**13.** Kohlenhydratrezeptorblocker nach Anspruch 1 oder 2, dadurch gekennzeichnet, daß das hydrophile, biodegradabele Polymer aus wenigstens zwei gleichen oder verschiedenen Monomerbausteinen besteht, die linear, verzweigt, ringförmig, sternförmig oder kugelförmig miteinander verknüpft sind.

**14.** Kohlenhydratrezeptorblocker nach Anspruch 13, dadurch gekennzeichnet, daß Polycarbonate, Polyester, Polyamide, Polyanhydrid, Polyiminocarbonate, Polyanhydride, Polyorthoester, Polydioxanone, Polyphosphazene, Polyhydroxycarbonsäuren, Polyaminosäuren, oder Polysaccharide verwendet werden.

**15.** Kohlenhydratrezeptorblocker nach Anspruch 1 oder 2, dadurch gekennzeichnet, daß der Wirkungsverstärker ein intramolekularer Vernetzer, eine hydrophobe, hydrophile oder ionische Gruppe oder ein Löslichkeitsverbesserer ist.

**16.** Kohlenhydratrezeptorblocker nach Anspruch 1, 2 oder 15, dadurch gekennzeichnet, daß der Wirkungsverstärker die folgende Formel hat: (hydrophiles, biodegradables Polymer)

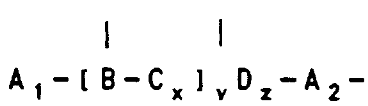

wobei
x für 0 oder 1;

y für 1, 2;

z für 0 oder 1 steht, mit der Maßgabe, daß z nur 1 sein kann, wenn x = 1 ist;

B, D gleich oder verschieden sind und Alkylen mit 0 bis 20 C-Atomen, die geradkettig, verzweigt oder mono- oder bicyclisch sein können, unsubstituiert oder substituiert sind, Einfach- und/oder Doppelbindungen oder 1 bis 2 aromatische Ringe bedeutet,

C einen Substituenten der folgenden Formel bedeutet:

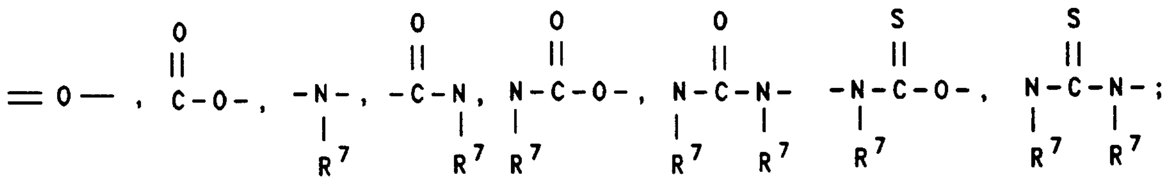

wobei $R^7$ unabhängig voneinander Wasserstoff, Methyl-, Ethyl-, Benzyl-, Propyl- oder Acetylgruppe bedeuten können;

die durch den Substituenten y definierten, repetitiven Einheiten gleich oder verschieden sein können;

$A_1$ und $A_2$ gleich oder verschieden sind und aus der kovalenten Verknüpfung von je einer reaktiven mit einer aktivierten Gruppe entstehen.

**17.** Kohlenhydratrezeptorblocker nach Anspruch 1, 2, 15 oder 16, dadurch gekennzeichnet, daß C einen Substituenten der folgenden Formel bedeutet:

Bevorzugt sind =

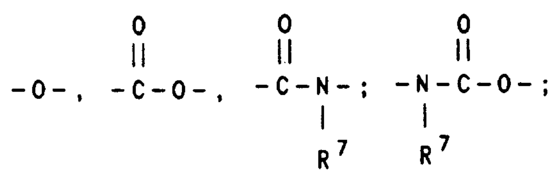

wobei $R^7$ die schon genannten Bedeutungen hat.

**18.** Arzneimittel, enthaltend Kohlenhydratrezeptorblocker nach Anspruch 1 oder 2 und gegebenenfalls pharmazeutische Träger.

**19.** Verfahren zur Herstellung eines physiologisch verträglichen und physiologisch abbaubaren Kohlenhydratrezeptorblockers auf Polymerbasis mit einem HLB*-Wert von 10 bis 20 nach Anspruch 1, dadurch gekennzeichnet, daß Kohlenhydratanteil, bifunktioneller Spacer, hydrophiles, biodegradabeles Polymer und Wirkungsverstärker enzymatisch, chemisch und/oder chemoenzymatisch verknüpft sind.

**20.** Verfahren zur Herstellung eines Kohlenhydratrezeptorblockers nach Anspruch 19, dadurch gekennzeichnet, daß die Synthese durch Bildung einer kovalenten Bindung zwischen Kohlenhydratanteil und bifunktionellem Spacer, anschließender kovalenten Verknüpfung des Kohlenhydratanteil-Spacer-Komplexes mit dem biodegradablen, hydrophilen Polymer und abschließender kovalenter Verknüpfung des Kohlenhydratanteil-Spacer-Polymer-Komplexes mit dem Wirkungsverstärker erfolgt.

**21.** Verfahren zur Herstellung eines Kohlenhydratrezeptorblockers nach Anspruch 19, dadurch gekennzeichnet, daß die Synthese durch Umsetzung des biodegradablen, hydrophilen Polymers mit dem Wirkungsverstärker einerseits und des Kohlenhydratanteils mit dem bifunktionellen Spacer andererseits unter Ausbildung kovalenter Bindungen unter anschließender kovalenter Verknüpfung des Polymer-Wirkungsverstärker-Komplexes mit dem Kohlenhydratanteil-Spacer-Komplex.

**22.** Verwendung eines Kohlenhydratrezeptorblockers nach Anspruch 1 oder 2 zur Herstellung eines Arzneimittels mit diagnostischer, therapeutischer oder prophylaktischer Anwendung bei der Bekämpfung von Krankheiten, bei welchen bakterielle oder virale Infektionen sowie metastasierende Tumore und entzündliche Prozesse involviert sind.